# EUROPÄISCHE PATENTSCHRIFT

(11) **EP 2 683 285 B1**
(45) Veröffentlichungstag und Bekanntmachung des Hinweises auf die Patenterteilung: **18.02.2015**
(21) Anmeldenummer: 12709586.7
(22) Anmeldetag: 12.03.2012
(51) Int. Cl.: A61B 1/015, A61B 1/00, A61M 1/00

(54) **VAKUUMSYSTEM UND ENDOSKOPIE-ANORDNUNG FÜR DIE ENDOSKOPISCHE VAKUUMTHERAPIE**
VACUUM SYSTEM AND ENDOSCOPY ARRANGEMENT FOR ENDOSCOPIC VACUUM THERAPY
SYSTÈME À VIDE ET ENSEMBLE D'ENDOSCOPIE POUR LE TRAITEMENT ENDOSCOPIQUE PAR LE VIDE

(30) Priorität: 11.03.2011 DE 102011013744; 11.03.2011 DE 102011013743; 08.12.2011 DE 102011120411; 17.02.2012 DE 102012003129
(43) Veröffentlichungstag der Anmeldung: 15.01.2014
(73) Patentinhaber: Lohmann & Rauscher GmbH & Co. KG, 56567 Neuwied (DE)
(72) Erfinder: LOSKE, Gunnar, 22926 Ahrensburg (DE)
(74) Vertreter: Leinweber & Zimmermann
(86) Internationale Anmeldenummer: PCT/EP2012/054276
(87) Internationale Veröffentlichungsnummer: WO 2012/123414

(56) Entgegenhaltungen:
- DE-A1-102009 039 515
- US-A1- 2004 093 026
- WEIDENHAGEN ROLF ET AL: "Anastomotic leakage after esophageal resection: new treatment options by endoluminal vacuum therapy.", November 2010 (2010-11), THE ANNALS OF THORACIC SURGERY NOV 2010 LNKD- PUBMED:20971288, VOL. 90, NR. 5, PAGE(S) 1674 - 1681, XP002676647, ISSN: 1552-6259 das ganze Dokument

## Beschreibung

Die vorliegende Erfindung betrifft ein Vakuumsystem und eine Endoskopie-Anordnung für die endoskopische Vakuumtherapie, insbesondere für die endoskopische intracorporale, intraluminale, oder intracavitäre Vakuumtherapie.

### Stand der Technik

Endoskopische Untersuchungen des oberen und unteren Gastrointestinaltraktes (Ösophagogastroduodenoskopie/Recto-, Sigmoido-, lleocoloskopie, Dünndarmendoskopie) sind diagnostische und therapeutische Routineuntersuchungen.

Die Untersuchung des mittleren Intestinaltraktes, insbesondere des Dünndarmes ist endoskopisch schwierig, da er sehr lang und außerordentlich beweglich ist. Es werden zum einen überlange Endoskope zur sogenannten Pushenteroskopie, zum anderen die sogenannte Single- bzw. Doppelballonenteroskopie eingesetzt. Letztere nutzt zum besseren Vorschub des Endoskops Ballonsysteme am Endoskop und/oder am Overtube, die bei der Untersuchung aufgepumpt sind und sich von innen an die Darmwand pressen können. Dadurch kann sich das Endoskop bzw. der Overtube an der Darmwand festklemmen und so eine tiefere Untersuchung des Darmes gelingen. Eine weitere Untersuchungsmöglichkeit des Darmes besteht in der fotographischen Aufzeichnung durch eine verschluckbare Videokapsel.

Konventionelle Vakuumschwammtherapie (Niederdruck-Wundtherapie) wird zur Behandlung von äußeren Wunden eingesetzt. Ein offenporiger Polyurethanschwamm oder ein anderes Fluidsammelmittel wird in die Wunde eingelegt, mit einer Folie versiegelt und dann unter einen Unterdruck gesetzt. Hierunter kann die Wundsäuberung und Wundheilung stattfinden.

Die Offenlegungsschrift DE 10 2009 039 515 A1 offenbart eine Vakuumtherapievorrichtung zur Förderung der Heilung einer Wunde.

### Erläuterung der Erfindung

Erfindungsgemäß wird ein Vakuumsystem für die endoskopische intracavitäre, intraluminale oder intracorporale Vakuumtherapie vorgeschlagen, zum Absaugen von Körperflüssigkeiten, Wundsekreten oder Gasen aus einem Hohlvolumen wie einer Körperhöhle, einem Hohlorgan, einem Gewebeabszess oder einem Intestinallumen, insbesondere beim Herstellen eines passageren endoskopischen Verschlusses eines Intestinallumens. Das Vakuumsystem umfasst:
- eine Vakuumpumpe, die einen Steuereingang zum Empfang eines Steuersignals für eine Steuerung ihrer Saugleistung hat und die unterdruckseitig einen Anschluss für eine Vakuumdrainageanordnung aufweist, und
- eine mit dem Steuereingang der Vakuumpumpe verbundene oder verbindbare Druck-Regeleinheit,
   -- die einen Messsignaleingang zum Empfang mindestens eines Druck-Messsignals aufweist, welches ein Maß für einen am zu behandelnden Hohlvolumen herrschenden Druck oder Unterdruck bildet,
   -- und die ausgebildet ist,
      nach Vorgabe
      a) eines Unterdruckwertes an dem zu behandelnden Hohlvolumen, der aus einem vordefinierten Unterdruck-Werteintervall wählbar ist, und
      b) einer Evakuierungszeitspanne, deren zwischen 0,5 und 5 Sekunden liegender Wert wählbar ist,
         i) unter Einrechnen eines vorbestimmten Totvolumens der an die Vakuumpumpe anschließbaren Vakuumdrainageanordnung eine zum Herstellen des vorgegebenen Unterdrucks an dem zu behandelnden Hohlvolumen in der vorgegebenen Evakuierungszeitspanne erforderliche erste Saugleistung der Vakuumpumpe zu ermitteln und dem Steuereingang der Vakuumpumpe ein entsprechendes erstes Steuersignal zuzuführen,
         ii) nach Herstellen des vorgegebenen Unterdrucks am zu behandelnden Hohlvolumen das Druck-Messsignal zu überwachen und in Abhängigkeit vom aktuellen Druck-Messsignal eine zur Aufrechterhaltung des vorgegebenen Unterdrucks erforderliche zweite Saugleistung der Vakuumpumpe zu ermitteln und dem Steuereingang der Vakuumpumpe ein entsprechendes zweites Steuersignal zuzuführen; und
         iii) nach Herstellen des vorgegebenen Unterdrucks am zu behandelnden Hohlvolumen bei Vorliegen einer eine vordefinierte Schwelle überschreitenden Abweichung des gemessenen Drucks oder Unterdrucks vom vorgegebenen Unterdruck eine zum Herstellen des vorgegebenen Unterdrucks innerhalb der vorgegebenen Evakuierungszeitspanne erforderliche dritte Saugleistung zu ermitteln und dem Steuereingang der Vakuumpumpe ein entsprechendes drittes Steuersignal zuzuführen; wobei
- die Vakuumpumpe ausgebildet ist, unterdruckseitig in Abhängigkeit von dem an ihrem Steuereingang aktuell anliegenden Steuersignal eine durch das Steuersignal bestimmte Saugleistung zu erzeugen.

Nachfolgend werden zunächst der Erfindung zugrunde liegende Erkenntnisse näher erläutert. Anschließend werden Ausführungsbeispiele vorgestellt.

Die Erfindung beruht auf der Erkenntnis, dass die Erfahrungen der Vakuumtherapie an äußeren Wunden bei der endoskopischen Vakuumtherapie nicht anwendbar sind. Davon ausgehend erkennt sie einen raschen Aufbau des Vakuums mit einer kurzen Evakuationszeit als wesentliche technische Voraussetzung, die über den Erfolg einer endoskopischen Vakuumtherapie entscheiden kann.

Die Anforderungen an eine Vakuumpumpeneinheit für die endoskopische Vakuumtherapie lassen sich erfindungsgemäß folgendermaßen spezifizieren: Der an einem Fluidsammelelement anliegende Unterdruck muss schnell ausreichend hoch sein, damit sich das Fluidsammelelement fest an das umgebende Gewebe ansaugen kann. Das Vakuum darf nicht zu hoch sein, damit es über eine offenporige Struktur eines anzuschließenden Fluidsammelelements eine Drainage-Wirkung auf das umliegende Gewebe erzielen kann. Es darf durch die Sogwirkung keine Verletzung des angesaugten Gewebes eintreten. In solchen Fällen fehlt die ausreichende Drainagewirkung an der Wunde.

Die Saugleistung der Vakuumpumpe ist daher entsprechend der vorliegenden Erfindung so ausgelegt und mittels der Druck-Regeleinheit einstellbar, dass ein unter diesen Randbedingungen definiertes Vakuum in einem sehr kurzen Zeitintervall, bzw. mit einer hohen Geschwindigkeit aufgebaut und konstant aufrechterhalten werden kann. Die Therapie wäre ineffektiv, wenn die den Vakuumaufbau betreffenden Parameter nicht den spezifischen Anforderungen entsprächen. Nur bei schnellem Vakuumaufbau und einer Aufrechterhaltung sowie im Bedarfsfall schnellen Wiederherstellung des Vakuums wird beispielsweise bei einer typischen Behandlungsindikation, nämlich der Behandlung von Speiseröhrenvertetzungen, gleichzeitig ein Verschluss des Perforationsdefektes und eine effektive Wunddrainage vorgenommen. Der permanente gesicherte Verschluss und die Drainage entgegen dem physiologischen intrathorakaten Unterdruck in Richtung des Speiseröhrenlumens stoppt eine Kontamination durch Speichel oder Sekret in Richtung der Brusthöhle und wirkt so als Infektbarriere. Bei längerem Vakuumaufbau und schon bei kurzen Unterbrechungen des vorgegebenen Unterdrucks kann eine Dislokation eines Fluidsammelelements eintreten. Unterbrochener oder ineffektiver Sog am Wundgrund führt zum Therapiestillstand oder zur Verschlechterung der Wundsituation. Bei einer intraluminalen Behandlung in der Speiseröhre bei einer Speisenröhrenperforation kann beim Verlust des Unterdrucks verschlucktes zähes Speichelsekret zwischen Speiseröhrenwand und Fluidsammelelement gelangen und zu einer Verstopfung der Poren und damit zum Therapieabbruch führen. Bei anderen Anwendungsmöglichkeiten im Dünn-oder Dickdarm kann bei ungenügenden Druckparametern eine Verstopfung der Poren durch Dünn- oder Dickdarmstuhl eintreten.

Bei der endoskopischen Vakuumtherapie wird gemäß der Erkenntnis der vorliegenden Erfindung ein permanenter Unterdruck an der Wunde hergestellt und ein Abfall des Unterdrucks unter der Therapie vermieden. Im Falle eines manchmal unvermeidlichen Abfalls des Unterdrucks wird das Vakuum durch das erfindungsgemäße Vakuumsystem sehr rasch wiederaufgebaut. Beispielsweise kann bei der Anlage einer Schwammdrainage in der Speiseröhre durch den physiologischen Schluckakt ein Abfall des Vakuums sowohl durch das Verschlucken von Speichel, Nahrung, Luft und Gas aber auch die Darmperistaltik auftreten. Gleiches gilt für die Anwendung der Vakuumtherapie am Dünn-, Dickdarm oder Magen bzw. im gesamten Intestinum.

Im Unterschied zur Vakuumtherapie an äußeren Wunden fehlt bei der endoskopischen Vakuumtherapie die Möglichkeit der visuellen und palpatorischen Kontrolle, ob der Unterdruck an dem Fluidsammelelement und an der inneren Wunde anliegt, da das Fluidsammelelement unterhalb der Körperoberfläche nach der Platzierung nicht mehr zu sehen ist. Ein Therapiestillstand tritt ein bzw. die Therapie ist ineffektiv, wenn die Sogausübung an der Wunde zu langsam aufgebaut oder unterbrochen wird. Eine Sogunterbrechung kann unter anderem auch durch Verstopfung, Abknickung oder Kollabieren des Fluidkommunikationselements oder des Fluidsammelelements eintreten. Auch bei einem zu hohen Vakuum kann es zum Verstopfen der offenporigen Struktur durch die Gewebeansaugung kommen und bei einem elastischen Fluidsammelmittelelement kann es zu einem kompletten Kollaps des Fluidkommunikationselementes mit Aufhebung der Sogwirkung am Gewebe kommen. Erst durch die in der Erfindung spezifizierten Merkmale des erfindungsgemäßen Vakuumsystems wird eine effektive innere Vakuumtherapie ermöglicht.

Bei der durch die Erfindung ermöglichten endoskopischen Vakuumtherapie handelt es sich um eine mit flexiblen Endoskopen unter endoskopischer Sicht und mit Hilfe endoskopischer Techniken durchgeführte Unterdrucktherapie von inneren Wunden, wobei Vakuumdrainagen in Höhlen (intracavitär), Darmlumina (intraluminal) über natürliche oder künstliche Körperöffnungen intracorporal in Hohlräume eingebracht werden. Es wird mit dem erfindungsgemäßen Vakuumsystem also eine endoskopische Vakuumtherapie von inneren Wunden möglich, die unbehandelt z.T. mit einer hohen Mortalität einhergehen oder häufig einer komplexen operativen Behandlung bedürfen.

Im Unterschied zur Vakuumtherapie an äußeren Wunden wird bei der endoskopischen intraluminalen, intracavitären und intracorporalen Vakuumtherapie mit dem Vakuumsystem der vorliegenden Erfindung erst durch eine gegenseitige Anlagerung von Wund -und Weichteilgewebe um das Fluidsammelelement, welches im chirurgischen Einsatz fluidleitend durch Fluidkommunikationselemente mit der Vakuumpumpe des erfindungsgemäßen Vakuumsystems verbunden ist, nach Anlage des Unterdrucks eine innere Wundversiegelung erzielt. Erst durch diese Gewebeversiegelung entsteht der möglichst luftdicht geschlossene Raum, der die dauerhafte Erzeugung und Aufrechterhaltung eines Vakuums ermöglicht. Der Hohlraum, in welchem sich das Fluidsammelelement befindet, wird leergesaugt und kollabiert vakuumbedingt über dem Fluidsammelelement. Wenn das Fluidsammelelement elastisch konstruiert ist, kollabiert auch dieses durch das Vakuum. Mit Hilfe der Vakuumpumpe des erfindungsgemäßen Vakuumsystems wird eine permanente Sogwirkung an der Wunde, welche hierdurch verschlossen wird, erzeugt. Die Fixierung des Fluidsammelelements am Platzierungsort geschieht nur durch das Festsaugen an dem anliegenden Gewebe bzw. der Darmschleimhaut.

Im Unterschied zur Vakuumtherapie an äußeren Wunden, bei der eine Versiegelung durch einen Folienokklusionsverband vorgenommen wird, ist die Versiegelung bei der endoskopischen Vakuumtherapie, die erst vakuumbedingt durch das aneinanderliegende Gewebe entsteht, weniger stabil. Die Versiegelung und damit auch die Fixierung des Fluidsammelmittels erfolgt ausschließlich dadurch, dass sich das Fluidsammeletement durch das Vakuum saugnapfartig am Gewebe festsaugt und das Vakuum permanent und konstant aufrecht gehalten wird. Der Erfindung liegt daher die Erkenntnis zugrunde, dass zur erfolgreichen Durchführung der endoskopischen Vakuumtherapie ein Vakuumsystem die spezifizierten und von der Druck-Regeleinheit eingestellten und überwachten Anforderungen erfüllen muss.

Zum Aufbau eines Vakuums zur Durchführung einer Vakuumendoskopie ist im erfindungsgemäßen. Vakuumsystem eine Vakuumpumpe vorgesehen, deren Saugleistung steuerbar ist und die ausgebildet ist, innerhalb einer kurzen, definierten Evakuierungszeitspanne von zwischen 0,5 und 5 Sekunden einen vorgegebenen Unterdruck am Einsatzort des Fluidsammelelements zu erzeugen und dann auf einem konstanten Wert aufrechtzuerhalten.

Die Erfindung beruht weiterhin auf der Erkenntnis, dass eine weitere Einflussgröße, nämlich das Volumen der zu evakuierenden Wundhöhle oder das Darmlumen vernachlässigbar ist. Umgekehrt lässt sich festhalten, dass bei konstanten zu evakuierenden Volumina (Fluidkommunikationselement, Fluidsammelelement, Sekretbehälter) die Geschwindigkeit des Sogaufbaus in dem vorliegend relevanten Wertbereich des Unterdrucks bei bekanntem Volumen des Sekretauffangbehälters praktisch allein über die Saugleistung der Vakuumumpe (Liter/Minute) steuerbar ist. Da sich der Sekretauffangbehälter mit Sekret anfüllen kann, verkleinert sich dann das Totvolumen. Dieses kann auch gemessen und die Saugleistung entsprechend automatisch an den Füllungszustand des Behälters angepasst werden.

Nachfolgend werden Ausführungsbeispiele des erfindungsgemäßen Vakuumsystems beschrieben.

In der vorliegenden Beschreibung werden Werte des Unterdrucks gegenüber dem Umgebungsdruck angegeben. In der Literatur werden diese Unterdruckwerte häufig auch mit negativem Vorzeichen versehen. Darauf wird vorliegend verzichtet, sondern allein der Betrag des Unterdrucks angegeben. Die Unterdruckwerte werden, wie in der Fachwelt üblich, in Einheiten von mmHg angegeben, wobei für die Zwecke der Umrechnung in SI-Einheiten ein Verhältnis von 1 mmHg = 133,322368421 Pa verwendet werden kann. Der Begriff Vakuum und wird in der vorliegenden Beschreibung synonym zum Begriff Unterdruck verwendet.

Der Erfinder hat erkannt, dass Unterdrücke eines Betrages von weniger als 60 mmHg und eines größeren Betrages als 500mmHg in der Praxis nicht erforderlich sind und die Leistungsfähigkeit der Vakuumpumpe insofern zugunsten einer begrenzt leistungsfähigen, dafür leichteren und vorzugsweise vom Patienten tragbaren Konstruktion begrenzt werden kann.

Als Vakuumpumpe ist in unterschiedlichen Ausführungsbeispielen eine Verdrängerpumpe, wie beispielsweise eine Sperrschieberpumpe, eine Drehschieberpumpe, eine Trochoidenpumpe, eine Scrollpumpe, eine Kolbenpumpe, eine Schraubenpumpe, eine Drehkolbenpumpe, eine Rollenpumpen oder eine Membranpumpe vorgesehen.

Als die im erfindungsgemäßen Vakuumsystem vorgesehene Vakuumpumpe sollen im Rahmen der vorliegenden Beschreibung auch Kombinationen von mindestens zwei Pumpen oder mehrstufige Pumpensysteme verstanden werden. In einer Ausführungsform ist die Vakuumpumpe beispielsweise mit zwei Pumpstufen ausgerüstet. Bevorzugt ist die Vakuumpumpe dabei mit einer Pumpenkombination ausgestattet. Bevorzugt wird das Vakuum über ein Vorväkuum mit Hilfe einer Vorpumpe erzeugt.

Ein für die erfolgreiche Behandlung wichtiger Parameter des erfindungsgemäßen Vakuumsystems ist die für die Evakuierung des jeweils betroffenen Volumens bis zum erforderlichen Unterdruck in den zu behandelnden Partien der Hohlvolumina benötigte Evakuierungszeitspanne. Die maximale Saugleistung der Vakuumpumpe ist in bevorzugten Ausführungsformen so ausgelegt, dass unter Berücksichtigung der in der Praxis auftretenden Totvolumina in einem kurzen Zeitraum von etwa einer halben Sekunde der erfindungsgemäß vorgegebene Druckwert des Vakuums erreicht wird.

In anderen Ausführungsformen ist die maximal mögliche Evakuierungszeitspanne bis zu wenige Sekunden, insbesondere maximal 2 Sekunden lang, um ein definiertes kontinuierliches Vakuum zu erreichen. Entsprechend ist in den genannten Ausführungsformen des Vakuumsystems die Druck-Regeleinheit ausgebildet, die Vakuumpumpe im Betrieb zur Erzielung des Vakuums innerhalb eines Wertbereichs der Evakuierungszeitspanne anzusteuem, der die genannten Minimal- bzw. Maximalwerte der Evakuierungszeitspanne umfasst. Der Unterdruck wird nach der Evakuierungszeitspanne konstant aufrechterhalten.

Vorteilhafte Ausführungsformen des Vakuumsystems haben zusätzlich eine mit der Druck-Regeleinheit verbundene Nutzereingabeeinheit, die ausgebildet ist, eine Nutzereingabe der Evakuierungszeitspanne und/oder eines Unterdruckwertes anzunehmen und der Druck-Regeleinheit zuzuleiten. Die Druck-Regeleinheit ist ausgebildet, das betreffende Steuersignal unter Einrechnung der aktuellen Nutzereingabe zu ermitteln und dem Steuereingang der Vakuumpumpe zuzuleiten.

In einer Variante ist für Ausnahmesituationen zusätzlich ein Betrieb des Vakuumsystems mit einer Evakuierungszeitspanne von mehr als 5 Sekunden durch entsprechende Nutzereingabe an der Nutzer-Eingabeeinheit (oder an einem vom Arzt zu bedienenden Handstück oder Fußpedal, das mit der Nutzereingabeeinheit verbunden ist) möglich. Dies kann auch sinnvoll sein, wenn mit einem einzigen Pumpsystem sowohl die vorliegend fokussierte Vakuumendoskopie als auch eine Vakuumbehandlung äußerer Wunden durchführbar sein soll.

Eine zeitweilige gewisse Erhöhung des Vakuums gegenüber dem für die Therapie vorgesehenen Wert kann initial, also zu Beginn der Therapie, über eine kurze Zeitspanne angezeigt sein, um eine sichere Befestigung einer Drainage am Therapieort sicherzustellen. Auch nach der Platzierung ist initial vorrübergehend ein höherer Unterdruck vorteilhaft, damit das Fluidsammelmittel sich so festsaugen kann, damit es beispielsweise nicht akzidentiell durch ein in dieser initialen Phase in den Körper eingeführtes Endoskop disloziert. Hierbei handelt es sich aber gegebenenfalls im Vergleich mit der gesamten Therapiedauer um eine relativ kurze initiale Zeitspanne von beispielsweise 15 Minuten, während die Therapiedauer sich typischerweise über mehrere Tage erstrecken kann.

Nach der erfolgreichen Platzierung wird der Patient das Vakuumsystem typischerweise mit sich herumtragen. Bevorzugt weist die Nutzereingabeeinheit einen sperrbaren Modus-Schalter auf, der eine nutzerseitige Einstellung entweder eines Therapiemodus oder eines Endoskopiemodus erlaubt, wobei die Druck-Regeleinheit ausgebildet ist, im Therapiemodus nur das zweite oder dritte Steuersignal, jedoch nicht das erste Steuersignal auszugeben, und wobei das vordefinierte Unterdruck-Werteintervall sich im Therapiemodus über Unterdruckwerte gegenüber einem Umgebungsdruck zwischen einem minimalen Unterdruck von 60mmHg und einem maximalen Unterdruck von 250mmHg erstreckt. Die Sperrung des Modus-Schalters ist vorzugsweise nur mit einem Schlüssels möglich, wobei unter Schlüssel auch ein Code verstanden werden kann.

Gemäß einer zur Weiterbildung der Erfindung führenden Erkenntnis des Erfinders wird der Therapieerfolg weiter verbessert, wenn die Evakuierungszeitspanne und der Unterdruck entsprechend einer jeweils durchzuführenden Untersuchung oder Therapie adaptierbar ist. Bei der Behandlung von Speiseröhrenverletzungen beispielsweise sind der pathophysiologische intrathorakale Unterdruck und die Druckschwankungen durch Atembewegung entgegen der Sogwirkung der Vakuumpumpe gerichtet. Dieser physiologische Unterdruck muss durch eine sehr kurze Evakuationszeitspanne der Vakuumpumpe in Sogrichtung der Pumpe aufgehoben bzw. entgegengewirkt werden. Im Unterschied zur Therapie an der Speiseröhre kann bei einer Unterdruckbehandlung nach Anastomoseninsuffizienzen am Enddarm bei vorgeschaltetem Anus praeter und nur geringer Sekretion auch eine längere Evakuationszeitspanne innerhalb der erfindungsgemäßen Grenzen von bis zu 5 Sekunden therapeutisch erfolgreich sein.

Wenn das Vakuum bis zum definierten Druck aufgebaut ist, dann muss dieser Druck konstant aufrecht gehalten werden. Ein Abfall des Unterdrucks ist vom erfindungsgemäßßen Vakuumsystem mit Hilfe der Messsonde sofort registrierbar und innerhalb der Evakuierungszeitspanne wieder beseitigbar, wobei aufgrund des meist noch verbleibenden Restvakuums vorteilhafterweise sogar eine deutlich kürzere Zeitspanne als die Evakuierungszeitspanne zur Wiederherstellung des Sollwerts des Vakuums benötigt wird. In diesem Sinne kann ein konstanter Unterdruck am Therapieort sichergestellt werden. Bevorzugt sind die Druck-Regeleinheit und die Vakuumpumpe daher ausgebildet, in Abhängigkeit von eingehenden Messsignalen mit einer Frequenz von mindestens 30 Vakuumaufbauten/Minute den definierten Unterdruck aufbauen zu können. Dies erweist sich für die Durchführung einer endoskopischen Vakuumschwammtherapie am oberen Gastrointestinaltrakt als vorteilhaft. In weiteren Ausführungsformen sind mit dem Vakuumsystem bis zu 60, weiter bevorzugt 120 Vakuumaufbauten/Minute durchführbar.

In einer bevorzugten Ausführungsform ist weiterhin über die Druck-Regeleinheit die Evakuierungszeitspanne durch Nutzereingabe einstellbar. Dazu weist das Vakuumsystem zusätzlich eine mit der Druck-Regeleinheit verbundene Nutzereingabeeinheit auf, welche ausgebildet ist, eine Nutzereingabe der Evakuierungszeitspanne anzunehmen und der Druck-Regeleinheit zuzuleiten. Die Druck-Regeleinheit ist ausgebildet, die Vakuumpumpe in Abhängigkeit von der Nutzereingabe anzusteuern, den Unterdruck in der vorgegebenen Evakuierungszeitspanne zu erzeugen.

Die Druck-Regeleinheit ist in einer weiteren Ausführungsform ausgebildet, eine Auswahl zwischen folgenden vordefinierten Therapieeinstellungen über die Nutzereingabeeinheit durch entsprechende vordefinierte Steuerparameter zu unterstützen:
a) eine Evakuierungszeit von höchstens 2 Sekunden;
b) eine durch weitere Nutzereingabe näher definierbare Evakuierungszeit von zwischen 0,5 und 5 Sekunden;
c) eine einstellbare Evakuierungszeit von 2-5 Sekunden.

Beispielsweise ist für die Behandlung einer Leckage am Ösophagus ein Unterdruck zwischen 80 und 150 mmHg (10665 und 20000 Pa) und die Auswahl einer Evakuationszeit von höchstens 2 Sekunden vorteilhaft.

Der Wert des zu wählenden Unterdruckes ist in vielen Anwendungsfällen auch abhängig von einer Kontaktfläche eines Fluidsammelmittels mit dem umgebenden Gewebe. Bei einer großen Kontaktfläche kann zur Fixierung des Fluidsammelmittels im Vergleich zu einer kleinen Kontaktfläche ein geringerer Unterdruck erforderlich sein.

Die Nutzereingabeeinheit ist daher vorzugsweise zusätzlich ausgebildet, eine zusätzliche Eingabe einer Identifikation eines Typs eines Fluidsammelelements zu empfangen. Die Druck-Regeleinheit ist in dieser Ausführungsform ausgebildet, auf der Grundlage vorgespeicherter Therapiedaten dem eingegebenen Typ des Fluidsammelelements zugeordnete Werte des Vakuums und/oder der Evakuierungszeitspanne zu ermitteln, und die Vakuumpumpe im Betrieb entsprechend diesen ermittelten Werten anzusteuern.

In der klinischen Anwendung hat sich grundsätzlich ein kontinuierlicher dauerhafter Unterdruck bewährt. Eine Variante sieht jedoch vor, dass die Druck-Regeleinheit ausgebildet ist, die Vakuumpumpe anzusteuern, den Unterdruck ondulierend zwischen mindestens zwei Unterdruckwerten, zum Beispiel zwischen etwa etwa 100 mmHg und etwa 150 mmHg, schwankend anzulegen. Durch eine schwankende Unterdruckausübung kann der Granulationsreiz auf die Wunde erhöht werden. Es darf jedoch zu keinem Zeitpunkt eine länger als wenige Sekunden dauerende Sogunterbrechung auftreten.

Die Druck-Regeleinheit ist ausgebildet, Unterdruckwerte während einer Unterdruckanwendung und einer mit Unterdruck durchgeführten Untersuchung zu überwachen. Messfühler sind entweder in elektrischer, also drahtgebundener, oder drahtloser Kommunikation mit der Druck-Regeleinheit des Vakuumsystems verbindbar, so dass voreingestellte zu erzeugende Unterdruckwerte der Vakuumpumpe durch die Druck-Regeleinheit kontrollier- und regulierbar sind. Auf diese Weise kann aufgrund der Druckerfassung durch Messfühler eine Kontrolle und Steuerung des Pumpensoges direkt an einem Fluidsammelelement vorgenommen werden. Hierdurch wird verhindert, dass ein Therapiestillstand zum Beispiel bei einer Verstopfung des Fluidsammelmittels oder des Fluidkommunikationselementes eintritt. Dieses ist insbesondere bei der Behandlung bei Ösophagusverletzungen wichtig, da anderenfalls eine Entzündung der Brusthöhle eintritt, welche eine langwierige Behandlung nach sich zieht und häufig zum Tode führt.

Bevorzugt erfasst die Druck-Regeleinheit durch Auswertung der eingehenden Messsignale der Drucksensoren unter Vergleich mit einem jeweiligen Sollwert auch die tatsächlich benötigte Evakuationszeit.

Das Vakuumsystem ist vorzugsweise mit einer Vakuumdrainageanordnung ausgestattet, die der Vakuumpumpe unterdruckseitig vorgeschaltet ist. Bei gleicher Saugleistung der Vakuumpumpe ist ein schneller Sogaufbau bei einem kleineren Sekretauffangbehälter als bei einem großen Auffangbehälter möglich. Die Druck-Regeleinheit ist daher bevorzugt ausgebildet, über die Nutzereingabeeinheit eine Nutzereingabe eines Volumens des Auffangbehälters entgegenzunehmen und die Pumpleistung in zusätzlicher Abhängigkeit vom eingegebenen Volumen einzustellen. Dabei steuert die Druck-Regeleinheit die Pumpleistung nicht nur wie bereits erläutert entsprechend der vom Nutzer gewünschten Evakuierungszeit, sondern berücksichtigt hierfür zusätzlich auch das Volumen des Sekretauffangbehälters.

Die Druck-Regeleinheit ist ausgebildet, ein Volumen des Sekretauffangbehälters als Teil des Totvolumens einzurechnen. Je nach Anwendung können unterschiedliche Volumina für den Sekretauffangbehälter erforderlich sein, so dass die Druck-Regeleinheit entsprechend unterschiedliche Werte des Totvolumens verwenden können muss. Diese können beispielsweise in einem Speicher der Druck-Regeleinheit abgespeichert und durch Nutzereingabe ausgewählt werden. Zur Vermeidung von Eingabefehlern kann alternativ eine am Sekretauffarigbehälter selbst angebrachte und von der Druck-Regeleinheit lesbare Kodierung erfasst werden, aus der der zutreffende Wert des Totvolumens ableitbar ist. Bei einem kleinen Volumen des Auffangbehälters wird die Saugleistung der Pumpe niedriger eingestellt, bei einem größeren Volumen wird die Saugleistung entsprechend höher eingestellt.

Der Sekretauffangbehälter ist ausgebildet, im Betrieb anfallendes Sekret und Gas, welches durch die Vakuumpumpe angesaugt wird, aufzunehmen und/oder abzuleiten. Bevorzugt ist die Pumpe zusätzlich mit einem unterdruckstabilen Vorsekretauffangbehälter ausgestattet, der in Saugrichtung vom Anwendungsort am Patienten zur Vakuumpumpe hin dem Sekretauffangbehälter vorgeschaltet ist und mit dem Sekretauffangbehälter fluidleitend verbunden. Die Druck-Regeleinheit ist dann entsprechend ausgebildet, ein Volumen des Vorsekretauffangbehälters als weiteren Teil des Totvolumens einzurechnen.

Aufgefangenes Sekret kann in dieser Variante aus dem Vorsekretauffangbehälter zum Sekretauffangbehälter weitergeleitet werden. Bevorzugt sind der Vorsekretauffangbehälter und der Sekretauffangbehälter über ein Ventil miteinander verbunden. Zusätzlich oder alternativ zum Ventil sind der Vorsekretauffangbehälter und der Sekretauffangbehälter über einen zwischenschaltbaren Filter miteinander verbindbar. Bevorzugt sind diese Auffangbehälter und ihre Verbindung mit dem Vakuum auswechselbar gestaltet.

Diese Ausführungsformen sehen einen Sogaufbau über das Totvolumen des Sekretauffangbehälters vor. Wenn der Sogaufbau der Vakuumpumpe über einen Sekretauffangbehälter erfolgt, bestimmt im Wesentlichen dessen Totvolumen zusammen mit der Sogleistung der Pumpe (l/min) die Geschwindigkeit des Sogaufbaus. Die Saugleistung der Vakuumpumpe ist daher bevorzugt ausgelegt, zusätzlich das Totvolumen, das vom Sekretauffangbehälter und Vorsekretauffangbehälter gebildet ist, innerhalb der Evakuierungszeitspanne zu evakuieren.

Der Sekretauffangbehälter ist über vorzugsweise unterdruckstabile Fluidkommunikationselemente, insbesondere Drainageschläuche, mit einem Fluidsammelelement verbindbar, so dass der Unterduck am Fluidsammelelement über den Sekretauffangbehälter aufbaubar ist. Die Druck-Regeleinheit ist ausgebildet, ein zusätzliches Volumen, welches mindestens ein unterdruckstabiles Fluidkommunikationselement, insbesondere ein Drainageschlauch bildet, der distal mit einem Fluidsammelelement und proximal mit dem Sekretauffangbehälter oder dem Vorsekretauffangbehälter verbindbar ist, als weiteren Teil des Totvolumens einzurechnen. Bei konstanten Evakuationsvolumina an Fluidkommunikationselementen, Fluidsammelelementen und Sekretauffangbehältern ist in einer Ausführungsform die Evakuationszeit durch die Druck-Regeleinheit über eine Regelung einer Saugleistung der Vakuumpumpe zu regulieren. Dabei empfängt die Druck-Regeleinheit der Vakuumpumpe neben der Nutzereingabe als weiteren Input zur Regelung über den Messsignaleingang Messwerte von einer Unterdruckmesssonde, die am zu evakuierenden Hohlvolumen platziert ist. Einzelheiten der Ausbildung und Platzierung der Messsonde werden weiter unten erläutert.

Wird ein Vorsekretauffangbehälter verwendet, ist er bei diesen Ausführungsformen bevorzugt so mit der Vakuumpumpe verbunden, dass er mit einem Vorvakuum beaufschlagt werden kann. Bei dieser Ausführungsform hat die Vakuumpumpe zwei Pumpenstufen und ist ausgebildet, den Unterdruck an der Untersuchungs-/Behandlungsstelle mit Hilfe einer ersten der beiden Pumpenstufen über ein Vorvakuum im Vorsekretauffangbehälter zu erzeugen. Dieser hat vergleichsweise das kleinere Volumen der beiden Auffangbehäfter, um eine möglichst kurze Evakuierungszeit zu erzielen. Der Vorsekretauffangbehälter hat typischerweise ein Volumen von 50 bis 300 ml. Der Sekretauffangbehälter dagegen hat typischerweise ein Volumen von 100 ml bis 1000 ml. Es können aber auch kleinere oder größer Volumina gewählt werden, unter Anpassung der erforderlichen Saugleistung der Vakuumpumpe.

Eine weitere bevorzugte Ausführungsform sieht eine Druck-Regeleinheit vor, die die Leistungsfähigkeit der Pumpe nicht nur entsprechend dem Volumen des Sekretauffangbehälters anpasst, sondern zusätzlich ein Volumen eines zu evakuierenden Fluidsammelelements berücksichtigt. Auch hier ist wie bereits oben beschrieben eine zusätzliche Nutzereingabe über die Nutzereingabeeinheit vorgesehen, welche an die Druck-Regeleinheit weitergeleitet wird, welche wiederum die Pumpleistung zur Erzielung der jeweils erforderlichen Evakuierungszeitspanne entsprechend steuert. Dabei wird innerhalb der Evakuierungszeitspanne zugleich eine Evakuierung der Sekretauffangbehälter vorgenommen.

Bevorzugt ist die Leistungsfähigkeit der Pumpe so ausgelegt, dass innerhalb der Evakuierungszeitspanne das Totraumvolumen des Sekretauffangbehälters und des Fluidsammelelements evakuiert wird. Die bisherige Erfahrung zeigt, dass eine steuerbare Pumpleistung im Bereich von 1 l/min bis 20l/min erforderlich ist.

Die Druck-Regeleinheit ist in einer Ausführungsform des Vakuumsystems mit einer Überwachungseinheit ausgerüstet, die ein Über- und/oder Unterschreiten des Unterdrucks, der Dauer der Evakuierungszeitspanne sowie eine Zeitdauer einer Unterdruckanlage automatisch überwacht und bei Überschreiten vorgegebener Grenzwerte die Pumpleistung reguliert. So kann im Betrieb bei einem Abfall des Unterdrucks, zum Beispiel durch eine Insufflation von Untersuchungsgas, das Vakuum schnell wiederhergestellt und im Ergebnis kontinuierlich aufrechterhalten werden.

Bevorzugt sind neben dem Steuersignal-Eingang weitere Schalt- und Regelelemente an der Vakuumpumpe vorgesehen, über die eine Bedienung der Vakuumpumpe vorgenommen werden kann. Insbesondere können die Druck-Regeleinheit und die NutzereingabeEinheit mit der Vakuumpumpe als bauliche Einheit integriert sein.

Zur Erfassung und Überwachung des definierten Unterdrucks und der Evakuierungszeitspanne ist mindestens eine Unterdruckmesssonde an der Vakuumpumpe und/oder mindestens ein Anschluss für eine externe Unterdruckmesssonde vorgesehen. Die Unterdruckmesssonde ist direkt oder Indirekt mit einem an die Vakuumpumpe angeschlossenen Fluidsammelmittel und/oder einem Fluidkommunikationselement verbunden, und ausgebildet, ihre Messergebnisse als Messsignale an die Druck-Regeleinheit der Vakuumpumpe weiterzuleiten. Bevorzugt handelt es sich bei den Fluidkommunikationselementen um Drainageschläuche.

Mit der Vakuumpumpe kann an einem einzelnen oder an mehreren Fluidsammelelementen ein Vakuum aufgebaut werden. Für den Fall mehrerer Fluidsammelelemente ist die Vakuumpumpe bevorzugt ausgelegt, eine jeweilige Vakuumerzeugung völlig unabhängig voneinander auszuführen. Hierzu sind nicht nur eine Vielzahl entsprechender Anschlüsse und Drainageeinheiten vorgesehen. Zusätzlich ist auch die Pumpleistung der Vakuumpumpe an die höheren Anforderungen gleichzeitiger Unterdruckerzeugung an verschiedenen Fluidsammelelementen angepasst. Die Druck-Regeleinheit ist ausgebildet, Steuersignale an individuell steuerbare Drosselelemente auszugeben, die im jeweiligen Zweig angeordnet sind, um den individuell angepassten Aufbau eines jeweiligen Vakuums zu bewirken.

Insbesondere ist die Vakuumpumpe geeignet zur Vakuumerzeugung bei der endoskopischen intracavitären und intraluminalen Vakuumtherapie. Sie ist jedoch auch einsetzbar bei der Vakuumschwammtherapie an äußeren Wunden. Sie ist desweiteren einsetzbar bei der Vakuumendoskopie.

Das Vakuumsystem ist bevorzugt als tragbare Einheit konstruiert, damit sich ein Patient möglichst ungehindert mobil bewegen kann. Die elektrische Energieversorgung der Pumpe findet in der tragbaren Version beispielsweise über Batterie oder Akku statt.

Es sei angemerkt, dass in einer alternativen Ausführungsform in Behandlungsräumen die Vakuumpumpe in Form einer integrierten, zentral gesteuerten Vakuumwandsaugung vorliegen kann, welche in ihrer Pumpleistung entsprechend anzupassen ist, um zumindest das erfindungsgemäß erforderliche Vakuum in der erfindungsgemäß erforderlichen Evakuierungszeitspanne zu liefern. Auf diese Weise kann bei entsprechender Infrastruktur in einem Behandlungsraum die Erzeugung des notwendigen Vakuums auch ohne separate Vakuumpumpe stattfinden, wobei die entsprechend ausgebildete Wandabsaugung also die Vakuumpumpe ersetzt. Die Druck-Regeleinheit des erfindungsgemäßen Vakuumsystems muss in einer solchen Infrastruktur angepasst werden, um Vakuumdrucksteuerungselemente, beispielsweise Drosselelemente, je nach gegebener (meist nicht beeinflussbarer) Pumpleistung der Wandabsaugung zeitabhängig steuern zu können, damit die erforderlichen Unterdrücke zwischen Fluidsammelelement und Wandsaugung in der vorgegebenen Zeitspanne erreicht werden. Über Verbindungs-, Filter-, Schalt- und Ventilelement kann das Weiterleiten eines Vakuums auf die Fluidkommunikationselemente ermöglicht werden und die Bedienung über den Handgriff des Endoskops erfolgen.

Vorteilhaft umfasst die Nutzereingabeeinheit des Vakuumsystems eine Einrichtung zur manuellen Steuerung der Vakuumpumpe, mit welcher ein Startsignal zum Start und ein Steuersignal Nachlassen des Vakuums am Fluidsammelelement zur Weiterleitung an die Druck-Regeleinheit erzeugt und ausgegeben werden kann. Vorzugsweise ist die Nutzereingabeeinheit mit einer oder mehreren Schalteinrichtungen am Handgriff des Endoskops verbunden, alternativ ist auch eine Bedienung der Pumpe über Fuß/Handschalter oder direkt an der Pumpe möglich.

Um die Untersuchung, insbesondere eine Vakuumendoskopie bequem vornehmen zu können, muss man in kurzen Abständen während des Untersuchungsganges den Unterdruck aufbauen und ablassen können. Dafür ist in bevorzugten Ausführungsformen eine Schalteinheit am Endoskop oder ein Fußschalter vorgesehen.

Das Vakuumsystem hat in einer Ausführung eine Mehrzahl unterdruckseitiger Anschlüsse für einen oder mehrere Drainageschläuche. Die Druck-Regeleinheit ist in dieser Ausführungsform ausgebildet, auf eine entsprechende Nutzereingabe über die Nutzereingabeeinheit hin die Vakuumpumpe anzusteuem, wahlweise entweder einseitig nur an einem der Anschlüsse oder alternierend an zweien der Anschlüsse oder zeitgleich an zwei Anschlüssen zu saugen oder zu spülen. So kann gleichzeitig und unabhängig voneinander das Vakuum an mehreren Fluidsammelelementen gesteuert werden, was weiter unten im Rahmen der Figurenbeschreibung näher erläutert wird.

Das erfindungsgemäße Vakuumsystem bildet in bevorzugten Ausführungsbeispielen einen technischen Bestandteil einer erfindungsgemäßen Endoskopie-Anordnung mit
- einem solchen Vakuumsystem gemäß der Erfindung oder einem seiner im Rahmen dieser Anmeldung beschriebenen Ausführungsbeispiele,
- einer Overtubeeinheit, welche unterdruckseitig mit der Vakuumpumpe des Vakuumsystems durch mindestens ein Fluidkommunikationselement verbundenen ist und die ein Fluidsammelelement trägt,
- einem Endoskop, welches in die Overtubeeinheit eingeführt oder einführbar ist und relativ zur Overtubeeinheit in einer von proximal nach distal oder umgekehrt weisenden Richtung verschiebbar ist und
- einer Unterdruckmesssonde, die mit der Druck-Regeleinheit des Vakuumsystems verbunden ist.

Vorzugsweise ist auch das Endoskop unterdruckseitig mit der Vakuumpumpe des Vakuumsystems durch ein Fluidkommunikationselement verbundenen und trägt ein weiteres Fluidsammelelement.

Diesem erfindungsgemäßen Ausführungsbeispiel in Form einer Endoskopie-Anordnung liegt die Erkenntnis zugrunde, dass bei der im Stand der Technik bekannten Ballonenteroskopie eine ausreichende Fixierung eines Endoskops oder Overtubes durch Festklemmen des Ballons an der Darmwand häufig nicht möglich ist und hierdurch tiefere Untersuchungen nicht gelingen. Der Ballon kann leicht verrutschen, insbesondere ist eine ausreichende Fixierung bei weiten Darmlumina (Magen/Colon) nicht möglich. Wenn der Ballon zu sehr aufgepumpt wird, besteht das Risiko einer Darmwandverletzung bis hin zur Wandruptur.

Die Endoskopie-Anordnung nutzt diese Erkenntnis zur Ausbildung einer Endoskopie-Anordnung für die endoskopische intraluminale Vakuumschwammtherapie, um endoskopisch ein oder mehrere Fluidsammelelemente, beispielsweise Schwammdrainagen, beispielsweise intraluminal im Darmlumen zu platzieren und mit einem Vakuum gemäß den erfindungsgemäß vorgegebenen Parametern am Platzierungsort zu verankern. Die Schwammdrainagen saugen sich in diesem Beispiel mit dem am Schwamm angelegten Vakuum an der Darmschleimhaut fest und werden durch den Unterdruck am Platzierungsort fixiert.

Durch ein wechselseitiges gegenseitiges Verschieben eines Overtubes gegenüber einem in diesem eingeführten Endoskop ist es möglich, ein Endoskop im Darm vorzuschieben. Endoskop und Overtube benötigen hierzu eine Verankerung gegenüber dem anliegenden Gewebe z. B. der Darmschleimhaut. Diese Verankerung wird bei dem vorliegenden Ausführungsbeispiel der Endoskopie-Anordnung durch das Festsaugen der Schwammdrainage an der Darmschleimhaut erreicht. Daher wird die darauf basierende Behandlungs- oder Untersuchungsform auch als Vakuumendoskopie bezeichnet.

Die endoskopische Vakuumtherapie wird zur Behandlung von inneren Wunden eingesetzt. Ihre Effektivität konnte zunächst bei Nahtundichtigkeiten am Enddarm, dann auch bei Darmleckagen anderer Lokalisation wie Speiseröhre, Magen, Dünn- und Dickdarm nachwiesen werden. Bei inneren, unter der Hautoberfläche liegenden Wunden, Hohlräumen, Abszessen, Empyemen, Fisteln, die über eine Öffnung nach außen endoskopisch zugängig sind oder zugängig gemacht werden, kann die endoskopische Vakuumtherapie ebenfalls zur Wundbehandlung eingesetzt werden. Bei der endoskopischen Vakuumtherapie werden die natürlichen oder künstlichen Zugangswege zu Hohlorganen, Magen-Darmtrakt und Körperhöhlen endoskopisch genutzt. Es werden Schwammdrainagen unter Zuhilfenahme des Endoskops innerlich, intracorporal, intraluminal und intracavitär eingebracht. Bei der intraluminalen Therapievariante wird der Schwammkörper in einem Darmlumen in Defekthöhe platziert. Bei der intracavitären Variante wird der Schwammkörper durch den Defekt hindurch in eine (extraluminale) Wundhöhle eingebracht. Beide Therapien können auch kombiniert werden. Nachdem der Schwammkörper positioniert ist, wird an den ausgeleiteten Drainageschlauch ein Vakuumsog angelegt. Die Wundhöhle bzw. das Darmlumen kollabiert unter dem Sog gemeinsam mit dem elastischen Schwammkörper. Die Schwammoberfläche saugt sich an der Wundoberfläche saugnapfartig an, gleichzeitig fixiert sie sich durch den Sog am Platzierungsort. Es findet eine effektive Wunddrainage statt, gleichzeitig wird der Wunddefekt verschlossen. Unter der dauerhaften Drainagewirkung und Vakuumausübung an der Wundfläche reinigt sich die Wunde, es bildet sich Granulationsgewebe und die Wunde verheilt sekundär. Im mehrtägigen Intervall wird ein endoskopischer Wechsel der Schwammdrainage vorgenommen.

Eine Sonderform der endoskopischen Vakuumschwammtherapie zielt nicht auf einen kompletten Verschluss eines Hohlraumes, so wie oben dargestellt, sondern auf eine maximale Sekretableitung. Auch hierbei wird die Schwammdrainage in ein Hohlorgan z.B. dem Zwölffingerdarm (postpylorische Vakuum-Duodenalableitung) eingelegt und unter Sog gesetzt. Hierbei ist die Drainagewirkung so dosiert, dass kein vollständiger Darmverschluss erzielt werden muss, sondern das Fluidsammelmittel so weit unter einem Sog liegt, dass eine optimale Fluidleitung (im Beispiel einer duodenalen Platzierung von Pankreas- und Gallensekreten)aus dem Darmlumen erzielt wird. Es ist vorstellbar, diese Art der Anwendung auch in anderen Hohlorganen oder Hohlräumen anzuwenden, wo eine maximale Sekretableitung gewünscht wird.

Mit der Erfindung lässt beispielsweise auch eine komplette dauerhaft Entleerung des Magens erreichen. Durch die Erfindung ergeben sich zahlreiche innovative Therapiemöglichkeiten zur Therapie an inneren Wunden.

Nachfolgend werden Weiterbildungen der Endoskopie-Anordnung beschrieben.

Die Vakuumpumpe ist vorzugsweise durch ein oder mehrere Fluidkommunikationselemente in Form von Drainageschläuchen und/oder in Form eines Kanals im Endoskop, welcher auch wenigstens teilweise in oder an der Schwammdrainageeinheit angeordnet sein kann, mit der Schwammdrainageeinheit verbunden. Besonders bevorzugt ist das Fluidkommunikationselement durch Öffnungen in seiner Wandung fluidleitend mit dem Fluidsammelelement verbunden. Diese Perforationsöffnungen befinden sich besonders vorteilhaft in einem Abschnitt zwischen dem proximalen oder distalen Ende des Schlauches. Die Perforationsöffnungen befinden sich vorteilhaft im mittleren Abschnitt des Fluidkommunikationselementes. Die Perforationsöffnungen sind in einer Ausführungsform in mehreren Abschnitten zwischen dem proximalen und distalen Endes des Schlauches angeordnet. Die Perforationsöffnungen haben vorzugsweise einen Durchmesser von 1mm bis 10 mm. Über den Perforationsöffnungen der Schlauchwand kann von außen mit Klebung, Naht oder einer anderweitigen Befestigungsmöglichkeit das Fluidsammelmittel befestigt sein.

Solche Fluidkommunikationselemente sind in Weiterbildungen doppellumig oder sogar mehrkanälig ausgerüstet. Ein solches Fluidkommunikationselement ist zum Spülen und zum Absaugen über unterschiedliche Kanäle geeignet. Mindestens einer der Kanäle ist vorzugsweise in seinem Durchmesser so gestaltet, dass in das Fluidkommunikationselement auch eine drahtförmige Unterdruckmesssonde vorrübergehend oder dauerhaft eingeführt werden kann.

Besonders vorteilhaft kann eine Hälfte des Fluidkommunikationselementes dünnlumig sein und die andere Hälfte dicklumig sein. Dieses kann insbesondere dann vorteilhaft sein, wenn die Drainage so platziert werden kann, dass beispielsweise beim Vorliegen einer ösophagocutanen Fistel der eine Schenkel der Drainage percutan über die cutane Fistel nach außen ableitet und der andere Drainagenschenkel nach innen über die Speiseröhre nach oral ableitet. Die ausgeleiteten Schenkel des Fluidkommunikationselements können mit Klemmen verschlossen werden. Über das Fluidkommunikationselement kann insbesondere auch eine Spülbehandlung vorgenommen werden. insbesondere kann bei einer Platzierung des Fluidsammelelementes im mittleren Abschnitt und Ausleitung beider Fluidkommunikationsschenkel der eine Schenkel zur Saugung der andere zur Spülung benutzt werden.

Die verschiedenen Durchmesser des Fluidkommunikationselementes gehen vorteilhaft kontinuierlich konisch und ohne Stufung von dem dicklumigen zum dünnlumigen Durchmesser über. Hierdurch wird eine atraumatische Platzierung der Drainage sichergestellt. Die Perforationsöffnungen befinden sich insbesondere am distalen Ende des Schlauches. Um die Einführung des Schwammsystems zu erleichtern, kann in das Fluidkommunikationselement ein drahtförmiges Führungselement eingeführt werden.

Vorteilhafterweise sind die Fluidsammel- und Fluidkommunikationselemente röntgendicht.

Vorzugsweise haben die Fluidkommunikationselemente einen inneren Durchmesser von 1mm bis 10 mm. Bevorzugt hat das Fluidsammelelement in etwa Zylinderform mit einem Außendurchmesser von 5 mm bis 30 mm.

Größere Durchmesser des Fluidsammelelements sind beispielsweise vorteilhaft anwendbar, wenn ein Darmlumen mit einem großen Innendurchmesser (wie z.B. beim Magen oder Dickdarm) verschlossen werden soll. Kleinere Durchmesser des Fluidsammelelements und des Fluidkommunikationselements sind beispielsweise vorteilhaft anwendbar, wenn dünnlumige Fistelgänge verschlossen und drainiert werden sollen.

Der Außendurchmesser des Fluidkommunikationselements und des Fluidsammelelements sind in einer Ausführungsform an den Innendurchmesser eines inneren Arbeitskanals des Endoskops angepasst, so dass sie innerhalb des inneren Arbeitskanals verschieblich sind und ihre Platzierung über den inneren Arbeitskanal des Endoskops vorgenommen werden kann. Hierdurch wird insbesondere eine Platzierung der Drainage unter Sicht durch kleine Öffnungen erreicht. Weiterhin wird durch die Minimierung des Durchmessers erreicht, dass mit Hilfe der endoskopischen Techniken die Anzahl der Regionen vergrößert wird, welche endoskopisch zu erreichen sind und somit leicht mit einer Vakuumdrainageeinheit versorgt werden können.

In einer alternativen Variante sind der Außendurchmesser des Fluidkommunikationselements und des Fluidsammelelements an den Innendurchmesser eines äußeren Arbeitskanals des Endoskops angepasst, so dass sie innerhalb des äußeren Arbeitskanals verschieblich sind und ihre Platzierung über den äußeren Arbeitskanal des Endoskops vorgenommen werden kann.

Im Overtube sind in bevorzugten Ausführungsformen als Fluidkommunikationsmittel ein oder mehrere Drainagekanäle integriert. Sie sind zylindrisch. Diese Drainagekanäle sind unterdruckstabil, so dass sie unter dem applizierten Vakuum nicht kollabieren. Sie sind durch unterdruckstabile Drainageschläuche mit der Vakuumpumpe verbindbar. Insbesondere weisen die Drainagekanäle an ihrem distalen Ende in ihrer Wandung eine oder mehrere Öffnungen auf, welche fluidleitend den Overtube nach außen perforieren, so dass Flüssigkeiten und Gase durch Sog abgeleitet werden können. In Höhe der Öffnungen der Drainageelemente ist das Fluidsammelelement befestigbar oder befestigt, beispielsweise durch Klebung, Faden, oder Klemmung.

Das mit dem Fluidkommunikationselement fluidleitend verbundene Fluidsammelelement kann sowohl endoskopisch, laparoskopisch, thorakoskopisch, offen operativ intraluminal, intracavitär, intracorporal platziert werden. Die Schwarmmdrainageeinheit ist in einem Ausführungsbeispiel am distalen Ende des Endoskops und/oder am distalen Ende der Overtubeeinheit befestigt. Zur Platzierung von Schwammdrainagen in tiefer liegenden Körperregionen wie beispielsweise Dickdarm, Speiseröhre oder Zwölffingerdarm mit teilweise kurvenreichen Zugangswegen wird ein Drainageschlauch vorgeschlagen, an dessen Ende die Schwammdrainageeinheit in Form eines Polyurethanschwammkörpers angenäht ist. Bevorzugt hat die Schwammdrainageeinheit einen kreisförmigen oder hohlzylindrischen, also röhrenförmigen Grundkörper. Sie besteht beispielsweise aus einem offenporigen elastischen komprimierbaren Polypurethanschwammkörper. Bevorzugt ist eine Porengröße im Polypurethanschwammkörper von 200 µm bis 1000 µm, insbesondere bevorzugt ist eine Porengröße von 400 µm bis 600 µm. Der Schwamm kann durch Zuschneiden in Länge und Volumen den Erfordernissen angepasst werden.

In einer bevorzugten Ausführungsformt ist das Fluidsammelmittel eine offenporige Folie. Alternativ kann ein Polyurethanschwammkörper mit einer solchen offenporigen Folie überzogen sein. Die Folie kann beispielsweise nach Anpassen eines Längenmaßes des Schwamms, was typischerweise durch Schneiden geschieht, über den Schwamm gezogen werden. Hierzu ist die offenporige Folie verzugsweise als kleines Säckchen gestaltet und kann mit einem Faden zugebunden werden. Die Folie kann eine Struktur aus zwei Folienblättern aufweisen, die über ihre gesamte Fläche durch Poren fluidleitend verbunden sind.

Die Länge und Dicke des Fluidsammelelementes können wie schon erwähnt variabel gestaltet werden. Beispielsweise ist das Fluidsammelelement in verschiedenen Ausführungsformen zwischen 2 und 10 cm lang, es sind aber je nach Anwendungsfeld auch andere Längen möglich, wie unten angegeben. In anderen Ausführungsformen hat das Fluidsammelelement einen Außendurchmesser von 1,5 bis 3,0 cm. Auch hier können Anpassungen außerhalb dieses Wertebereiches für bestimmte Anwendungen sinnvoll sein. Für die intracavitäre Therapie beispielsweise ist das Fluidsammelelement bevorzugt 0,5 bis 1,5 cm durchmessend und 1 bis 4 cm lang. Für die intraluminale Therapie dagegen ist das Fluidsammelelement bevorzugt 1,5 bis 2,5 cm durchmessend und 4 bis 10 cm lang.

Bevorzugt hat der zentrale Kanal im Fluidsammelelement einen Durchmesser von 0,5 bis 1,0 cm, es sind aber je nach Anwendungsfall auch andere Durchmesser möglich.

Der Schwammkörper ist mit Greifzangen, Polypengreifer oder Schlingen greifbar und orthograd unter endoskopischer Führung einbringbar. Die Platzierung kann jedoch technisch schwierig sein. Die Sichtverhältnisse sind eingeschränkt. Die inneren Wundöffnungen, durch die beispielsweise bei der intracavitären Therapie der Schwammkörper eingelegt wird, sind häufig klein, abgewinkelt und schwer zugängig. Die Beweglichkeit des Endoskopes ist durch die Schwammdrainage eingeschränkt. Die zu endoskopierenden Räume sind beengt. Eine stumpf endende Schwammdrainage verhakt sich leicht sich an der inneren Wundöffnung oder Darmschleimhaut. Daher endet bevorzugt der Drainageschlauch distal mit einer Spitze.

Die Spitze der Drainage ist besonders vorteilhaft konisch sowie insbesondere weich und atraumatisch gestaltet, um eine Verletzung von anliegendem Gewebe zu vermeiden. Das spitz endende distale Ende des Drainageschlauchs kann über das distale Ende des Fluidsammelelementes hinausragen, es kann auch in dem Schwammkörper enden.

Eine konisch zusammenlaufende Konfiguration des Schlauchendes ist vorteilhaft in einem aufsitzenden Schwammkörper der Schwammdrainageeinheit fortgesetzt, so dass der Schwammkörper stufenlos der Drainage anliegt. Hierdurch wird das Platzierungsmanöver der Drainage erleichtert.

Auch die konische projektilartige Spitze des Drainageschlauchs ist in einer Ausführungsform mit einem zentralen Kanal versehen, so dass hierdurch ein Führungsdraht eingebracht werden kann.

Die Spitze kann vorteilhaft auch mit einem querverlaufenden Kanal ausgerüstet sein, durch den beispielsweise ein Faden gelegt werden kann. An dem distalen Ende des Drainageschlauchs, an dem Fluidsammelelement oder in dem Fluidsammelelement ist vorteilhaft eine Vorrichtung befestigt, die mit einer Zange, Haken, Schlinge oder anderem Legeinstrument gefasst werden kann. Insbesondere kann eine Faden- oder Drahtschlaufe befestigt sein. Insbesondere kann eine Greifperle aus Metall oder Kunststoff befestigt sein. Alternativ kann eine Metall- oder Kunststofföse befestigt sein. Oder es kann ein Faden befestigt sein. Der Faden kann beispielsweise 1 cm bis 250 cm lang sein.

Wenn noch ein zusätzlicher äußerer Zugang zur inneren Wunde besteht (beispielsweise in Form einer Fistel) kann der Faden in endoskopischer Technik über die Fistel von innen nach außen ausgeleitet werden. Bei einem Verlust der Spitze beim Legemanöver kann der Faden zur Bergung genutzt werden. Beim Vorliegen einer zusätzlichen Verbindung nach außen kann mit dem Legeinstrument oder dem gefestigten Faden auch in der (Durch-) Zugstechnik platziert werden. Die Wechselmanöver können durch Nutzung der Durchzugtechnik erheblich vereinfacht werden.

Die Vorrichtung, die mit einer Zange, Haken, Schlinge oder anderem Legeinstrument gefasst werden kann, ist insbesondere zugfest gestaltet, so dass die Drainage an diesen durch Gewebe, Darmluminal, Fisteln gezogen werden kann. Die Vorrichtung ist flexibel und atraumatisch zu gestalten.

Insbesondere vorteilhaft ist die Aufsatzspitze so gestaltet, das nach Aufbringen auf dem Ende des Drainageschlauches die Außenseite des Schlauches bündig mit der Außenseite der Aufsatzspitze abschließt.

Wenn keine Fistel zur Wunde nach außen besteht, kann durch eine Punktion von außen eine zusätzliche Verbindung nach außen geschaffen werden, über die der Faden ausgeführt werden kann. Der Faden kann desweiteren für endo, laparo-, thorakoskopische oder offen chirurgische Rendezvousmanöver genutzt werden. Das intraoperative Platzierungsmanöver kann hierdurch erheblich erleichtert werden.

Beispielsweise kann die Durchzugstechnik beim Legen einer Schwammdrainage in die Speiseröhre angewandt werden, wenn eine percutane endoskopische Gastrostomie zur Magenvorderwand angelegt wurde. Durch diesen percutanen Zugangsweg zum Magen kann ein Faden eingeführt und mit einem Gastroskop durch den Mund ausgeführt werden. Der Faden wird mit der Spitze der Schwammdrainage verbunden und dann durch Zug am Faden an den Platzierungsort in der Speiseröhre gezogen werden. Hierdurch können auch voluminösere und sehr lange Schwammkörper atraumatisch eingebracht werden. Die intraluminale Platzierung wird sehr vereinfacht.

Der Faden ist vorzugsweise so an dem Schwammkörper oder Drainageschlauch befestigt, dass er zu jedem Zeitpunkt entfernt werden kann. Dieses ist beispielsweise dann möglich, wenn der Faden als Doppelfaden oder Endlosschleife durch eine Fadenschlaufe oder Öse geführt wird, die an dem Ende des Drainageschlauches oder Schwammkörper, befestigt ist. Wenn der Faden entfernt werden soll, wird die Endlosschleife durchtrennt und gezogen.

Bevorzugt verläuft eine Längsachse der Schwammdrainageeinheit im Wesentlichen parallel zur Längsachse des Overtubes.

Bevorzugt umgreift ein in der Schwammdrainageeinheit (also dem Fluidsammelelement) ausgebildeter Kanal den gesamte Umfang des Overtubes, wobei ein die Schwammdrainageeinheit eingeführter Drainageschlauch dort Öffnungen in seiner Wandung aufweist. Das Fluidsammelelement kann jedoch alternativ auch nur partiell den Overtube umgreifen.

Das Fluidsammelelement ist vorteilhaft mit einer fluidleitenden Außenbeschichtung versehen, die das Gleiten gegenüber der Darmschleimhaut bei fehlendem Unterdruck erleichtert. Vorteilhaft ist diese Außenbeschichtung eine fluidleitende Folie. Bei der Vakuumendoskopie ist die Folienbeschichtung vorteilhaft hydrophil, so dass das Fluidsammelelement leichter auf der Schleimhaut gleiten kann. Es ist jedoch darauf zu achten, dass die Außenbeschichtung den auszuübenden Sog fluidleitend unvermindert, insbesondere mit einer möglichst großen Fläche auf die Darmschleimhaut leiten kann, so dass sich das Fluidsammelelement festsaugt und fixiert.

Die Sogwirkung der endoskopischen Vakuumtherapie kann sich an der Wundoberfläche nur bei offenen Poren des Schwamms im Innenbereich der Schwammdrainageeinheit entfalten. Wenn die Poren beispielsweise durch Schleim, Speichel oder zähes Sekret verstopft sind, kann sich eine Sogwirkung an der Wunde nicht entfalten. Insbesondere bei der intraluminalen Therapie der Speiseröhre kann der Schwammkörper teilweise oder ganz durch verschluckten zähflüssigen Speichel verstopfen. Bei einer teilweisen Verstopfung saugt sich der Schwammkörper nicht mit der ganzen Oberfläche, sondern nur teilweise mit den offenen Poren am Gewebe an. Wenn Poren durch Sekret verstopfen, kann sich der Schwamm an diesen Stellen nicht ansaugen. Es lässt sich beobachten, dass zwischen verstopfter Schwammoberfläche und Speisenröhrenschleimhaut Speichel und Sekret bis in den Magen entleeren kann, während hierbei gleichzeitig der Schwammkörper mit den noch offenen Poren an der Schleimhaut angesaugt ist.

Die zum Vakuumaufbau notwendige luftdichte Begrenzung besteht zum einen in dem Kontakt zur angesaugten Gewebeoberfläche, zum anderen in der Oberflächenversiegelung durch verstopfenden Schleim oder zähes Sekret. Unter diesen Bedingungen kann weiterhin ein effektiver Vakuumsog an umschriebener Schleimhaut oder Wundoberfläche bestehen bleiben. Wenn allerdings die Poren des Schwammkörpers vollständig durch zähes Sekret verstopft sind, kann sich keine Sogwirkung am Wundgrund entfalten, das Vakuum ist dann nur im fluldleitenden System vorhanden. Es tritt ein Therapiestillstand oder sogar eine Verschlechterung der Wundsituation ein.

Eine Ausführungsform der Endoskopie-Anordnung sieht daher vor, dass der Schwammkörper an seiner Außenfläche Aussparungen zur Aufnahme einer Sonde aufweist, die sich im Betrieb der Endoskopie-Anordnung zwischen Darmwand und Schwammkörper einlegen lässt. Eine solche zusätzliche Sonde kann zur enteralen Ernährung, Entlastung des Magens oder Spülung genutzt werden. An den Schwammkörper kann bei gleichzeitig einliegender zusätzlicher Sonde ein Vakuum angelegt werden. An denjenigen Stellen, an denen die zusätzliche Sonde zwischen Schwamm und Darmwand zu liegen kommt, entfaltet der Schwamm keine direkte Sogwirkung an der Darmwand. Hier lassen sich auch nicht die typischen schwamm- und sogbedingten Schleimhaut- bzw. Wundveränderungen beobachten. Beim direkten Schwammkontakt auf der Schleimhaut formt sich die Schleimhaut der Schwammoberfläche an, so dass die Schleimhaut noppenartig in den Poren des Schwammes anhaftet.

Das Fluidsammelelement ist in einer anderen Ausführung abschnittsweise mit einer Oberflächenversiegelung zum Verschluss der offenen Poren versehen. Die Oberflächenversiegelung kann durch einen elastischen Klebestoff vorgenommen sein, der in flüssiger Form oder als Spray auf die Oberfläche des Schwammes aufgetragen werden kann und hier elastisch aushärtet.

Hierdurch wird erreicht, dass von der versiegelten Oberfläche des Schwamms keine Sogwirkung auf die hier anliegende Schleimhaut oder Wundoberfläche erfolgt. Der Schwammkörper saugt sich dann nur mit der Oberfläche seiner offenen Poren an der Gewebeoberfläche an. Durch die Versiegelung kann weiterhin ein effektives lokales Vakuum aufgebaut werden. Bei entsprechend gezielter Platzierung des Schwammkörpers wird erreicht, dass der Vakuumsog und das saugnapfartige Festsaugen des Schwammkörpers nur an einem umschriebenen Gewebebereich vorgenommen wird. Hierdurch wird eine mögliche Verletzung durch den Vakuumsog auf Gewebe, welches nicht behandelt werden muss, vermieden. Gleichzeitig kann der lokale Vakuumsog am therapiebedürftigen Ort angelegt werden.

Bei einem zylindrischen Schwammkörper, der in eine Speiseröhre eingelegt werden soll, kann die Versiegelung vorteilhaft über ein Drittel oder die Hälfte der Oberfläche über die gesamte Länge des Schwammkörpers vorgenommen werden. In Abhängigkeit zur Konfiguration des Schwammkörpers sind verschiedene Muster zur Oberflächenversiegelung möglich. Bei der Platzierung eines teilweise versiegelten Schwammkörpers in der Speiseröhre kann erreicht werden, dass sich Speichel, Sekret, Flüssigkeiten zwischen der versiegelten Schwammoberfläche und der anliegenden nicht dem Vakuumsog ausgesetzten Schleimhaut auf physiologischem Weg entlang der Speiseröhre bis in den Magen entleeren kann. Es wird eine Speichelretention vermindert, es kann flüssige Ernährung ermöglicht werden. Es kann entlang der Versiegelung auch eine Emährungssonde zur enteralen Ernährung eingelegt werden.

Die Oberflächenversiegelung kann alternativ mit auf den Schwamm aufgeklebten elastischen Folien vorgenommen werden. Vorteilhaft können diese Folien in Längsrichtung profiliert sein, so dass sich Sekret entlang der Folien durch die Kapillarwirkung besser nach distal entleeren kann. Die Oberflächenversiegelung kann auch mit in Längsrichtung gehälfteten elastischen Röhren vorgenommen werden, die mit der konvexen Seite auf den Schwammkörper durch Klebung befestigt werden. Mit der an der konkaven Seite anliegenden Schleimhaut entsteht ein röhrenförmiger Tunnel, durch den sich Sekret entleerten kann, ohne dass es vom Schwammkörper angesaugt wird. Die genannten unterschiedlichen Arten der Oberflächenversiegelung können miteinander kombiniert werden.

Desweiteren ist in einer Ausführungsform zur Durchleitung von Sekreten mindestens ein röhrenartiger Tubus in den Schwammkörper integriert. Hiermit wird ein Sekretfluss (z. B. Speichelfluss zum Magen) durch den Schwammkörper unter Vakuumsog ermöglicht. Ein vorzeitiges Verstopfen der Schwammporen durch zähes Sekret wird verhindert oder verzögert, damit das Vakuum seine Wirkung am Wundgrund bzw. Schleimhaut besser und langfristiger entfalten kann. Gleichzeitig kann bei einer Behandlung an der Speiseröhre eine Retention von Speichel verhindert werden und eine enterale Ernährung bei der intraluminalen Vakuumtherapie ermöglicht werden. Durch diese Ausführungsform ergeben sich zahlreiche neue Therapiemöglichkeiten.

Das Fluidsammelelement soll besonders vorteilhaft mit einem weiteren kompletten Kanal in Längsrichtung ausgerüstet sein. Durch diesen Kanal kann eine weitere Sonde eingeführt werden.Es kann besonderes vorteilhaft ein röhrenartiger Tubus eingeführt werden, der das Sammelelement in ganzer Länge und darüber hinausragend an den Enden durchtritt. Der Tubus ist in einer anderen Ausführungsform gleich lang wie das Fluidsammelelement, typischerweise einem Schwammkörper. Er ist nicht fluidleitend mit dem Schwammkörper verbunden.Er kann sowohl am proximalen Ende als auch am distalen Ende mit einer tulpenförmigen Aufweitung versehen sein. Der Tubus kollabiert nicht, wenn das Vakuum angelegt wird, ist also unterdruckstabil. Der Tubus ist biegsam, ohne dass er abknickt. Der Tubus dient als Fluiddurchleitelement für zähflüssige Sekrete wie z.B. Speichel oder Stuhl. Wenn diese Sekrete durch den unter Vakuumsaugung stehenden Schwammkörper hindurchgeleitet werden, wird hierdurch eine Verstopfung der Schwammporen verhindert werden, gleichzeitig kann die Vakuumausübung am Wundgrund beibehalten werden. In das Fluiddurchleitelement können auch Sonden, endoskopische Instrumente, ein Führungsdraht oder ein elastischer Lege- und Platzierungsstab eingeführt werden. Insbesondere kann auch ein Endoskop eingeführt werden. Insbesondere kann auch ein Endoskop als Leitelement zum Legen eines Vakuumsystems mit Fluiddurchleitelement benutzt werden. Dass das Endoskop selbst als Leitschiene für die Schwammdrainage genutzt werden kann, erleichtert das Manöver erheblich, man hat volle endoskopische Kontrolle und Sicht und spart Arbeitsschritte bei der Platzierung des Fluidsammelelements. Das Endoskop muss nicht aus dem Körper entfernt werden. Das Endoskop hat hierfür vorzugsweise einen Durchmesser zwischen 5 und 10 mm.

Es hat sich besonders vorteilhaft gezeigt, dass bei dieser Art von Konstruktion die Abdichtung bei Anwendung in der Speiseröhre zu einem besonders innigen, vollständig circulären Ansaugen über die gesamte Länge des Schwammes gelingt und somit eine sehr gute und sichere Abdeckung eines Defekts in der Speiseröhre bei gleichzeitig effektiver Drainage am Wundgrund sicher möglich ist. Vorteilhafterweise ist gleichzeitig eine physiologisch orale enterale Ernährung mit dieser Ausführungsform möglich. Dies sind deutliche Vorteile gegenüber dem im Stand der Technik praktizierten alleinigen Stenting mit seibstexpandierenden gecoverten Stents, welches über eine Expansionskraft nach außen die Defektdeckung erzielen soll.

Der Tubus kann mittels Naht, Klebung oder anderweitig im Schwammkörper fixiert sein. Es ist jedoch keine spezielle Fixierung des Tubus innerhalb des Kanals des Fluidsammelmittels notwendig. Im Gegenteil, wenn keine Fixierung vorgenommen wird, ist dies besonders vorteilhaft. Denn dann kann bei einem Entfemungsmanöver der Tubus leicht unabhängig von dem Schwammkörper aus diesem entfernt werden. Dies ist insbesondere dann vorteilhaft, wenn der Schwammkörper sehr fest an der Darmwand anhaftet und mechanisch mit einem Endoskop von der Wand gelöst wird. Bei angelegtem Sog wird der Tubus durch den Vakuumsog im Schwammkörper fixiert.

Zur Platzierung eines mit einem solchen Fluiddurchleitelement ausgerüsteten Vakuumdrainagesystems (d. h. die Schwammdrainageeinheit, ggf. mit Overtube,) kann ein Puscher verwandt werden. Der Pusher weist eine Röhre auf, in die ebenfalls ein Lege- und Platzierungsstab oder ein Endoskop eingeführt werden kann. Der Pusher kann gleitend auf diesen Leitelementen bewegt werden. Mit dem Pusher kann ein Vakuumdrainagesystem nach distal verschoben und somit am Platzierungsort von dem Leitelement getrennt werden. Vorteilhaft ist der Pusher wie das Vakuumdrainagesystem mit einem Längsschlitz versehen, so dass sie zu jedem Untersuchungszeitpunkt auf einem Endoskop seitlich aufgesetzt oder entfernt werden können.

Vorteilhaft sind das distale und proximale Ende des Fluiddurchleitelements radiär geteilt und sind gegenüber einem mittleren Tubusteil des Fluiddurchleitelements scharnierartig oder flügelartig nach außen beweglich. Beim Verschieben auf dem Leitelement mit dem Pusher liegen alle Abschnitte des Fluiddurchleitelements diesem an. Wenn der Vakuumsog an den Schwammkörper angelegt wird, kollabiert der Schwammkörper, zieht sich zusammen und saugt sich an der Darmwand an. Gleichzeitig klappen hierdurch scharnierartig die beweglichen Enden des Fluidsammelements auseinander und spreizen sich tulpenartig auf. Die Vakuumdrainage verankert sich hierdurch zusätzlich zum Ansaugen am Platzierungsort nach proximal und distal. Durch die tulpenartige Aufspreizung kann sich Speichel und/oder Sekret leichter in dem Fluiddurchleitelement ansammeln und durch das Fluidsammelelement hindurchgeleitet werden, ohne angesaugt zu werden. Besonders vorteilhaft kann diese Ausführungsform angewandt werden, um physiologisch anfallendes Sekret wie (je nach Anwendungsort) Speichel, Dünndarmstuhl oder Dickdarmstuhl oder Luft durchzuleiten. Vorteilhaft gegenüber einem kompletten Verschluss von Darmlumen durch die Vakuumtherapie ist mit dieser Ausführungsform im Fall einer Speiseröhrenbehandlung gleichzeitig zur Vakuumtherapie eine physiologisch orale enterale Ernährung und/oder die Einlage von Emährungs- oder Magenentlastungssonden möglich. Bei der Behandlung am Dickdarm wird erreicht, dass sich Stuhl entleeren kann und die Anlage eines künstlichen Darmausganges verhindert werden kann.

Die Enden des Fluiddurchleitelements können auch aus einer elastischen Folie oder anderen Oberflächenversiegelung bestehen.

Es kann alternativ zur Verwendung des Tubus ein in dem Schwammkörper gelegener Kanal mit einer Oberflächenversiegelung ausgerüstet sein. Vorteilhaft ist diese innere Oberflächenversiegelung aus einer längsprofilierten Folie gefertigt, an der entlang sich ebenfalls Sekret durch die Kapillarwirkung bevorzugt drainiert und so eine Verstopfung der Schwammkörpers am Darmwandkontakt verhindert. Vorteilhaft erstreckt sich die Oberflächenversiegelung auf proximales und distales Ende des Schwammkörpers.

Der Overtube bildet in einer Ausführungsform eine der Längserstreckung des Endoskops in Richtung von proximal nach distal (nachfolgend Längsrichtung) angepasste, biegsame Kunststoffhülse, in die das Endoskop eingeführt werden kann. Die Länge ist für die Vakuumendoskopie vorteilhafterweise so zu wählen, dass der Overtube ca. 20-80 cm kürzer als das Endoskop ist. Über diese Längendifferenz können beide in der Längsrichtung gegeneinander hin und her verschoben werden. Der Overtube kann in unterschiedlichen Längen und Durchmessern konstruiert werden. Er ist vorteilhaft auch aus einem Material konstruiert, das es erlaubt, seine Länge z.B. durch Abschneiden am proximalen und/oder distalen Ende individuell der Länge des Endoskops anzupassen. Bevorzugt ist der Overtube zwischen 80 und 160 cm lang. Es sind aber auch andere Längen möglich.

Der Innendurchmesser des Overtubes ist vorzugsweise nur wenig weiter als der Außendurchmesser des Endoskops, so dass beide leicht gegeneinander verschiebbar sind und der Gesamtdurchmesser nicht zu groß wird. Bevorzugt ist der Innendurchmesser 8 mm bis 15 mm weit. Es sind aber auch andere Innendurchmesser möglich. Bevorzugt ist der Außendurchmesser 10 bis 25 mm weit, es sind aber auch andere Außendurchmesser möglich.

Zum besseren Gleiten kann ein Gleitmittel eingesetzt werden. Bevorzugt sind die Außenhülle des Endoskops, Innenseite und Außenseite des Overtubes mit einem gleitfähigen, insbesondere zusätzlich hydrophilen Material beschichtet. Vorteilhaft weist der Overtube am proximalen Ende eine trichterförmige Erweiterung auf, damit ein Endoskop leichter eingeführt werden kann. Vorteilhaft ist am proximalen Ende des Overtubes ein ventilartiger Verschluss vorgesehen, durch welchen das Einführen eines Endoskops möglich ist, ein Entweichen von Untersuchungsgas oder Sekreten verhindert wird. Vorteilhaft verjüngt sich am distalen Ende das Lumen, so dass es dem Endoskop anliegt und hierdurch eine Stufenbildung verhindert, die ein Vorschieben der gesamten Einheit erschweren würde, bzw. das Gleiten gegenüber dem Endoskop erleichtert wird.

Der Overtube hat in einer Ausführung unmittelbar proximal und distal des aufsitzenden Fluidsammelelements eine ringförmige lippenartige Verdickung, so dass bei einem Sogaufbau proximal und distal des Schwammes an der Verbindung von der Lippe zur Darmwand eine innigere Verbindung und somit eine bessere Abdichtung entsteht, die den Vakuumaufbau am Schwamm erleichtert. Bevorzugt sind die ringartigen Aufwulstungen elastisch gefertigt. Bevorzugt sind die Aufwulstungen ebenfalls wie der Overtube geschlitzt.

Eine Ausführungsform der Schwammdrainage hat eine Trägerhülse. Sie ist so konstruiert, dass sie auf den Overtube und/oder auf das Endoskop aufgesetzt und wieder abgenommen werden kann. Sie ist insbesondere so konstruiert, dass sie fluidleitend den im Overtube und/oder Endoskop verlaufenden Drainageschlauch und das Fluidsammelelement, also den Schwamm der Schwammdrainage,verbindet. Bevorzugt ist die Trägerhülse gemeinsam mit dem Fluidsammelelement mit einer Klebung, Klebestreifen, Gummiband, Faden oder einer anderen Befestigungsmöglichkeit über den Saugöffnungen auf dem Overtube/dem Endoskop befestigbar oder in ihrer Produktion bereits entsprechend befestigt. Die Nutzung des Overtubes/Endoskops ist jedoch je nach Anwendung wahlweise mit oder ohne Fluidsammelelement möglich.

Der Overtube und das aufsitzende Fluidsammelelement und die Trägerhülse sind bevorzugt über ihre gesamte Länge längsgeschlitzt. Der Längsschlitz bietet den Vorteil, dass der Overtube zu jedem Zeitpunkt einer endoskopischen Untersuchung auf einem Endoskop angebracht und auch wieder entfernt werden kann. Dieser Schlitz kann durch Klebung, Klebeband, Faden, Reißverschluss oder eine andere technische Möglichkeit verschlossen werden. Der Verschlussmechanismus ist bevorzugt so konstruiert, dass er wiederholt geöffnet und verschlossen werden kann.

Besonders bevorzugt weist die Trägerhülse an ihrem proximalen und distalen Ende ringartige lippenförmige Aufwulstungen auf. Auch am proximalen und/oder am distalen Ende des Fluidsammelelementes kann eine ringförmige Lippe befestigt sein. Bevorzugt ist diese Lippe durch eine Klebung am Fluidsammelelement befestigt. Bevorzugt ist die ringförmige lippenartige Aufwulstung durch eine stabile Verpressung und Verklebung aus dem Fluidsammelmittel geformt. Das Fluidsammelelement ist auf den Overtube von seitlich aufsetzbar.

In einer besonderen Ausführung besteht das Fluidsammelelement aus einer offenporigen dünnen fluidleitenden Folie. Diese hat insbesondere den Vorteil, dass der Durchmesser des Overtubes im Bereich des Fluidsammelelements nicht wesentlich vergrößert ist und das hierdurch der Overtube ungehindert gleiten kann. Es ist jedoch darauf zu achten, dass die offenporige Folie den auszuübenden Sog fluidleitend unvermindert weiterleiten kann, so dass sich das Fluidsammelelement festsaugt und fixiert.

In einer Variante ist der Overtube ausgebildet, mehrere Fluidsammelelemente und Drainageschläuche in unterschiedlichen Längsabschnitten aufzunehmen. Hierdurch wird vorteilhaft erreicht, dass die Verankerung des Overtubes nicht nur am distalen Ende erfolgt, sondern auch noch an anderen Lokalisationen entlang des Overtubes.

In oder an dem Overtube können weitere Arbeitskanäle vorgesehen sein, die von proximal bis nach distal längs in dem Overtube verlaufen. Sie können ebenfalls wie der Overtube längsgeschlitzt, zum Öffnen und Verschließen konstruiert sein. Diese Arbeitskanäle lassen sich zur Spülung/Absaugung oder Einführen von Instrumenten nutzen.

Der Overtube und auch das Endoskop sind vorzugsweise mit Messmarkierungen versehen, so dass zum einen die Eindringtiefe bestimmt werden kann, zum anderen aber auch gemessen werden kann, in wieweit beide gegeneinander verschoben werden.

Die Vakuumenteroskopie kann mit konventionellen Endoskopen durchgeführt werden. Bei Nutzung eines konventionellen Endoskops mit einem Vakuumschwammovertube wird nur die Vakuumverankerung durch das Vakuum am Fluidsammelmittel des Overtubes genutzt.

Die Länge des Overtubes ist kürzer als das Endoskop zu wählen, so dass eine Verschieblichkeit gegenüber dem Endoskop möglich ist. Bevorzugt hat das Endoskop eine Länge zwischen 120 und 220 cm, es sind aber auch andere Längen möglich. Bevorzugt hat das Endoskop einen Außendurchmesser von 8 bis 12 mm. Es sind aber auch andere Außendurchmesser möglich.

Zur Nutzung des Vakuums über einer Schwammdrainage am Endoskop sind speziell konstruierte Endoskope erforderlich, die im Folgenden beschrieben werden:
In das Endoskop integriert sind vorzugsweise ein oder mehrere Fluidkommunikationselemente. Diese sind vorzugsweise als unterdruckstabile Kunststoffkanäle in einer Wandung des Endoskops ausgebildet, welche besonders bevorzugt am distalen Ende des Endoskops mit einer oder mehreren Perforationsöffnungen die Außenhülle des Endoskops fluidleitend perforieren und hier endogen. Diese unterdruckstabilen Saugkanäle sind mit unterdruckstabilen fluidleitenden Verbindungen mit der Vakuumpumpe verbunden, so dass Flüssigkeiten und Gase durch Sog abgeleitet werden können. Das Fluidkommunikationselement (der Kanal) im Endoskop ist bevorzugt zylindrisch. Bevorzugt ist der Kanal parallel zu einer Längsachse des Fluidsammelelementes angeordnet.

Ein solches Endoskop kann mit oder ohne Fluidsammelelement benutzt werden. Mit dem speziellen Endoskop können auch konventionelle Untersuchungen vorgenommen werden.

Bei Verwendung eines Fluidsammelelements ist dieses vorzugsweise in Höhe der Öffnungen der Fluidkommunikationselemente durch Klebung, Faden, Klemmung oder eine alternative Befestigungsmöglichkeit befestigt. Bevorzugt umgreift der Kanal des Fluidsammelelements den gesamten Umfang des Endoskops in Höhe der Öffnungen der Fluidkommunikationselemente. Das Fluidsammelelement kann auch nur partiell das Endoskop umgreifen. In einer besonderen Ausführung besteht das Fluidsammelelement aus einer offenporigen dünnen fluidleitenden Folie. Diese hat insbesondere den Vorteil, dass der Durchmesser des Endoskops im Bereich des Fluidsammelelements nicht wesentlich größer ist und das hierdurch das Endoskop ungehindert gleiten kann. Es ist darauf zu achten, dass die offenporige Beschichtung den auszuübenden Sog fluidleitend unvermindert leiten kann, so dass sich das Fluidsammelelement festsaugt und fixiert.

Bevorzugt fällt die Längsachse des Fluidsammelelements im Wesentlichen mit der Längsachse des Endoskops zusammen, oder ist zumindest parallel zu ihr. Ferner ist bevorzugt, dass der Kanal parallel zu einer Symmetrieachse des Fluidsammelelementes angeordnet ist.

Über Arbeitskanäle können endoskopische Instrumente im Endoskop bis zum distalen Ende des Endoskops geführt werden. Mit diesen Instrumenten können operative Eingriffe, z. B. eine Gewebeentfernung, unter endoskopischer Sicht vorgenommen werden. Das Endoskop kann beispielsweise einen oder 2 Arbeitskanäle aufweisen. Diese inneren Arbeitskanäle sind durch die Anordnung innerhalb des Endoskops sehr kleinkalibrig, um einen möglichst geringen Gerätedurchmesser für das Endoskop zu erzielen.

Ein bevorzugtes Ausführungsbeispiel sieht eine Führungshülse vor, die am Endoskop, beispielsweise an dessen Distalende befestigt ist und eine zusätzliche äußere Einführhilfe für endoskopische Instrumente oder Hilfsmittel und/oder einen Spül- und Absaugkanal bereitstellt. Die Hülse ist ein formstabiler Schlauch oder eine schlauchartige Struktur, der bzw. die nicht kollabiert oder abknickt. Sie ist flexibel, damit sie den Bewegungen des Endoskopes folgen kann. Ein weiterer Vorteil gegenüber den innenliegenden Führungskanälen liegt darin, dass ein äußerer Führungskanal einen größeren Durchmesser besitzen kann. Mit dieser Führungshülse ist das Endoskop mit zusätzlichen äußeren Arbeitskanälen ausgestattet und ermöglicht hierdurch, die endoskopischen Behandlungsmöglichkeiten zu erweitern.

Beispielsweise am proximalen Ende kann die Führungshülse mit einem Ventil verschlossen sein, um das Entweichen eines Untersuchungsgases zu vermeiden.

Die Führungshülse erlaubt in unterschiedlichen Ausführungsformen eine gleichzeitige Befestigung von einem oder mehreren äußeren Führungskanälen. Sie kann in unterschiedlichen Durchmessern gefertigt werden.

Die Befestigungshilfe auf dem Endoskop kann in Form einer das Endoskop umgreifenden Hülse, einem Gummi, einem Klebestreifen oder einer anderweitigen Befestigungsvorrichtung ausgebildet sein. Die Befestigungshilfe kann so konstruiert sein, dass auch eine Entfernung der äußeren Führungshilfe bei dem einliegenden Endoskop möglich wäre.

Auch durch die Möglichkeit der Entfernbarkeit der Führungshülse ergeben sich neue endoskopische Behandlungsmöglichkeiten. Es kann beispielsweise die äußere Einführhilfe auch genutzt werden, um einen Führungsdraht für andere endoskopische Hilfsmittel vorzuschieben. Nach der Platzierung des Führungsdrahtes lässt sich der äußere Arbeitskanal entfernen und über den Führungsdraht unter optischer Kontrolle des einliegenden Endoskopes z. B. ein Stent einführen. Das Endoskop muss zur Durchführung der Prozedur nicht entfernt werden. In einer besonderen Ausführung der Vakuumdrainage kann diese (analog wie bei dem inneren Arbeitskanal des Endoskopes) auch direkt durch das Lumen des äußeren Arbeitskanals am Platzierungsort eingelegt werden.

Vorteilhaft ist das Lumen des äußeren Arbeitskanals weiter als bei einem inneren Arbeitskanal, so dass unter Nutzung der Vorzüge der direkten endoskopischen Führung eine Vakuumdrainage voluminöser sein kann.

In einer besonderen Ausführungsform ist der Arbeitskanal distal mit seitlichen Perforationsöffnungen ausgerüstet und mit einem Fluidsammelelement verbunden und kann so selbst als Schwammdrainage eingesetzt werden.

Die Einführhülse kann auch in Längsrichtung geschlitzt gefertigt sein. Hierdurch besteht die Möglichkeit, dass ein durch die Hülse eingeführtes Instrument bei liegendem Endoskop seitlich aus der Hülse ausgelöst werden kann und weitere lösbare Instrumente über die geschlitzte Einführhilfe eingebracht werden können.

Nachfolgend werden Ausführungsbeispiele beschrieben, die die Unterdruckmesssonde fortbilden. Sie wird im Rahmen dieser Anmeldung mit gleicher Bedeutung auch als Messfühler bezeichnet.

Der Messfühler kann sowohl bei der Vakuumschwammtherapie an äußeren sichtbaren Wunden als auch bei von außen nicht zu sehenden intracorporale Wunden angelegt werden, um das Vakuum zu messen, welches an der Wunde tatsächlich anliegt. Der Messfühler kann bei der Wundversorgung mit dem Fluidsammelelement eingelegt werden.

Der oder die Messfühler sind entweder drahtgebunden oder drahtlos mit der Druck-Regeleinheit des Vakuumsystems, in einer Variante mit der Vakuumpumpe direkt verbunden, so dass voreingestellte erforderliche zu erzeugende Unterdruckwerte der Pumpe kontrolliert und reguliert werden können. Der Messfühler kann insbesondere in unterschiedlichen Ausführungsformen auf dem Polyurethanschwamm aufgelegt werden, in dem Schwamm angelegt sein, oder zwischen Schwamm und Drainage angeordnet sein. Er kann aber auch innerhalb des fluidleitenden Systems des Drainageschlauchs angeordnet sein.

Bevorzugt sind mehrere Messfühler zur Messung des hergestellten Unterdrucks vorhanden. Wenn mehrere Messfühler vorhanden sind, können diese auch an unterschiedlichen Orten Messungen durchführen und ableiten, beispielsweise am unterdruckseitigen Ausgang der Pumpe, im Sekretbehälter, am Fluidsammelelement, oder in einem Fluidkommunikationselement.

In einer Ausführungsform ist zumindest einer der Messfühler in dem Pumpensystem fest integriert. Alternativ oder zusätzlich ist zumindest ein Messfühler nachträglich z.B. in ein Fluidkommunikationselement einführbar konstruiert.

Die Drainageeinheit kann hierbei so konstruiert sein, dass der Messfühler bereits von vornherein in das System integriert ist, er kann aber auch nachträglich, nachdem das Fluidsammelelement in der Wunde eingelegt wurde an/in dem Fluidsammelelement angelegt werden. Er kann hierzu in dem Fluidkommunikationselement bis auf das Fluidsammelelement oder die Wunde geführt werden, oder er kann in einem zweiten Fluidkommunikationselement gesondert zum Wundort geführt werden. Es ist auch möglich das Fluidkommunikationselement zu diesem Zweck mit einem zweiten Lumen auszustatten. Vorteilhaft ist hierbei, wenn die Messsonde drahtförmig konstruiert ist, so dass man sie wie einen endoskopischen Führungsmandarin leicht auch in einem kleinen Lumen vorschieben kann.

Bevorzuge Ausführungsformen der Erfindung werden im Folgenden anhand von Figuren erläutert.

Weitere bevorzugte Ausführungsformen der Erfindung werden nachfolgend anhand der beiliegenden Figuren nach Aufbau und Handhabung erläutert.
- Figur 1a: ist eine schematische Darstellung eines Ausführungsbeispiels eines Vakuumsystems;
- Fig. 1b: ist ein Blockschaltbild mit näheren Details der Druck-Regeleinheit des Vakuumsystems der Fig. 1a
- Figur 2: ist eine teilweise Längsschnittdarstellung des Vakuumsystems der Figur 1a;
- Figur 3: ist eine schematische Darstellung eines anderen Ausführungsbeispiels eines Vakuumsystems;
- Figur 4: ist eine schematische Darstellung einer Anordnung eines Fluidsammelelementes;
- Figur 5: ist eine schematische teilweise Längsschnittdarstellung der Anordnung der Figur 4;
- Figur 6: ist eine Längsschnittdarstellung eines Fluidsammelelements 64, welches fluidleitend mit zwei Fluidkommunikationselementen 63 verbunden ist,
- Figur 7: ist eine Längsschnittdarstellung eines Fluidsammelelements, in welchem sowohl fluidleitend ein Fluidkommunikationselement und auf diesem aufliegend eine drahtförmige Unterdruckmesssonde angeordnet ist;
- Figur 8: zeigt eine Ausführung eines längsgeschlitzen Overtubes;
- Figur 9: ist eine Längsschnittdarstellung von Figur 8;
- Figur 10: ist eine Querschnittdarstellung eines Overtubes;
- Figur 11: ist eine Querschnittdarstellung einer anderen Variante eines Overtubes;
- Figur 12: zeigt die eine weitere Ausführung eines Overtubes;
- Figur 13: ist eine Querschnittdarstellung des Overtubes der Figur 12;
- Figur 14: ist eine andere Darstellung der Ausführung von Figur 12 und 13;
- Figur 15: ist eine Längsschnittdarstellung von Figur 14;
- Figur 16: ist eine Darstellung eines Overtubes, der eine Variante des Overtubes der Figuren 12 bis 15 bildet;
- Figur 17: ist eine Darstellung einer weiteren Variante eines Overtubes;
- Figur 18: ist eine Längsschnittdarstellung des Overtubes der Figur 17;
- Figur 19 Figur 17: zeigt eine Variante der Darstellungen der Ausführungsformen von und Figur 18;
- Figur 20: ist eine Längsschnittdarstellung des Overtubes von Figur 18 und 19;
- Figur 21: ist eine weitere Längsschnittdarstellung des Overtubes der Figuren 18 bis 20;
- Figur 22: ist eine Darstellung eines distalen Endes eines Endoskops;
- Figur 23: ist eine Längsschnittdarstellung des Endoskops der Figur 22;
- Figur 24: ist eine weitere Längsschnittdarstellung des Endoskops von Figur 22;
- Figur 25: ist eine Darstellung eines Fluidsammelelementes, das für die Verwendung am Overtube, Endoskop und der Trägerhülse geeignet ist ;
- Figur 26 25;: ist eine Längsschnittdarstellung des Fluidsammelelements von Figur
- Figur 27: ist eine Darstellung eines anderen Fluidsammelelements;
- Figur 28: ist eine Längsschnittdarstellung von Figur 27;
- Figur 29: ist eine Darstellung einer Trägerhülse für ein Fluidsammelelement;
- Figur 30: ist eine Längsschnittdarstellung der Trägerhülse der Figur 29;
- Figur 31: ist eine Darstellung einer Trägerhülse mit einem auf diesem zwischen lippenartigen Ringen befestigten längsgeschlitzten Fluidsammelelement;
- Figur 32: ist eine Längsschnittdarstellung der Trägerhülse von Figur 31;
- Figuren 33 a-i: zeigen verschiedene Varianten von Querschnittprofilen von lippenartigen Ringabschlüssen;
- Figur 34: ist eine Darstellung zur Erläuterung, wie ein flexibles Endoskop über den Längsschlitz des Overtubes eingebracht oder entfernt wird;
- Figur 35: zeigt eine Endoskopie-Anordnung gemäß einem weiteren Ausführungsbeispiel;
- Figur 36: a-n zeigen eine schematische Darstellung des Untersuchungsganges einer Videoendoskopiebehandlung;
- Figur 37: ist eine Darstellung einer Vakuumdrainage mit teilweiser Oberflächenversiegelung des Schwammkörpers;
- Figur 38 37;: ist eine Längsschnittdarstellung des Fluidsammelelements von Figur
- Figur 39: ist eine Darstellung einer anderen Ausführungsform einer Vakuumdrainage;
- Figur 40: ist eine Längsschnittdarstellung der Vakuumdrainage von Figur 39 ;
- Figur 41: ist eine Darstellung einer Vakuumdrainage mit einer profilierten Oberflächenversiegelung;
- Figur 42: ist eine Querschnittdarstellung der Vakuumdrainage von Figur 41;
- Figur 43: ist eine Darstellung einer Vakuumdrainage mit einem in einem Schwammkörper befestigen Tubus ;
- Figur 44: ist eine Darstellung einer anderen Ausführungsform einer Vakuumdrainage mit einem in einem Schwammkörper befestigten Tubus;
- Figur 45: ist eine Längsschnittdarstellung der Vakuumdrainage der Figur 43;
- Figur 46: ist eine Darstellung einer weiteren Ausführungsform einer Vakuumdrainage mit einem Drainageschlauch in einem Schwammkörper;
- Figur 47: ist eine Längsschnittdarstellung einer weiteren Vakuumdrainage mit im Schwammkörper einliegendem Tubus ;
- Figur 48: ist eine Darstellung der Vakuumdrainage aus Figur 47, wobei in dieser Darstellung an den Drainageschlauch ein Unterdruck angelegt ist;
- Figur 49: ist eine Darstellung einer weiteren Ausführungsform einer Schwammdrainage,
- Figur 50: ist eine Längsschnittdarstellung der Schwammdrainage aus Figur 49;
- Figur 51: ist eine Darstellung einer weiteren Ausführungsform einer Schwammdrainage;
- Figur 52: ist eine Längsschnittdarstellung der Schwammdrainage von Figur 51;
- Figur 53: ist eine Darstellung einer weiteren Ausführungsform einer Schwammdrainage;
- Figur 54: ist eine Längsschnittdarstellung der Schwammdrainage von Figur 53;
- Figuren 55a bis h: zeigen verschiedene Varianten eines distalen Endes einer Schwammdrainage in jeweiligen Längsschnittdarstellungen.
- Figuren 56a bis f: sind verschiedene Darstellungen eines Drainageschlauches und Aufsatzspitzen;
- Figuren 57a bis f: sind verschiedene Darstellungen eines endoskopischen Legeinstrumentes,
- Figuren 58a bis e: sind verschiedene Darstellungen eines weiteren endoskopischen Legeinstrumentes;
- Fig. 59: ist eine Darstellung einer Einführhilfe mit einer Hülse zur Befestigung auf einem Distalende eines Endoskops;
- Fig. 60: ist eine Darstellung zweier Einführhilfen unterschiedlichen Kalibers;
- Fig. 61: zeigt einen Querschnitt einer Einführhilfe, und einer Befestigungshülse mit Ventil;
- Fig. 62: zeigt eine Darstellung einer Einführhilfe mit einer Befestigungshülse auf einem distalen Ende eines Endoskops; und
- Fig. 63: ist eine Darstellung einer Einführhilfe mit einer Befestigungshülse auf einem distalen Ende eines Endoskops.

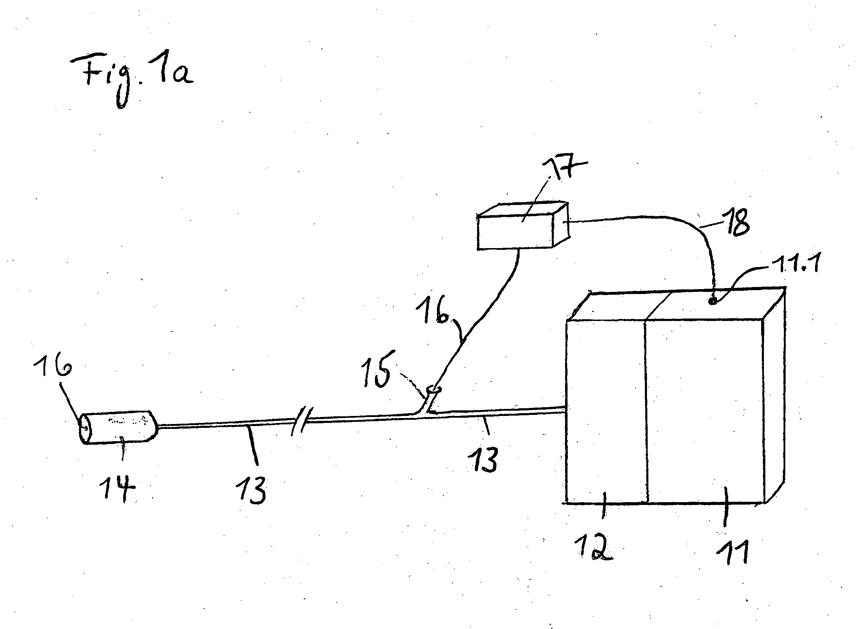

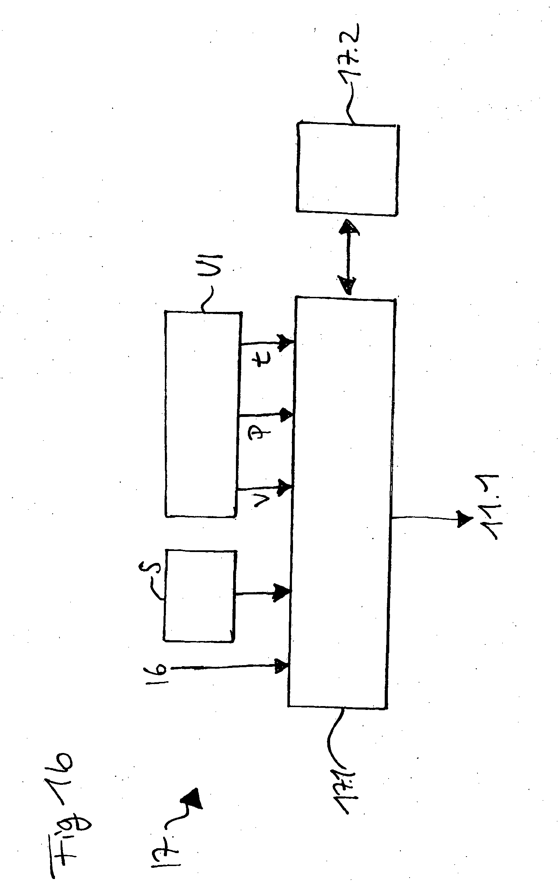

Figur 1a ist eine schematische Darstellung eines Ausführungsbeispiels eines Vakuumsystems mit einer Vakuumpumpe 11, einem Sekretbehälter 12 an der Pumpe, einem Fluidkommunikationselement 13, welches von der Vakuumpumpe zu einem Fluidsammelelement 14 führt. In das Fluidkommunikationselement ist über einen seitlichen Eingang 15 eine Unterdruckmesssonde 16 eingeführt, welche über eine Druck-Regeleinheit 17 Messwerte zur Regulierung, Voreinstellung und Steuerung elektronisch über Verbindungselemente 18 an die Vakuumpumpe übermittelt. Mit der Druck-Regeleinheit verbunden, jedoch hier nicht näher dargestellt (vgl. jedoch Fig. 1b), ist eine Nutzereingabeeinheit. Die Druck-Regeleinheit 17 hat einen Messsignaleingang zum Empfang von Messsignalen der Unterdruckmesssonde 16 und ist ausgebildet, die Vakuumpumpe 11 im Betrieb zur Herstellung und Aufrechterhaltung eines Vakuums mit einem vorbestimmten Unterdruck von in diesem Beispiel zwischen 60 und 500 mmHg an dem zu behandelnden Hohlvolumen in einer vorbestimmten Evakuierungszeitspanne von zwischen 0,5 und 5 Sekunden anzusteuern. Die Vakuumpumpe 11 hat hierfür einen Steuereingang 11.1.

Fig. 1b zeigt ein vereinfachtes Blockschaltbild mit näheren Details der Druck-Regeleinheit 17 des Vakuumsystems der Fig. 1a. Die Druck-Regeleinheit 17 weist eine durch einen programmierbaren Mikroprozessor oder einen Mikrocontroller oder durch eine spezielle integrierte Schaltung (ASIC) realisierte Steuerungseinheit 17.1 auf. Die Steuerungseinheit empfängt von der Unterdruckmesssonde 16 erzeugte Messsignale. Weiterhin ist sie mit der Nutzereingabeeinheit UI ausgebildet. Über die Nutzereingabeeinheit UI kann der Arzt Parameter wie einen einzustellenden Unterdruck, eine Evakuierungszeitspanne und ein ggf. vorhandenes Totvolumen eingeben. Diese Eingabe muss nicht notwendigerweise durch bestimmte Werte erfolgen. Es kann alternativ oder zusätzlich beispielsweise auch vorgesehen sein, über die Nutzereingabeeinheit UI einen vordefinierten Therapie- oder Untersuchungstyp per Menüauswahl oder Texteingabe zu identifizieren, zu dem in einem Speicher 17.2 der Druck-Regeleinheit vordefinierte Parameter des Unterdrucks (ggf. seiner zeitlichen Entwicklung) und der Evakuierungszeitspanne abgelegt und über die Eingabe abrufbar sind. Das ggf. für die Ermittlung einer Saugleistung der angeschlossenen Vakuumpumpe 11 zu berücksichtigende Totvolumen kann entweder durch Nutzereingabe, alternativ auch über das Einlesen einer Kodierung quasi selbsttätig eingegeben werden.

Die Druck-Regeleinheit ist ausgebildet, unter Heranziehung des Unterdruckwertes an dem zu behandelnden Hohlvolumen, der aus einem vordefinierten Unterdruck-Werteintervall (automatische Werte-Kontrolle auf Zulässigkeit nach Eingabe unter Heranziehung vorgespeicherter Grenzwerte) wählbar ist, und einer Evakuierungszeitspanne, deren zwischen 0,5 und 5 Sekunden liegender Wert wählbar ist, die erforderliche Saugleistung der Pumpe zu ermitteln. Dazu werden situationsabhängig weitere Parameter berücksichtigt:
i) unter Einrechnen eines vorbestimmten Totvolumens der an die Vakuumpumpe anschließbaren Vakuumdrainageanordnung wird eine zum Herstellen des vorgegebenen Unterdrucks an dem zu behandelnden Hohlvolumen in der vorgegebenen Evakuierungszeitspanne erforderliche erste Saugleistung der Vakuumpumpe ermittelt und dem Steuereingang 11.1 der Vakuumpumpe 11 ein entsprechendes erstes Steuersignal zugeführt.
ii) nach Herstellen des vorgegebenen Unterdrucks am zu behandelnden Hohlvolumen wird das Druck-Messsignal überwacht und in Abhängigkeit vom aktuellen Druck-Messsignal eine zur Aufrechterhaltung des vorgegebenen Unterdrucks erforderliche zweite Saugleistung der Vakuumpumpe 11 ermittelt und dem Steuereingang 11.1 der Vakuumpumpe 11 ein entsprechendes zweites Steuersignal zugeführt; und
iii) nach Herstellen des vorgegebenen Unterdrucks am zu behandelnden Hohlvolumen bei Vorliegen einer eine vordefinierte Schwelle überschreitenden Abweichung des gemessenen Drucks oder Unterdrucks vom vorgegebenen Unterdruck eine zum Herstellen des vorgegebenen Unterdrucks innerhalb der vorgegebenen Evakuierungszeitspanne erforderliche dritte Saugleistung ermittelt und dem Steuereingang 11.1 der Vakuumpumpe 11 ein entsprechendes drittes Steuersignal zuzuführen.

In den Fällen ii) und iii) ist das Totvolumen grundsätzlich ebenso zu berücksichtigen. Es kann für die bloße Aufrechterhaltung eines Vakuums in einer Variante vernachlässigt werden. Im Fall iii) ist es bevorzugt jedoch zu berücksichtigen.

Über einen Schalter S, der auch unmittelbar in die Nutzereingabeeinheit UI integriert sein kann, ist es möglich, von einem Endoskopiemodus in einen Therapiemodus und zurück zu schalten. Der Unterschied zwischen den Modi liegt in dem zur Verfügung stehenden Wertebereich für den Unterdruck. Ein Patient soll im Therapiemodus keinen hohen Unterdruck-Werten ausgesetzt werden, ohne dass ein Arzt zugegen ist. Solche höheren Unterdruck-Werte stehen daher nur im Endoskopiemodus zur Verfügung. Ein weiterer Unterschied liegt in den Eingabemöglichkeiten über die Nutzereingabeeinheit UI. Diese sind im Therapiemodus beschränkt, so dass der Patient keine unerwünschten, schädlichen Parameteränderungen vornehmen kann. Der Schalter ist durch einen Schlüssel gesichert und kann nur vom behandelnden Arzt betätigt werden.

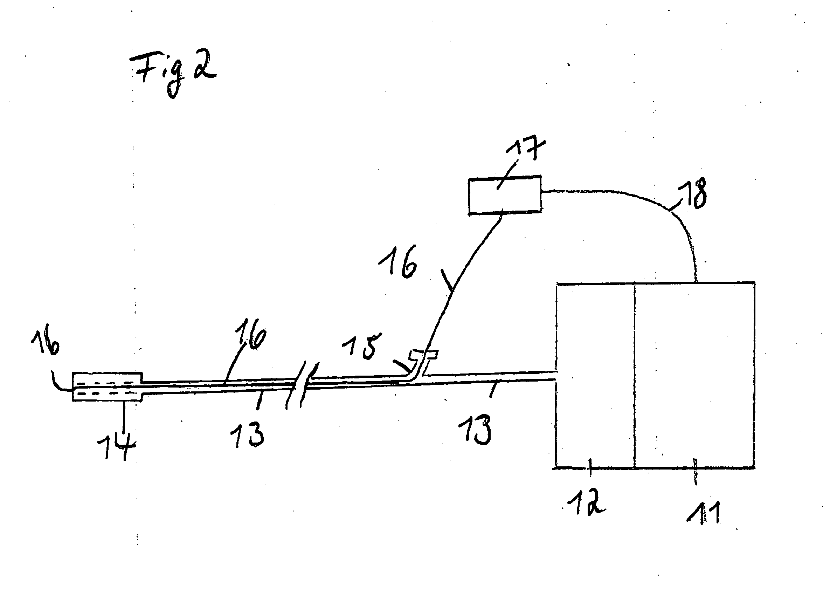

Figur 2 ist eine teilweise Längsschnittdarstellung des Vakuumsystems der Figur 1. In das Fluidkommunikationselement ist über einen seitlichen Eingang 15 die Unterdruckmesssonde 16 eingeführt, welche über die Druck-Regeleinheit 17 die Messwerte zur Regulierung, Voreinstellung und Steuerung über Verbindungselemente 18 an die Vakuumpumpe übermittelt.

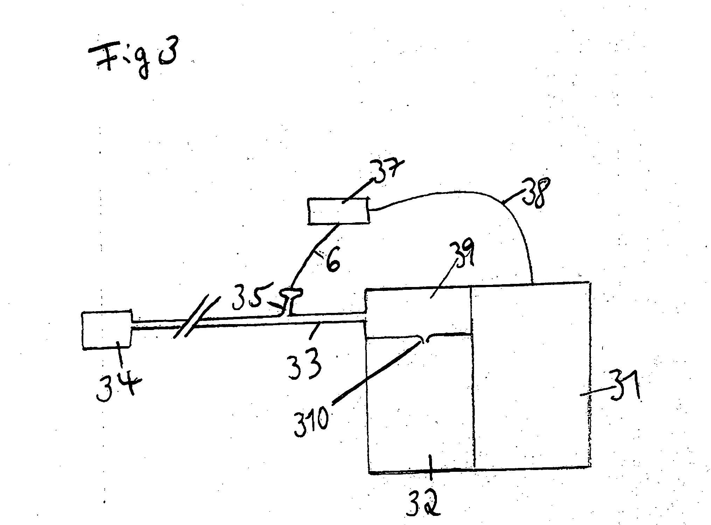

Figur 3 ist eine schematische Darstellung eines anderen Ausführungsbeispiels eines Vakuumsystems, mit einem Vorsekretbehälter 39 zum rascheren Sogaufbau und Sekretbehälter 32. Der Vorsekretbehälter ist über ein Filter/Ventil 310 mit dem Sekretbehälter verbunden. Die Druck-Regeleinheit 37 für die Unterdruckwerte, Zeiteinsteltungen. Evakuvationszeiten, und für Alarmfunktionen ist mit der Vakuumpumpe 31 mit Hilfe von Verbindungselementen 38 verbunden. Ein Fluidsammelelement 34 ist mit Hilfe eines Fluidkommunikationselements 33 an die Pumpeneinheit angeschlossen.

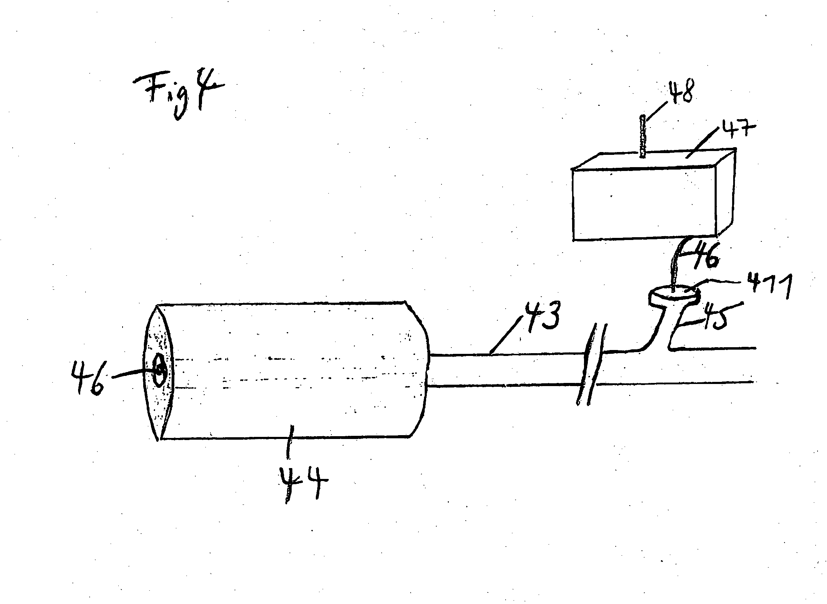

Figur 4 ist eine schematische Darstellung einer Anordnung eines Fluidsammelelementes 44, welches fluidleitend mit einem Fluidkommunikationselement 43 verbunden ist. In das Fluidkommunikationselement ist über einen seitlichen Eingang über ein Ventil 411 eine drahtförmige Unterdruckmesssonde 46 bis in das Fluidsammelelement 44 vorgeschoben worden. Die Unterdruckmesssonde ist mit einer Mess- und Druck-Regeleinheit 47 verbunden, die die Messsignale der Unterdruckmesssonde über eine elektronische Verbindung 48 weiterleiten kann.

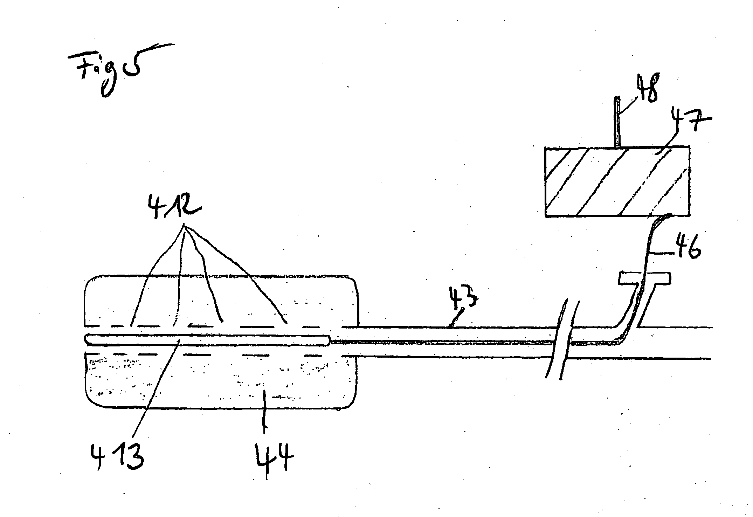

Figur 5 ist eine schematische teilweise Längsschnittdarstellung der Anordnung der Figur 4. Das Fluidsammelelement 44 ist mit dem Fluidkommunikationselement 43 verbunden, an dessen distalem Ende fluidleitende Öffnungen 412 zum Saugen vorhanden sind. In das Fluidkommunikationselement ist eine drahtförmige Unterdruckmesssonde 46 bis zum Fluidsammelelement vorgeführt worden. Am distalen Ende ist der Unterdruckmessfühler 413 der Sonde angebracht. Der Messfühler ist mit einer Mess- und Druck-Regeleinheit 47 verbunden, die die Informationen über eine elektrische Verbindung 48 weiterleiten kann.

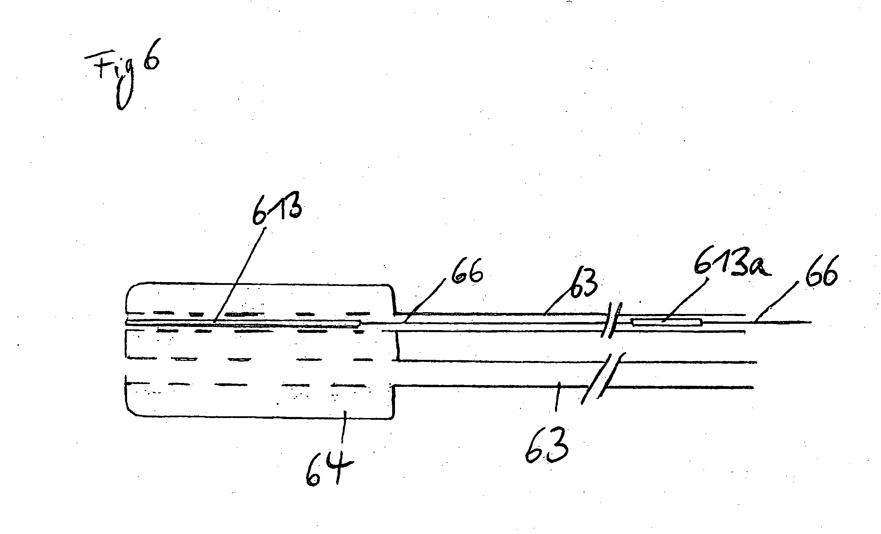

Figur 6 ist eine Längsschnittdarstellung eines Fluidsammelelements 64, welches fluidleitend mit zwei Fluidkommunikationselementen 63 verbunden ist. In eines der Fluidkommunikationselemente ist eine drahtförmige Unterdruckmesssonde 66 bis zum Fluidsammelelement vorgeführt worden. An deren distalem Ende ist ein Unterdruckmessfühler 613 angebracht. Ein weiterer Unterdruckmessfühler 613a ist im Fluidsammelmittel vorhanden.

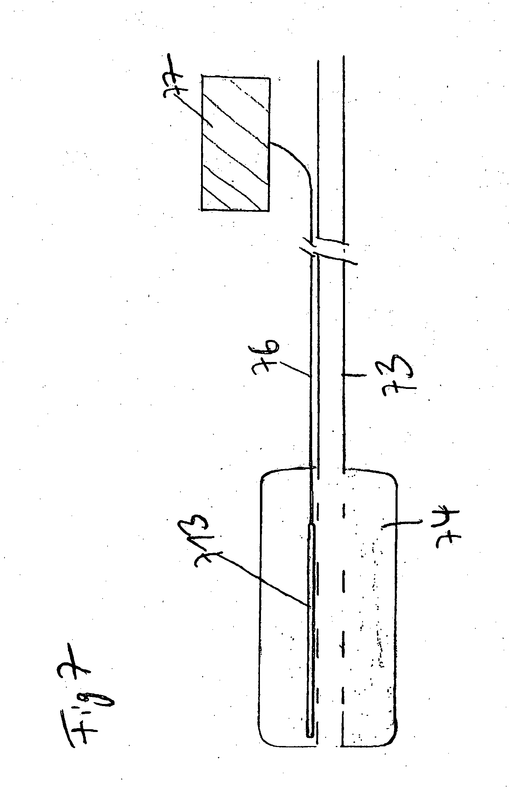

Figur 7 ist eine Längsschnittdarstellung eines Fluidsammelelements 74, in welchem sowohl fluidleitend ein Fluidkommunikationselement 73 und auf diesem aufliegend eine drahtförmige Unterdruckmesssonde 76 angeordnet ist. Die Unterdruckmesssonde 76 ist mit einer Mess- und Druck-Regeleinheit 77 verbunden und ist am distalen Ende mit einem Unterdruckmessfühler 713 ausgerüstet, der in dem Fluidsammelelement liegt. Die Druck-Regeleinheit ist mit einer Alarmfunktion ausgestaltet. Elektronische Steuerungssignale werden zur Regulierung des Unterdrucks insbesondere an die Vakuumpumpe übermittelt. Alarme bezüglich einer Fehlfunktion können ausgelöst werden.

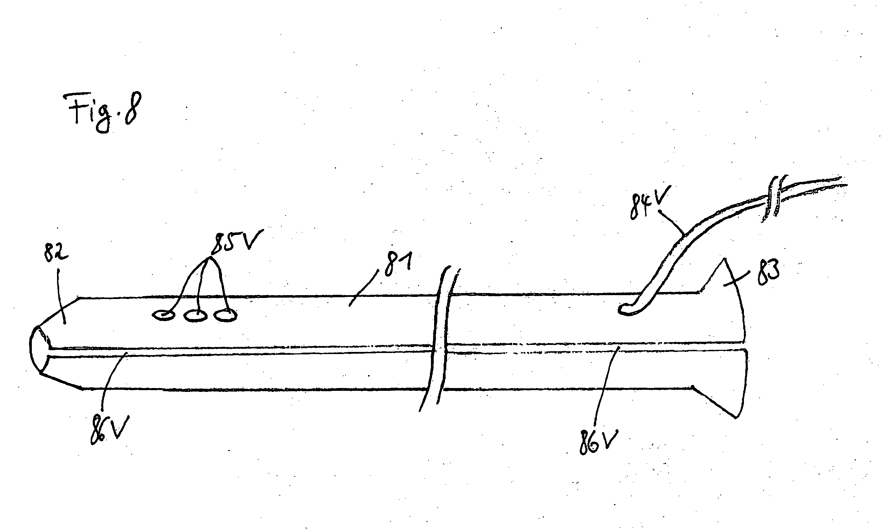

Figur 8 ist eine Darstellung, die eine Ausführung eines längsgeschlitzten Overtubes 81 zeigt. Am Distalende verjüngt sich der Overtube 81 konisch, um Verletzungen beim Einführen zu vermeiden. Über die ganze Länge ist ein kompletter Schlitz 86V vorhanden. Am proximalen Ende 83 ist der Overtube 81 trichterförmig konstruiert, um das Einführen eines Endoskops zu erleichtern. Der Overtube ist mit einem Fluidkommunikationselement 84V in Form einer in die Wand integrierten Drainageleitung ausgerüstet, die sich von proximal bis nach distal erstreckt. Sie endet am Distalende in seitlichen Öffnungen 85V und perforiert mit diesen die Wand des Overtubes. Am proximalen Ende wird sie schlauchförmig ausgeleitet (84V) und kann hier mit der Vakuumvorrichtung verbunden werden.

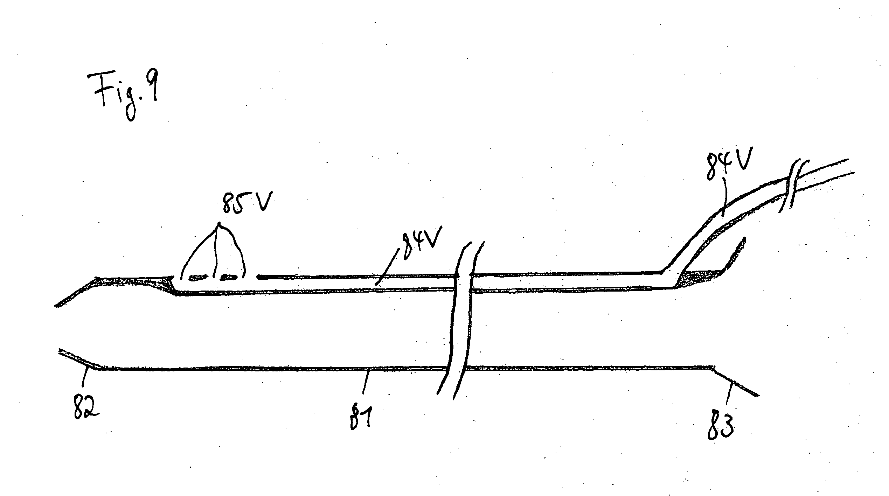

Figur 9 ist eine Längsschnittdarstellung des Overtubes 81 der Figur 8, mit Darstellung des Overtubes 81, welcher sich am Distalende 82 verjüngt, am proximalen Ende 83 trichterförmig erweitert ist, und mit dem Fluidkommunikationselement 84V, welches am distalen Ende in fluidleitenden Wändöffnungen 85V endet und proximal schlauchförmig aus der Wand ausgeleitet ist.

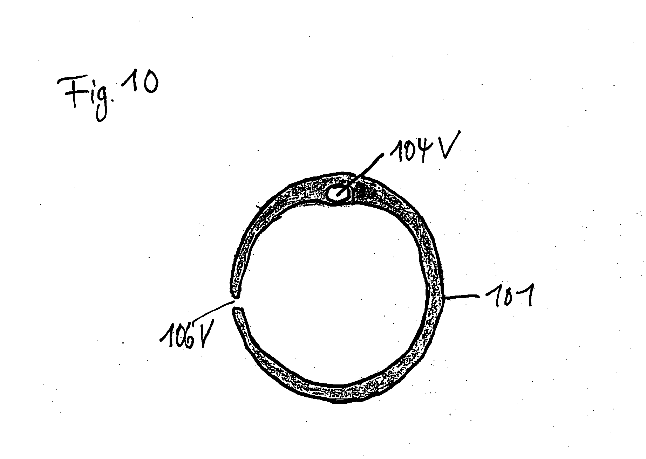

Figur 10 ist eine Querschnittdarstellung eines anderen Ausführungsbeispiels eines Overtubes 101 mit einem in der Wand integrierten Fluidkommunikationselement 104V. Der Overtube 101 ist mit einem Längsschlitz 106V dargestellt.

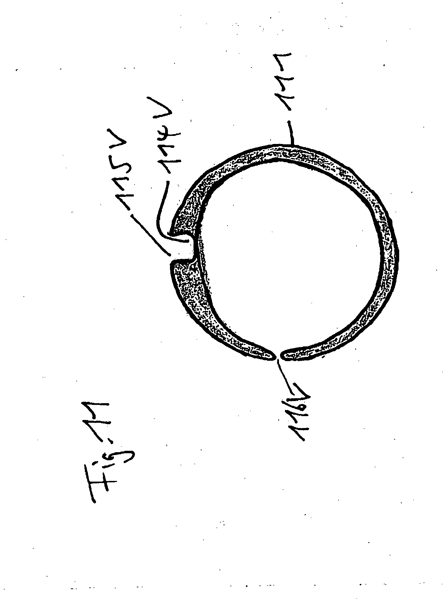

Figur 11 ist eine Querschnittdarstellung einer anderen Variante eines Overtubes 111, mit einem in der Wand integriertem Fluidkommunikationselement 114V, welches mit einer Öffnung 115V fluid leitend die Wand perforiert und mit der Außenwand des Overtubes 111 fluidleitend verbunden ist. Der Querschnitt ist in Höhe der Wandöffnung 115V gezeichnet. Der Overtube ist mit einem Längsschlitz 116V dargestellt.

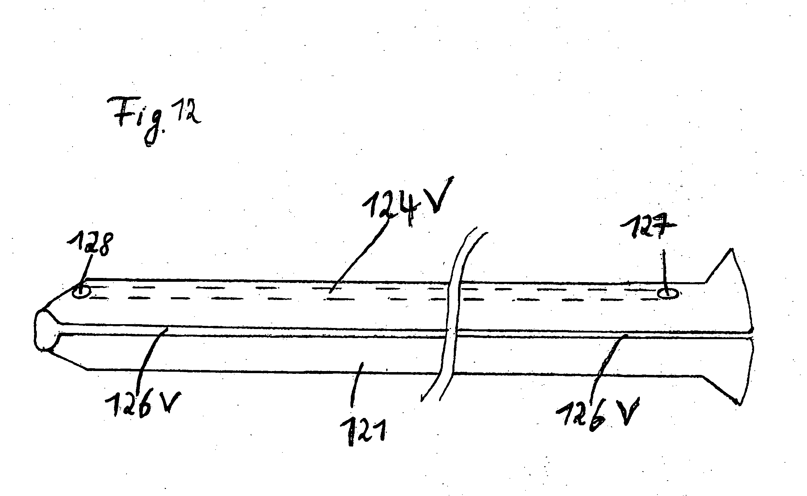

Figur 12 ist eine Darstellung, die eine Ausführung eines Overtube 121 zeigt. Über die gesamte Länge ist ein Längsschlitz 126V vorhanden. Der Overtube 121 ist mit einem Fluidkommunikationselement 124V in Form eines in die Wand integrierten Arbeitskanals ausgerüstet, der sich von einer proximalen Wandöffnung 127 des Overtubes bis zur distalen Spitze erstreckt und hier mit einer distalen Wandöffnung 128 endet.

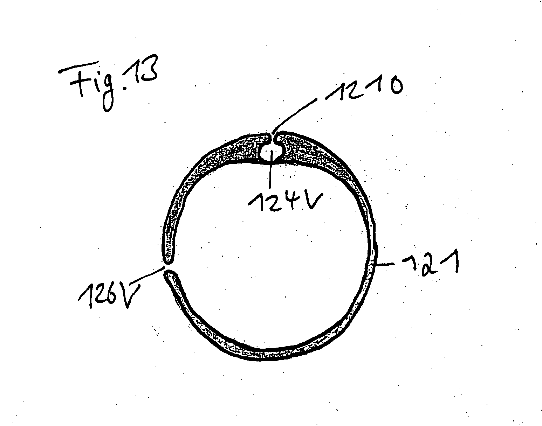

Figur 13 ist eine Querschnittdarstellung des Overtubes 121 von Figur 12, wobei das kanalförmige Fluidkommunikationselement 124V mit einem Längsschlitz 1210 versehen ist. Das Fluidkommunikationselement ist in die Wand des Overtubes 121 integriert. Der Overtube ist auch mit dem Längsschlitz 126V dargestellt.

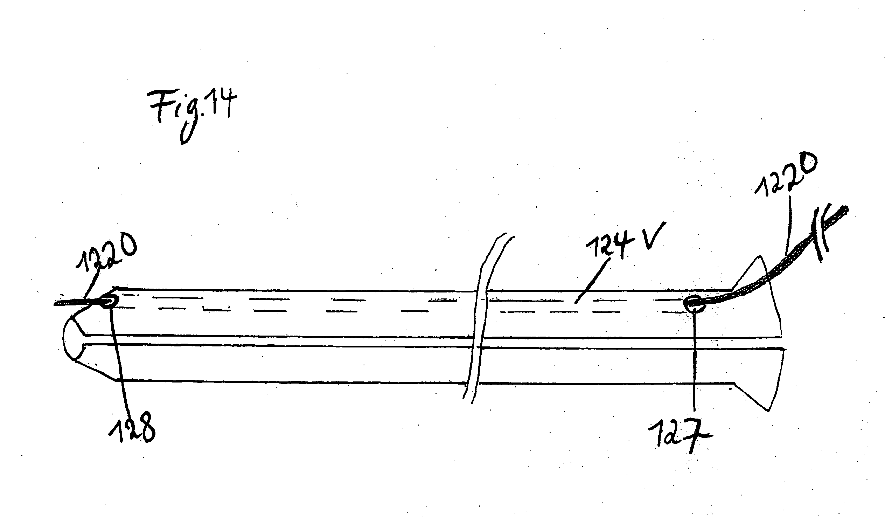

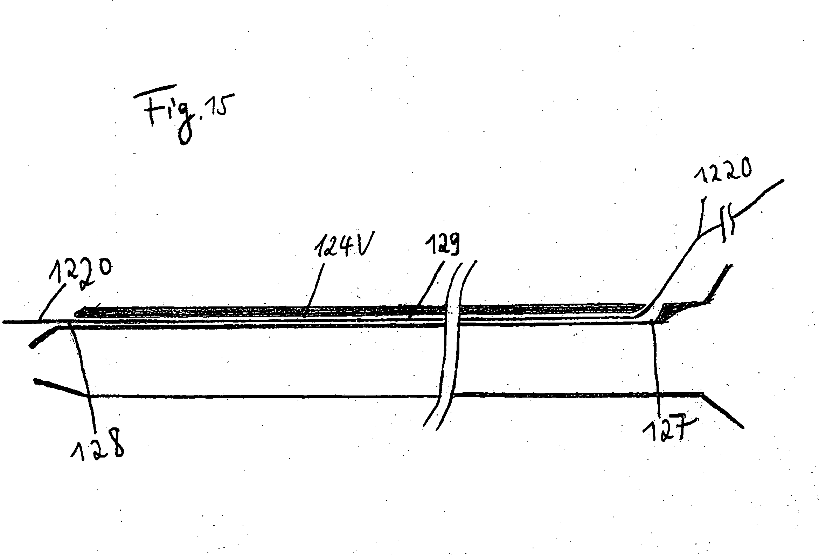

Figur 14 ist eine andere Darstellung der Ausführung von Figur 12 und 13, wobei in das Fluidkommunikationselement 124V über die proximale Öffnung 127 ein medizinisches Instrument 1220, hier ein Führungsdraht,eingeführt worden ist, und dieses durch die distale Öffnung 128 ausgeleitet wurde. Figur 15 ist eine Längsschnittdarstellung des Overtubes 121, welche den Arbeitskanal 129, welcher in der Wand des Overtubes 121 integriert ist, sowie die proximale Wandöffnung 127 und die distale Wandöffnung 128 zeigt.

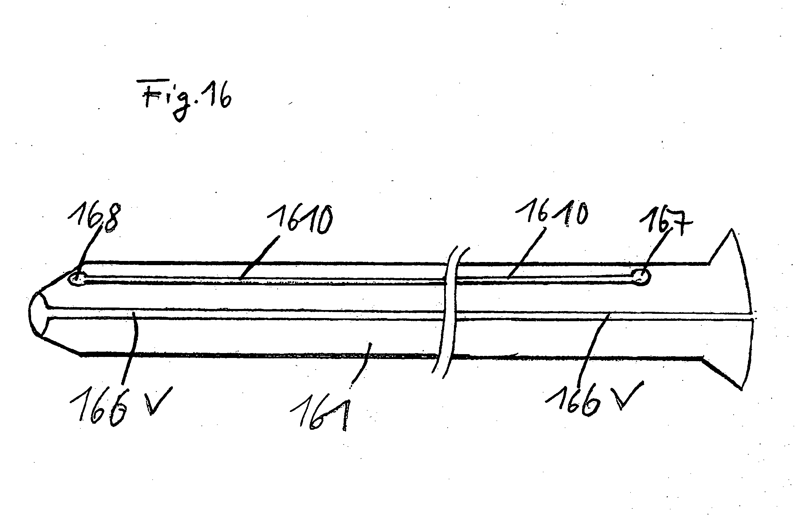

Figur 16 ist eine Darstellung eines Overtubes 161, der einer eine Variante des Overtubes der Figuren 12 bis 15 bildet. Ein in der Wand des Overtubes 161 integrierter Arbeitskanal weist über die gesamte Länge zwischen proximaler Wandöffnung 167 und distaler Wandöffnung 168 einen Längsschlitz 1610 auf. Der Overtube 161 weist ebenfalls in dieser Ausführung einen Längsschlitz 166V über die gesamte Länge auf.

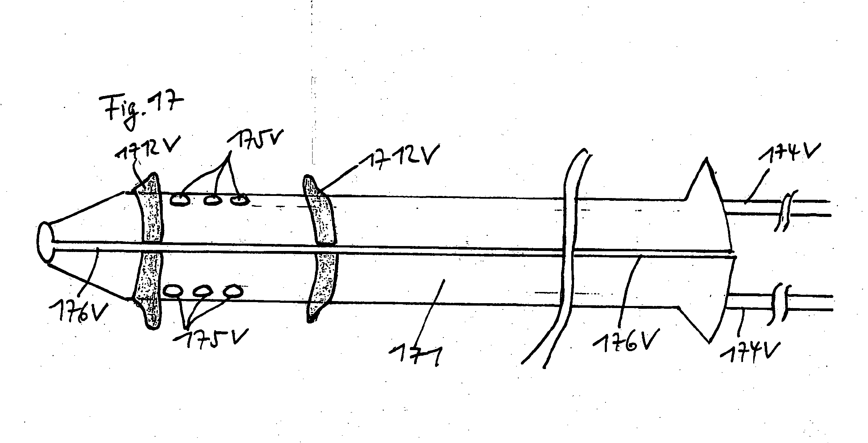

Figur 17 ist eine Darstellung eines Overtubes 171, über dessen ganze Länge ein kompletter Schlitz 176V vorhanden ist. Der Overtube 171 ist mit zwei Fluidkommunikationselementen 174V in Form von Drainageleitungen ausgerüstet, die in die Wand des Overtubes integriert sind. Sie enden in seitlichen Öffnungen 175V am distalen Ende des Overtubes. Die proximalen Enden 1711 der Fluidkommunikationselemente werden mit der Vakuumeinheit fluidleitend verbunden. Auf dem Overtube befestigt sind proximal und distal der seitlichen Öffnungen 175V der Fluidkommunikationselemente ringförmige lippenartige Aufwulstungen 1712V.

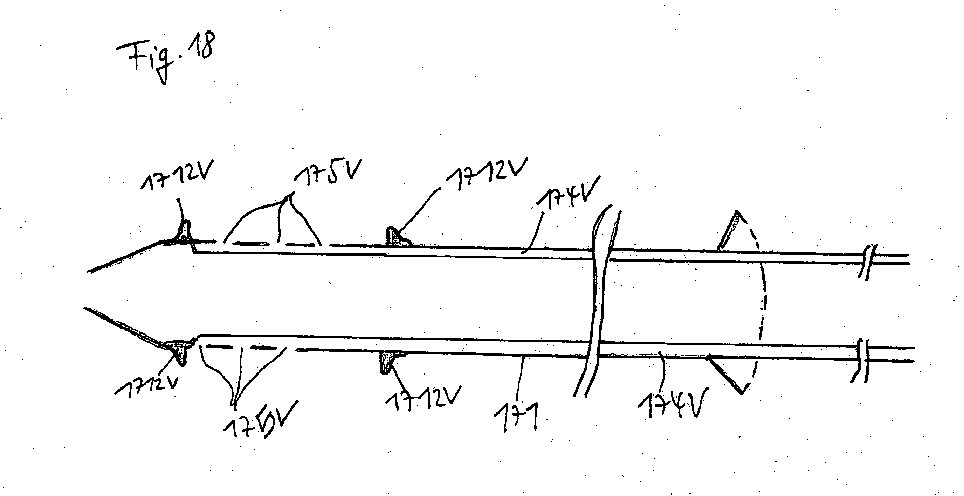

Figur 18 ist eine Längsschnittdarstellung des Overtubes 171 der Figur 17, welche die zwei in die Wand integrierten Fluidkommunikationselementen 174V, die fluidleitend seitlich im distalen Ende des Overtubes mit Öffnungen 175V enden, zeigt. Proximal und distal von diesen sind die ringförmigen lippenartigen Aufwulstungen 1712V auf dem Overtube befestigt.

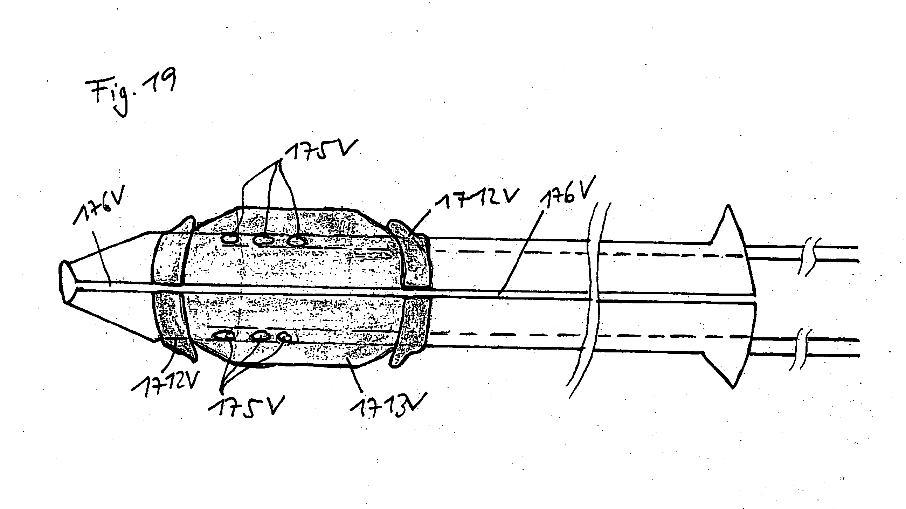

Figur 19 zeigt eine Variante der Darstellungen der Ausführungsformen von Figur 17 und Figur 18, wobei hier noch in Höhe der distalen Wandöffnungen 175V zwischen den ringförmigen Aufwulstungen 1712V das Fluidsammelelement 1713V befestigt ist. Auch das Fluidsammelelement ist mit einem Längsschlitz 176V in der Längsachse des Overtube 171 versehen.

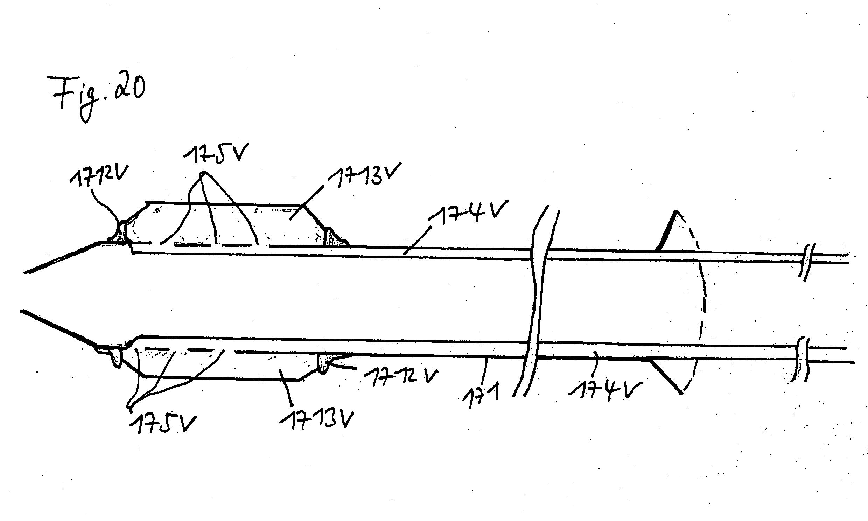

Figur 20 ist eine Längsschnittdarstellung des Overtubes 171 von Figur 18 und 19, welche deutlich die Fluidkommunikationselementen 174V mit fluidleitenden Wandöffnungen 175V, darüber befestigtem Fluidsammelelement 1713V und proximal und distalen lippenartigen Aufwulstungen 1712V zeigt.

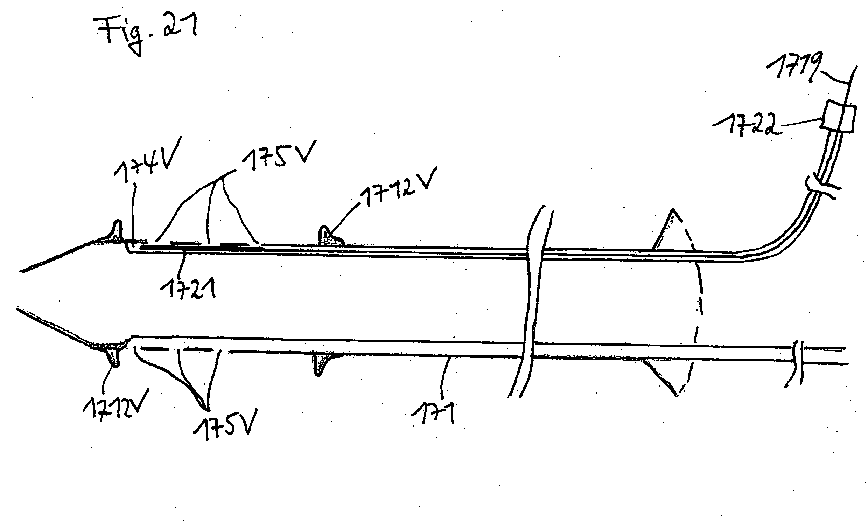

Figur 21 ist eine weitere Längsschnittdarstellung des Overtubes der Figuren 18 bis 20, welche die Fluidkommunikationselemente 174V mit den fluidleitenden Wandöffnungen 175V und die proximalen und distalen lippenartigen Aufwulstungen 1712V zeigt. Zusätzlich ist hier noch eine drahtförmige Messsonde 1719 dargestellt, die in eines der Fluidkommunikationselemente eingeführt wurde und die distal in eine Unterdruckmesseinheit 1721 endet. Die Messsonde 1719 ist in das Fluidkommunikationselement 174V über ein Ventil 1722 eingeführt worden.

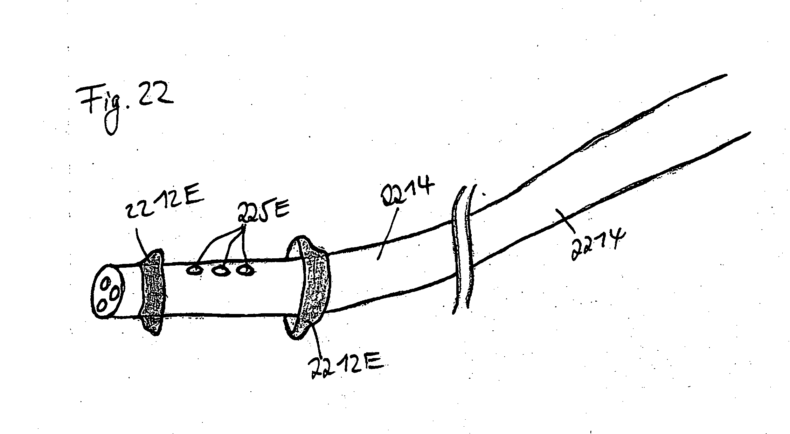

Figur 22 ist eine Darstellung eines distalen Endes eines Endoskops 2214. Am distalen Ende des Endoskops 2214 sind seitliche fluidleitende Wandöffnungen 225E eines in dem Endoskop eingebauten Fluidkommunikationselements dargestellt. Proximal und distal dieser Wandöffnungen 225E sind lippenartige Ringe 2212E auf dem Endoskop 2214 befestigt.

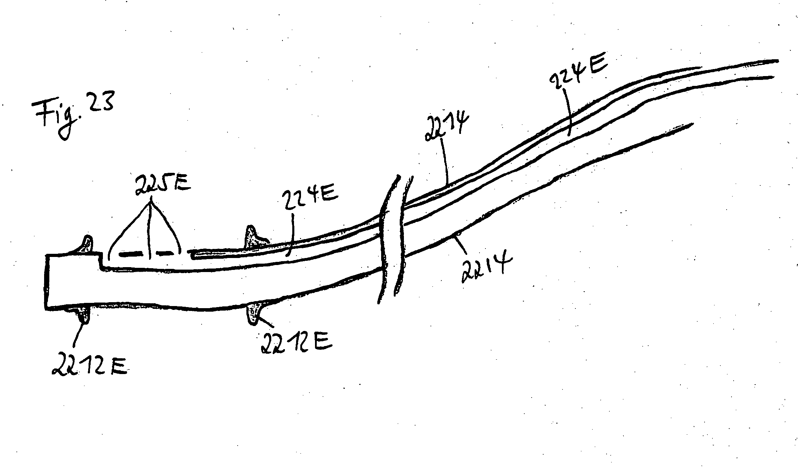

Figur 23 ist eine Längsschnittdarstellung des Endoskops 2214 der Figur 22, und zeigt das innenliegende Fluidkommunikationselement 224E, die lippenartigen Ringen 2212E proximal und distal der seitlichen Öffnungen 225E des Fluidkommunikationselements 224E.

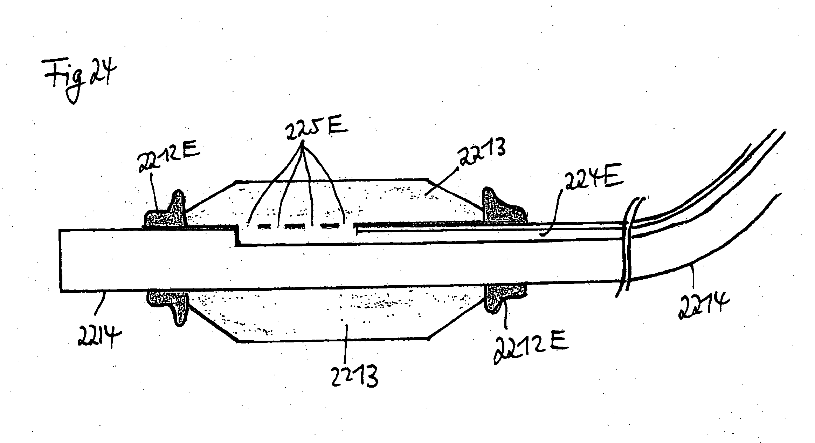

Figur 24 ist eine weitere Längsschnittdarstellung des Endoskops 2214 von Figur 22. Bei dieser Darstellung ist über den fluidleitenden Öffnungen des Fluidkommunikationselements 224E, zwischen den lippenartigen Ringen 2212E ein Fluidsammelelement 2213E eingesetzt.

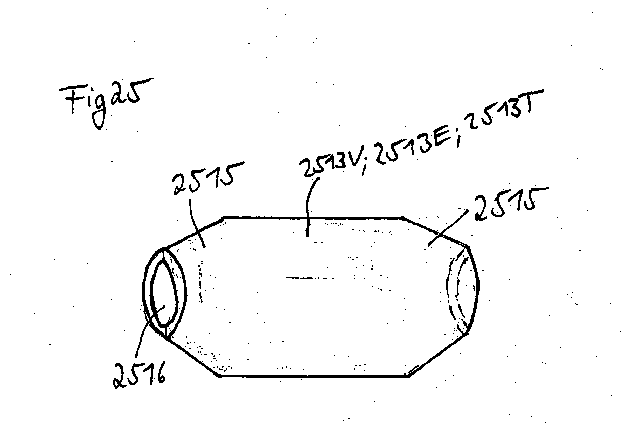

Figur 25 ist eine Darstellung eines Fluidsammelelementes 2513V, 2513E, 2513T, das für die Verwendung am Overtube, Endoskop und der Trägerhülse geeignet ist. In dieser Ausführung weist das Fluidsammelelement an seinen Enden eine konische Verjüngung 2515 auf. Zentral ist ein Kanal 2516 entlang der Längsachse des Fluidsammelelements angeordnet.

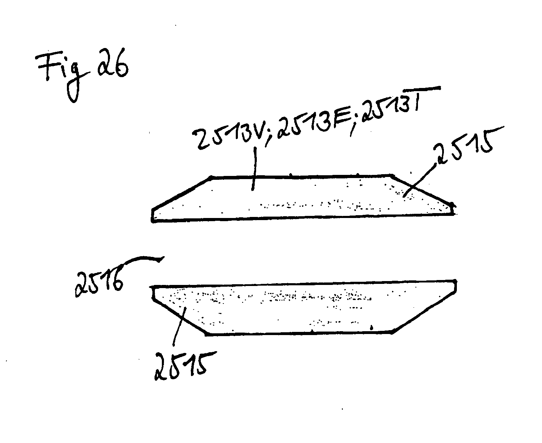

Figur 26 ist eine Längsschnittdarstellung des Fluidsammelelement 2513V, 2513E, 2513T von Figur 25. Erkennbar ist die konische Verjüngung 2515 an den Enden und der zentrale Kanal 2516 entlang der Längsachse.

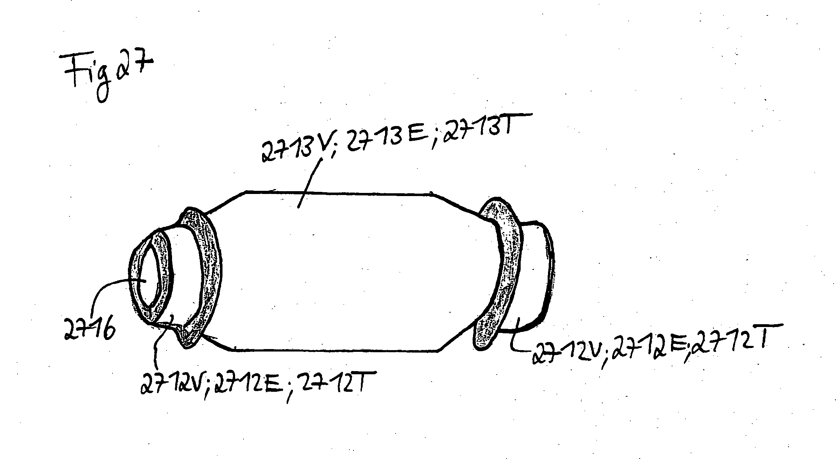

Figur 27 ist eine Darstellung eines anderen Fluidsammelelements 2713V, 2713E, 2713T für Overtube, Endoskop und Trägerhülse, wobei an den Enden des Elementes je ein lippenartiger Ring 2712V, 2712E, 2712T befestigt ist. Ein gemeinsamer zentraler Kanal 2716 erstreckt sich durch das Fluidsammelelement hindurch.

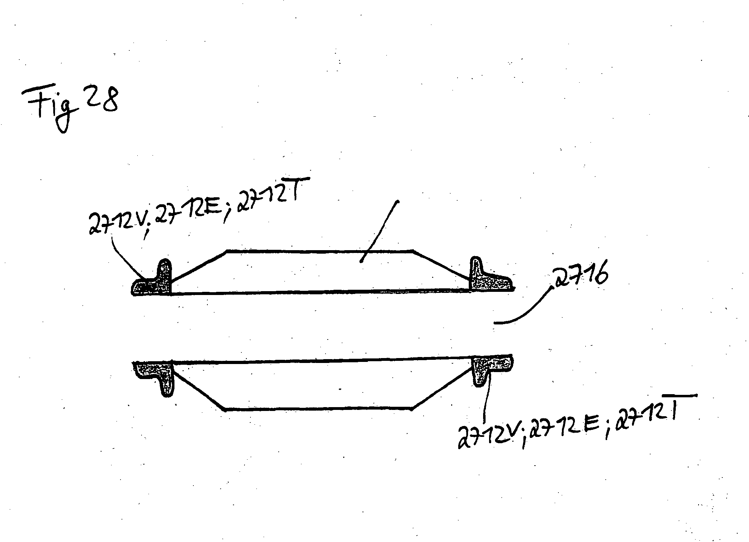

Figur 28 ist eine Längsschnittdarstellung von Figur 27 mit Fluidsammelelement 2713, 2713E, 2713 T, wobei an den Enden je eine lippenartiger Ring befestigt ist (2712,2712E, 2712T).

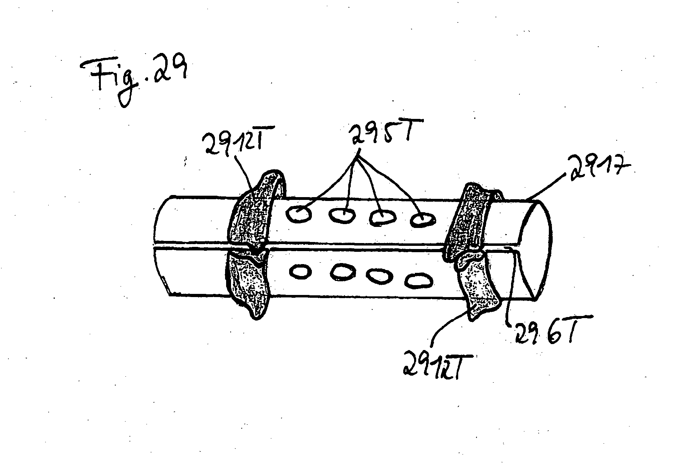

Figur 29 ist eine Darstellung einer Trägerhülse 2917 für ein Fluidsammelelement, dargestellt mit einem Längsschlitz 296T, fluidleitenden Wandperforationen 295T und lippenartigen Ringen 2912T proximal und distal der Wandperforationen 295T. Die Ringe sind ebenfalls geschlitzt.

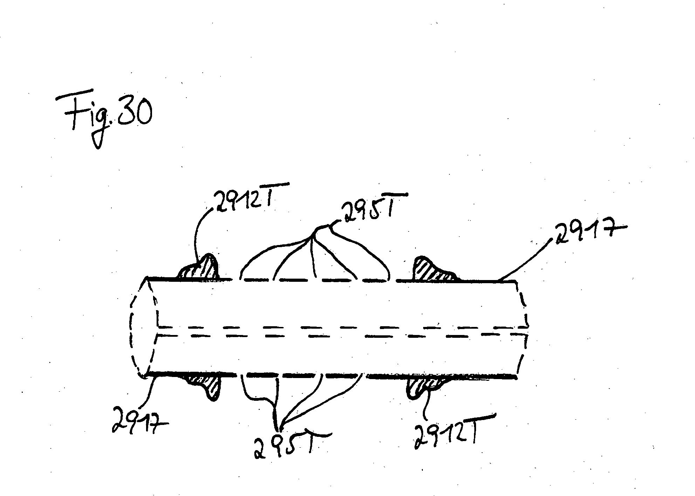

Figur 30 ist eine Längsschnittdarstellung der Trägerhülse der Figur 29 und zeigt die fluidleitenden Wandperforationen 295T und die lippenartigen Ringe 2912T proximal und distal von den Wandperforationen 295T.

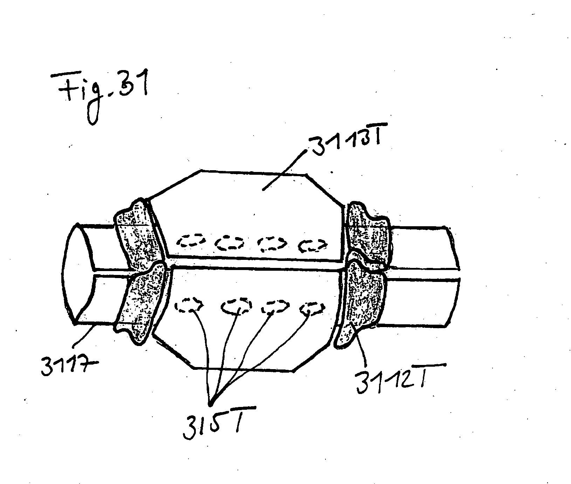

Figur 31 ist eine Darstellung einer Trägerhülse 3117 mit einem auf dieser zwischen lippenartigen Ringen 3112T befestigten längsgeschlitzten Fluidsammelelement 3113T. Wandperforationen 315T der Trägerhülse sind mit gestrichelten Linien angedeutet.

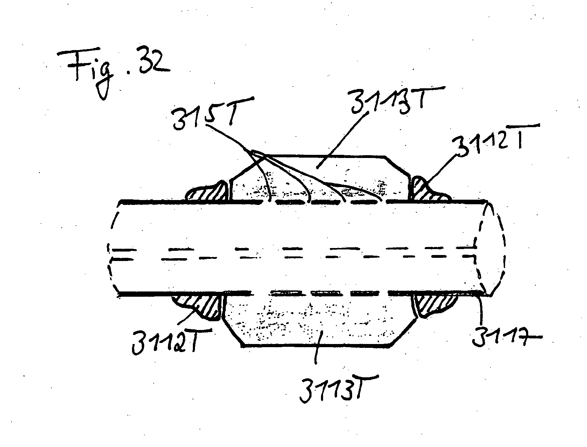

Figur 32 ist eine Längsschnittdarstellung der Trägerhülse von Figur 31, wobei auf der Trägerhülse 3117 zwischen den lippenartigen Ringen 3112T befestigt und fluidleitend mit den Wandperforationen 315T das Fluidsammelelement 3113T befestigt ist.

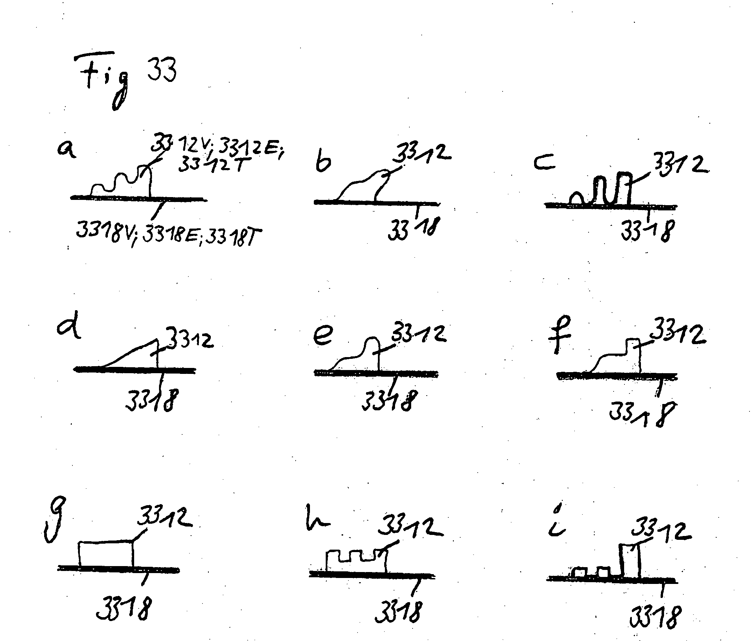

Figuren 33a-i zeigen verschiedene Varianten von Querschnittprofilen der lippenartigen Ringabschlüsse 3112V, 3112E, 3112T, die auf einer Außenwand 3118V, 3118E, 3118T von Overtube, Endoskop oder Trägerhülse angebracht werden.

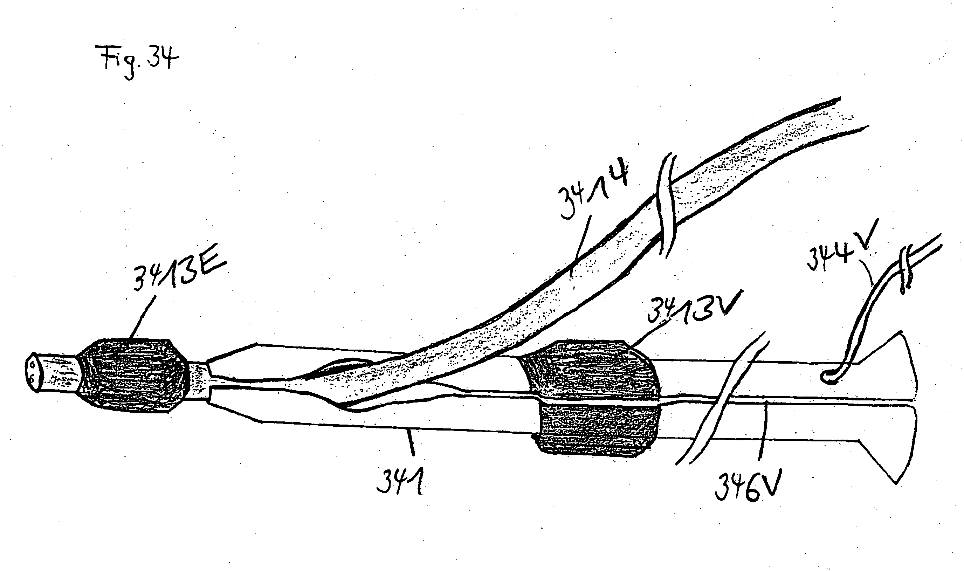

Figur 34 zeigt, wie ein flexibles Endoskop 3414 in einem Stadium, in dem es über den Längsschlitz 346V eines Overtubes 341 eingebracht oder entfernt wird. Auf dem Endoskop und dem Overtube sind Fluidsammelelemente 3413E, 3413V am jeweiligen distalen Ende angebracht. Das Fluidkommunikationselement 344V ist fluidleitend mit dem Fluidsammelelement des Overtubes 3413V verbunden.

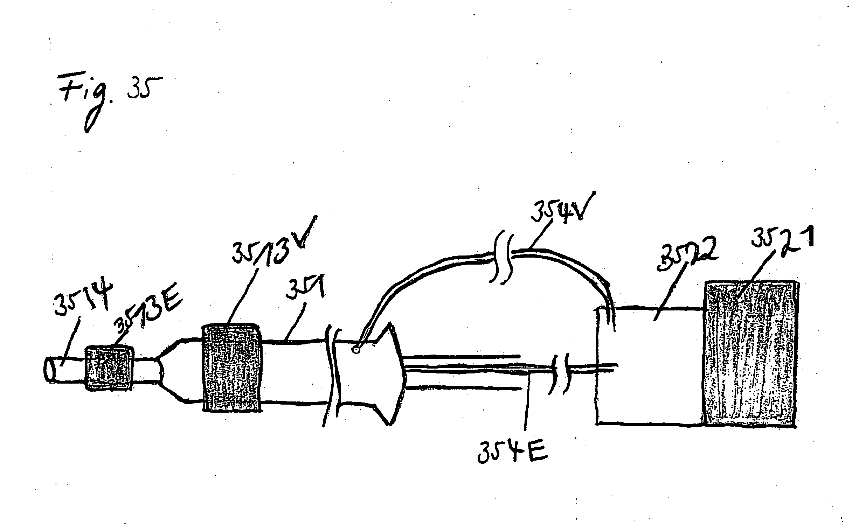

Figur 35 zeigt eine Endoskopie-Anordnung gemäß einem weiteren Ausführungsbeispiel. Dargestellt ist eine Vakuumpumpeneinheit 3521 mit einem Sekretauffangbehälter 3522,
an die ein Overtube 351 und ein Endoskop 3514 angeschlossen sind. An den distalen Enden des Overtubes 351 und des Endoskops 3514 sind Fluidsammelelemente 3513V und 3513E befestigt, die mit Fluidkommunikationselementen 354V (Overtube), und 354E (Endoskop) mit der Vakuumpumpeneinheit 3521 verbunden sind.

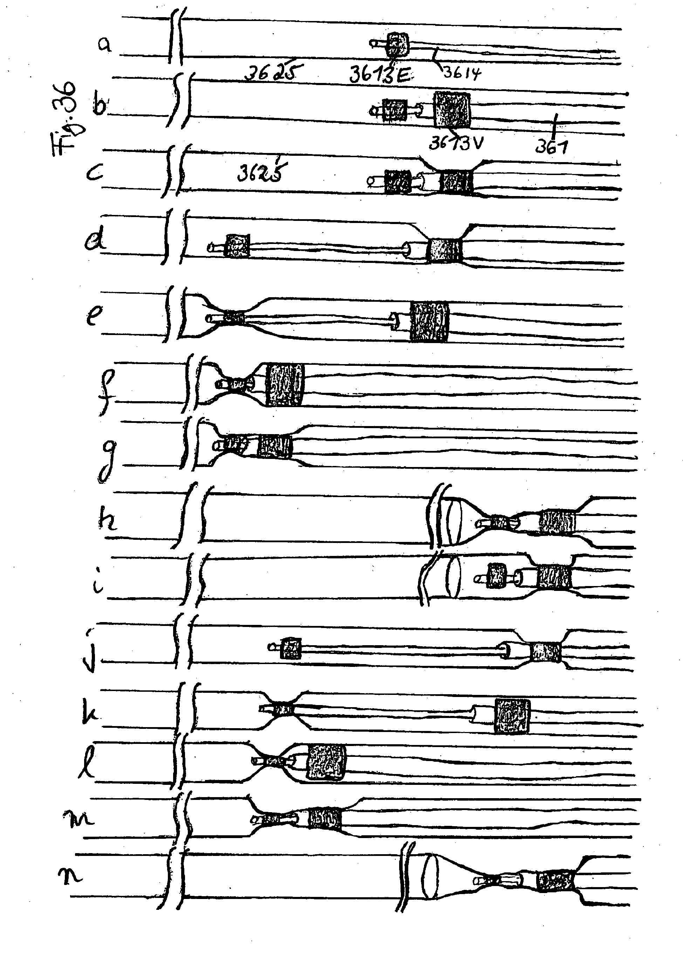

Figur 36a-n ist eine schematische Darstellung des Untersuchungsganges einer Vakuumendoskopie. Die Behandlung umfasst die folgenden Schritte:
a) Einführen des Endoskops 3614 mit Fluidsammelelement 3613E in einen Darm 3625 eines Patienten;
b) Nachführen des Overtubes 361 mit Fluidsammelelement 3613V über dem Endoskop;
c) Beaufschlagen des Fluidsammelelements 3613V mit einem Vakuum;
d) Vorschieben des Endoskops 3614 in den Darm;
e) Beaufschlagen des Fluidsammelelements 3613E am Endoskop 3614 mit Vakuum, dabei keine Vakuumbeaufschlagung des Fluidsammelelements 3613V auf dem Overtube 361;
f) Nachschieben des Overtubes 361 über dem fixiertem Endoskop 3614;
g) Beaufschlagen beider Fluidsammelelemente 3613E und 3613V mit Vakuum;
h) Optionales Begradigungsmanöver bei Bedarf, durch Zurückziehen des Fluidsammelelements 3613E zusammen mit dem Fluidsammelelement 3613V, beide unter Vakuumbeaufschlagung;
i) Abschalten der Vakuumbeaufschlagung des Fluidsammelelements 3613E bei Aufrechterhaltung der Vakuumbeaufschlagung des Fluidsammelelements 3613V;
j) Vorschieben des Endoskops 3614 bei durch Vakuum fixiertem Overtube 361;
k) Aufrechterhaltung der Vakuumbeaufschlagung des Fluidsammelelements 3613E, Abschalten der Vakuumbeaufschlagung des Fluidsammelelements 3613V;
l) Nachschieben des Overtubes 361 bei durch Vakuum fixiertem Endoskop 3614;
m) Vakuumbeaufschlagung beider Fluidsammelelemente 3613E und 3613V;
n) Begradigungsmanöver durch Zurückziehen des Fluidsammelelements 3613E zusammen mit dem Fluidsammelelement 3613V, beide unter Vakuum;

Danach kann der Untersuchungsfortgang weiter mit dem Schritt i) und folgenden durchgeführt werden.

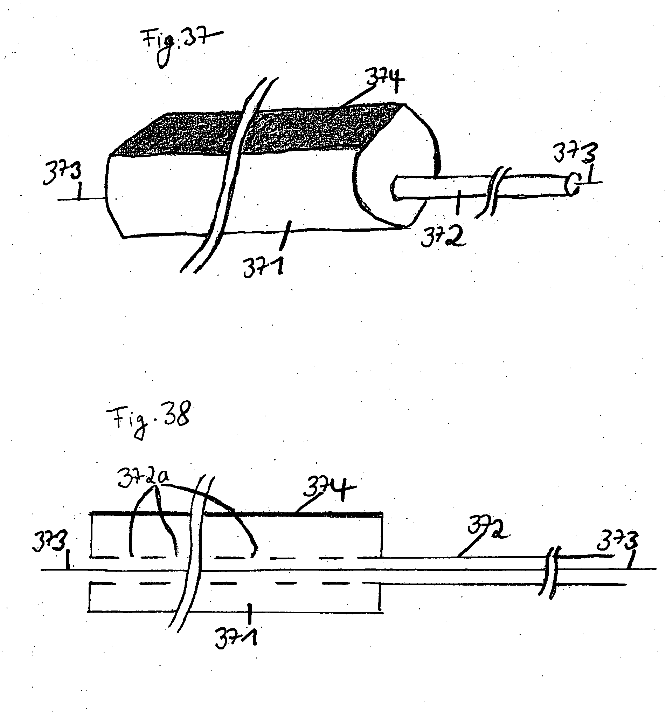

Figur 37 ist eine Darstellung eines Fluidsammelelements (einer Vakuumdrainage) in Form eines Schwammkörpers 371 mit teilweiser Oberflächenversiegelung 374 des Schwammkörpers 371. In einem Drainageschlauch 372, der in den Schwammkörper 371 eingeführt ist, ist in dieser Darstellung ein Führungsdraht 373 eingebracht.

Figur 38 ist eine Längsschnittdarstellung des Fluidsammelelements 371 von Figur 37. Die Oberflächenversiegelung 374 des Schwammkörpers 371 mit Drainageschlauch 372, welcher seitlich Perforationsöffnungen 372a hat und in dem ein Führungsdraht 373 eingebracht ist, sind dargestellt.

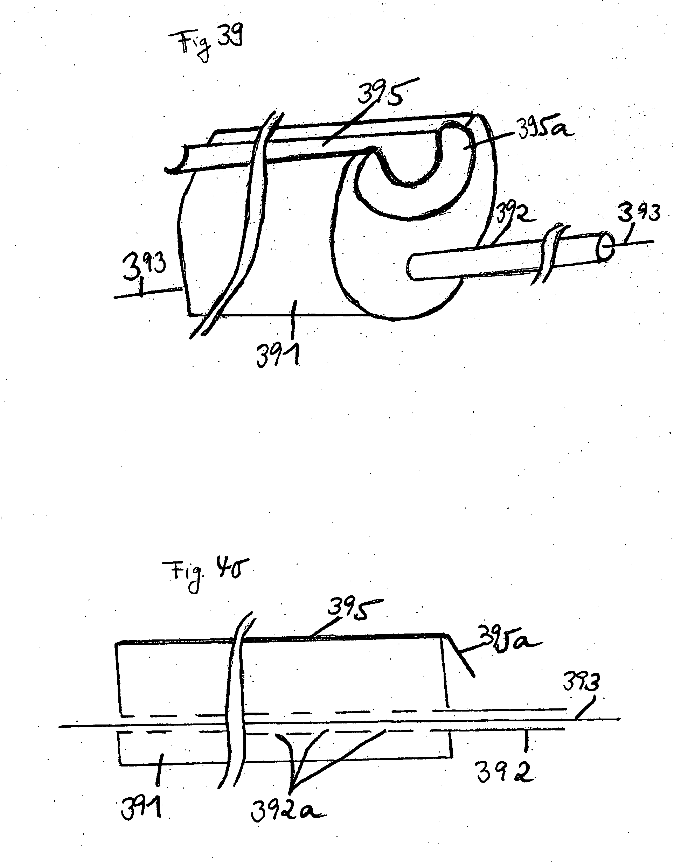

Figur 39 ist eine Darstellung einer anderen Ausführungsform einer Vakuumdrainage 391 mit einem Drainageschlauch 392 und einem einliegenden Führungsdraht 393. Auf der Außenseite des Schwammkörpers 391 ist eine schalenförmige Versiegelung 395 angebracht, welche an dem proximalen Ende eine trichterförmige Aufweitung 395a aufweist.

Figur 40 ist eine Längsschnittdarstellung der Vakuumdrainage 391 von Figur 39, welche ebenfalls die schalenförmige Versiegelung 395 an der Außenseite des Schwammkörpers der Vakuumdrainage 391 zeigt, die am proximalen Ende trichterförmig aufgeweitet ist (395a). Dargestellt ist ebenfalls der Drainageschlauch 392, welcher seitliche Perforationsöffnungen 392a hat und in dem ein Führungsdraht 393 liegt.

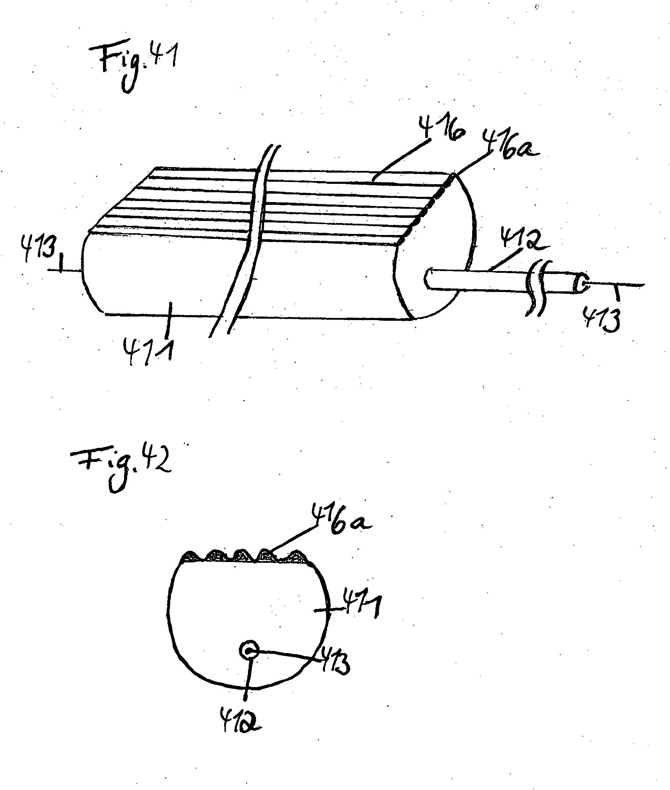

Figur 41 ist eine Darstellung einer Vakuumdrainage in Form eines Schwammkörpers 411 mit einer profilierten Oberflächenversiegelung 416 des Schwammkörpers 411. In dem Drainageschlauch 412 ist ein Führungsdraht 413 eingebracht. Die Oberflächenversiegelung 416 besitzt ein Rillenprofil 416a mit nebeneinander in Längsrichtung des Schwammkörpers 411 verlaufenden Längsrillen.

Figur 42 ist eine Querschnittdarstellung der Vakuumdrainage von Figur 41. In dem Drainageschlauch 412 liegt der Führungsdraht 413 ein. Die Oberflächenversiegelung zeigt ihr Längspröfil 416a.

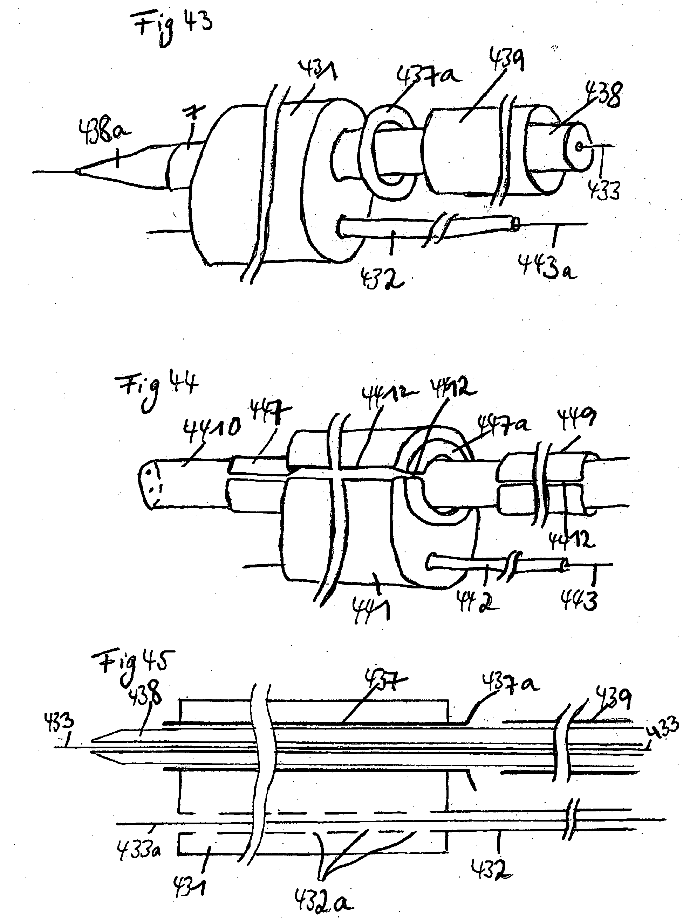

Figur 43 ist eine Darstellung einer Vakuumdrainage mit einem in dem Schwammkörper 431 befestigten Tubus 437. In den Schwammkörper 431 ist ein Drainageschlauch 432 eingebracht, in welchem ein Führungsdraht 433 einliegt. Am proximalen Ende der Vakuumdrainage ist eine trichterförmige Aufweitung 437a vorhanden. In den Tubus 437 ist ein Legestab 438 eingeführt, welcher am distalen Ende 438a konisch zuläuft. In den Legestab 438 ist ein weiterer Führungsdraht eingebracht. Auf dem proximalen Ende des Legestabs ist ein Pusher 439 aufgesetzt.

Figur 44 ist eine Darstellung einer anderen Ausführungsform einer Vakuumdrainage mit einem in einem Schwammkörper 441 befestigten Tubus 447, welcher am proximalen Ende eine trichterförmige Aufweitung 447a aufweist. In dem Tubus ist ein Endoskop 4410 eingeführt. Auf dem proximalen Ende des Endoskops 4410 ist ein Pusher 449 aufgesetzt. Tubus 447, Schwammkörper 441 und Pusher 449 sind mit einem seitlichen kompletten Längsschlitz 4412 ausgerüstet. Im Schwammkörper 4411 ist ein Drainageschlauch 442 eingebracht, in diesem liegt ein Führungsdraht 443 ein.

Figur 45 ist eine Längsschnittdarstellung der Vakuumdrainage der Figur 43. Im Schwammkörper 431 liegt der Tubus 437, welcher am proximalen Ende seine trichterartige Aufweitung 437a hat. Im Tubus 437 liegt der Legestab 438 ein. Im Legestab 438 ist ein Führungsdraht 433 eingebracht. Auf dem Legestab ist der Pusher 439 aufgesetzt. Im Schwammkörper 431 liegt der Drainageschlauch 432 mit seitlichen Öffnungen 432a. Im Drainageschlauch 432 liegt ein weiterer Führungsdraht 433a.

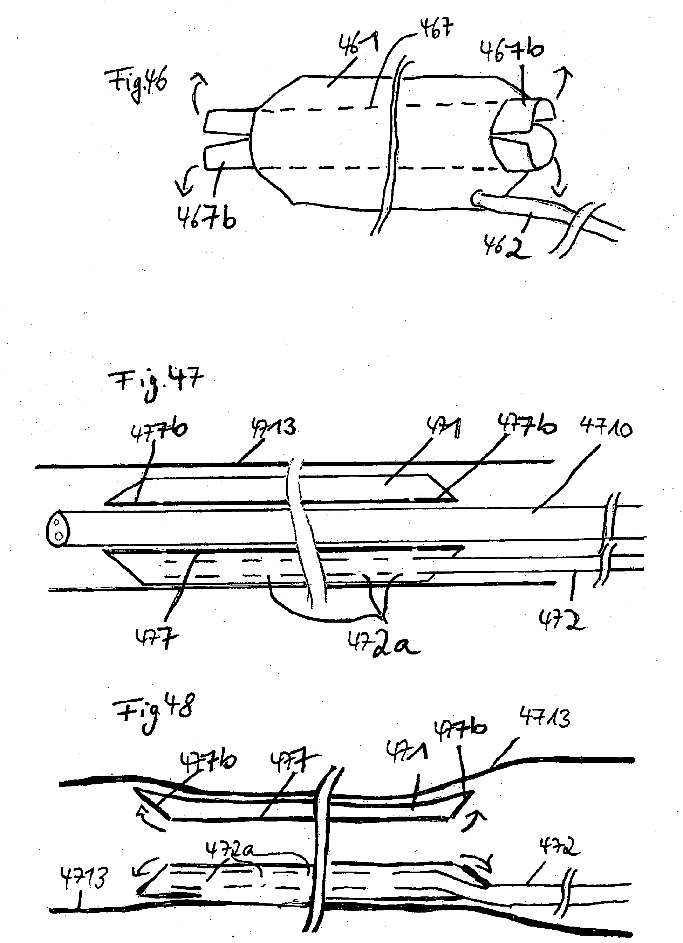

Figur 46 ist eine Darstellung einer weiteren Ausführungsform einer Vakuumdrainage mit Drainageschlauch 462 im Schwammkörper 461. Im Schwammkörper der Vakuumdrainage liegt ein Tubus 467 ein, welcher proximale und distal gespaltenen Enden 467b hat. Pfeile zeigen an, in welcher Richtung sich die gespaltenen Enden 467b öffnen können.

Figur 47 ist eine Längsschnittdarstellung einer weiteren Vakuumdrainage mit im Schwammkörper 471 einliegendem Tubus, welcher sich an seine Enden 477b unter Sog nach außen öffnen kann. In den Tubus 477 ist ein Endoskop 4710 eingeführt. Im Schwammkörper 471 liegt ein Drainageschlauch 472 mit seitlichen Öffnungen 472a. Die Vakuumdrainage befindet sich in einem Darmabschnitt, von dem eine Darmwand 4713 angedeutet ist.

Figur 48 eine Darstellung der Vakuumdrainage aus Figur 47, wobei in dieser Darstellung an den Drainageschlauch 472 ein Unterdruck angelegt ist. Der Schwammkörper 471 ist daher kollabiert, und die Darmwand 4713 hat sich an den Schwammkörper 471 angelegt. Bewegliche Enden 477b des Tubus 477 sind in Pfeilrichtung nach außen geklappt.

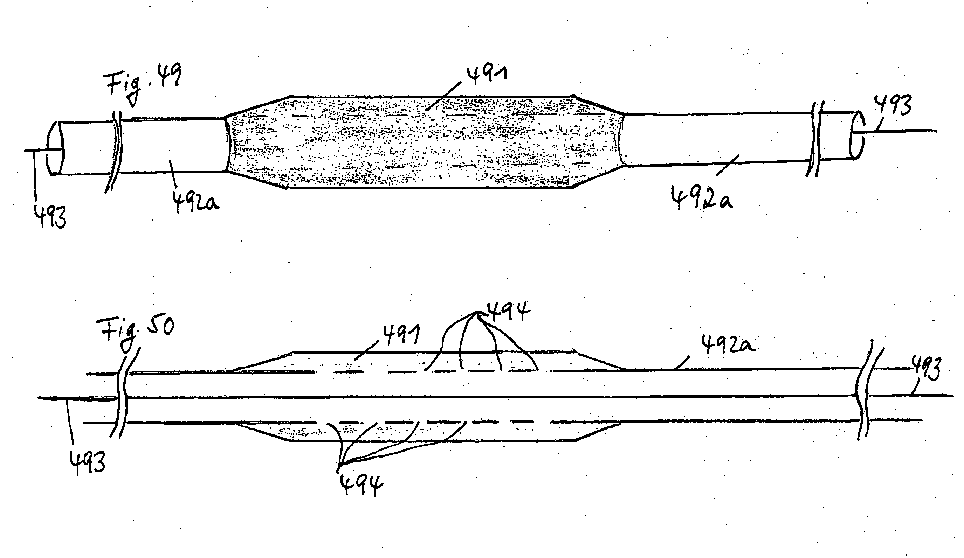

Figur 49 ist eine Darstellung einer weiteren Ausführungsform einer Vakuumdrainage, die im Rahmen dieser Anmeldung mit gleicher Bedeutung auch als Schwammdrainage bezeichnet wird. Ein Schwammkörper 491 ist auf einem Drainageschlauch 492a befestigt. Der Drainageschlauch 492 tritt proximal und distal aus dem Schwammkörper aus. In den Drainageschlauch 492a ist ein Führungsdraht 493 eingeführt worden.

Figur 50 ist eine Querschnittdarstellung der Schwammdrainage aus Figur 49. Der Schwammkörper 491 ist auf dem Drainageschlauch 492a über den Perforationsöffnungen 494 befestigt. Ein Führungsdraht 493 ist in den Drainageschlauch eingeführt.

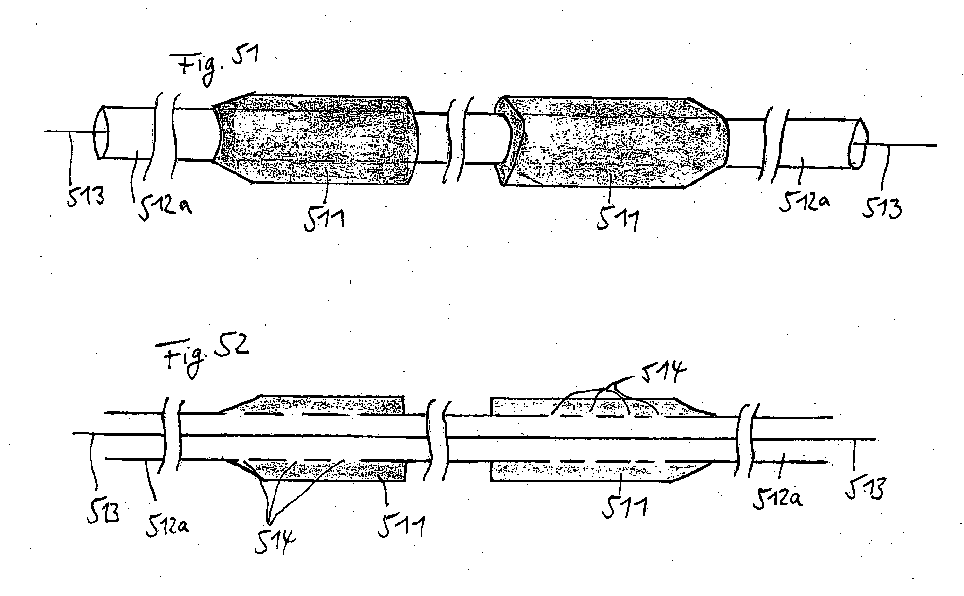

Figur 51 ist eine Darstellung einer weiteren Ausführungsform einer Schwammdrainage. Zwei Schwammkörper 511 sind in einem Abstand auf einem Drainageschlauch 512a befestigt. In den Drainageschlauch 512a ist ein Führungsdraht 513 eingeführt worden. Diese Ausführung ist vorteilhaft, wenn beispielsweise ein Darmabschnitt mit einer Fistel funktionell ausgeschaltet werden soll.

Figur 52 ist eine Querschnittdarstellung der Schwammdrainage von Figur 51. Erkennbar sind die zwei Schwammkörper in einem Abstand auf dem. Drainageschlauch 512a über Perforationsöffnungen 514 befestigt. Der Führungsdraht 513 ist in den Drainageschlauch eingeführt.

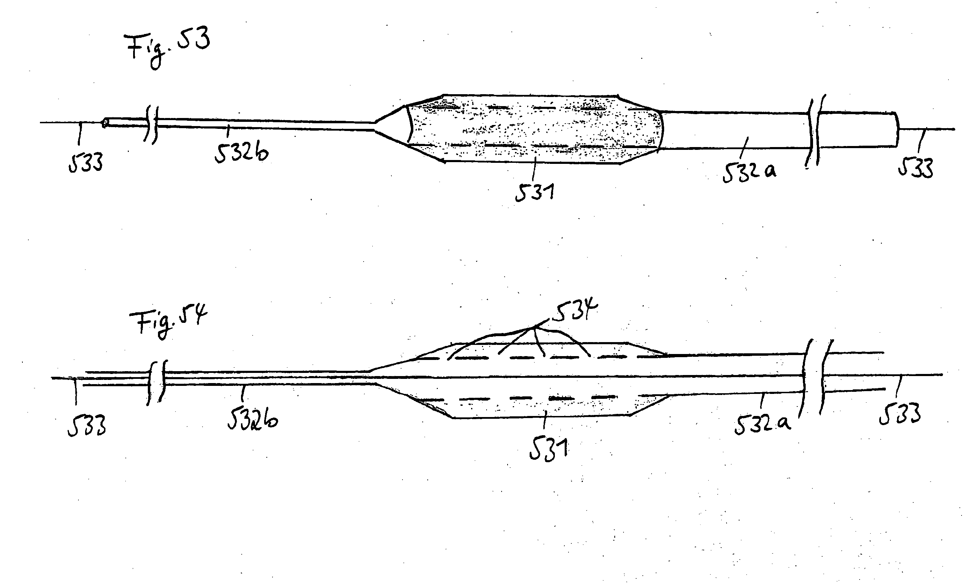

Figur 53 ist eine Darstellung einer weiteren Ausführungsform einer Schwammdrainage. Ein Schwammkörper 531 ist auf einem Drainageschlauch 532a befestigt. Der Drainageschlauch 532a verjüngt sich zu einem dünnlumigen Drainageschlauch 532b. In den Drainageschlauch ist ein Führungsdraht 533 eingeführt.

Figur 54 ist eine Querschnittdarstellung der Schwammdrainage von Figur 53. Der Schwammkörper 531 ist auf dem Drainageschlauch 532a über Perforationsöffnungen 534 befestigt. Der Drainageschlauch 532a verjüngt sich zum dünnlumigen Drainageschlauch 532b. Der Führungsdraht 533 ist in den Drainageschlauch eingeführt.

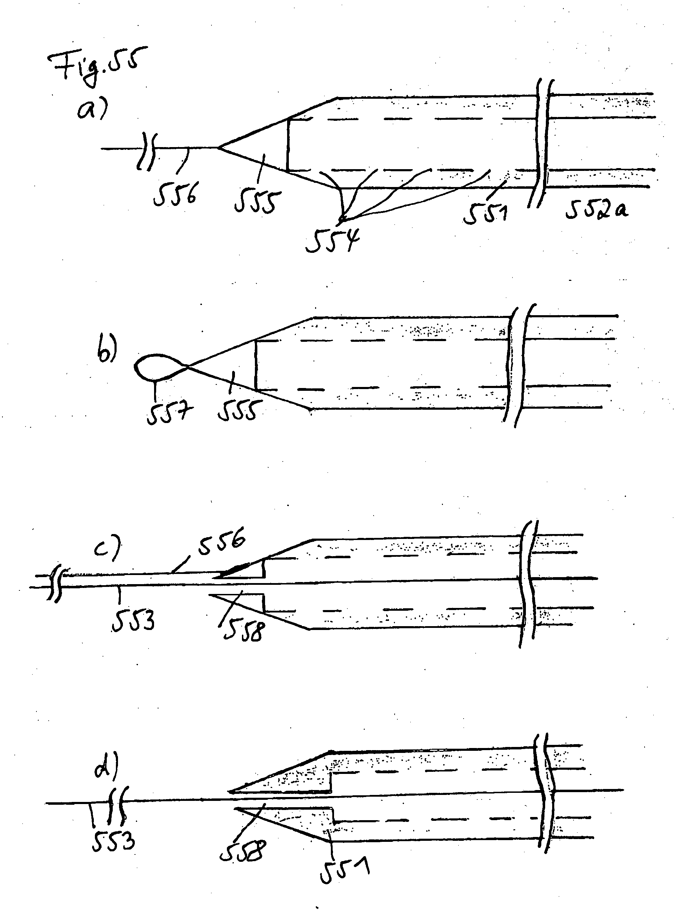

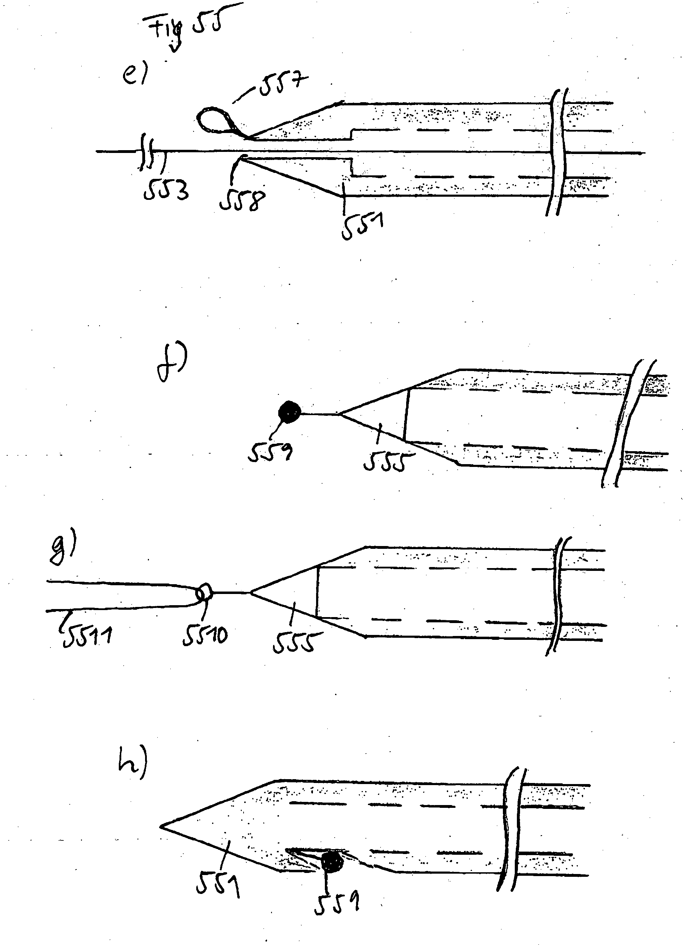

Figuren 55a bis h zeigen verschiedene Varianten eines distalen Endes einer Schwammdrainage 551 in jeweiligen Querschnittdarstellungen. Der Schwammkörper 551 ist auf einem Drainageschlauch 552a über Perforationsöffnungen 554 befestigt. Der Drainageschlauch 552a endet in einer Spitze 555. In Figur 55a ist an der Spitze 555 ein Faden 556 befestigt. In Figur 55b ist an der Spitze 555 eine Faden- oder Drahtschlaufe 557 befestigt. In Figur 55c ist an der Spitze 555 ein Faden 556 befestigt. Die Spitze hat hier jedoch einen Kanal 558, durch die ein Führungsdraht 553 durchgeführt werden kann. In Figur 55d ist der Schwammkörper 551 an seinem distalen Ende als Spitze gestaltet. Der Schwammkörper hat auch hier einen Kanal 558, durch den ein Führungsdraht 553 gelegt wurde. In Figur 55e ist der Schwammkörper 551 am distalen Ende ebenfalls als Spitze gestaltet. Der Schwammkörper 551 hat einen Kanal 558, durch den ein Führungsdraht 553 gelegt wurde. Am Schwammkörper ist eine Faden- oder Drahtschlaufe 57 befestigt. In Figur 55f ist an der Spitze 555 eine Greifperle 559 befestigt. In Figur 55g ist an der Spitze eine Öse 5510 befestigt, durch die ein Faden gezogen 5511 wurde. In Figur 55h liegt die Greifperle 559 in dem Schwammkörper 551.

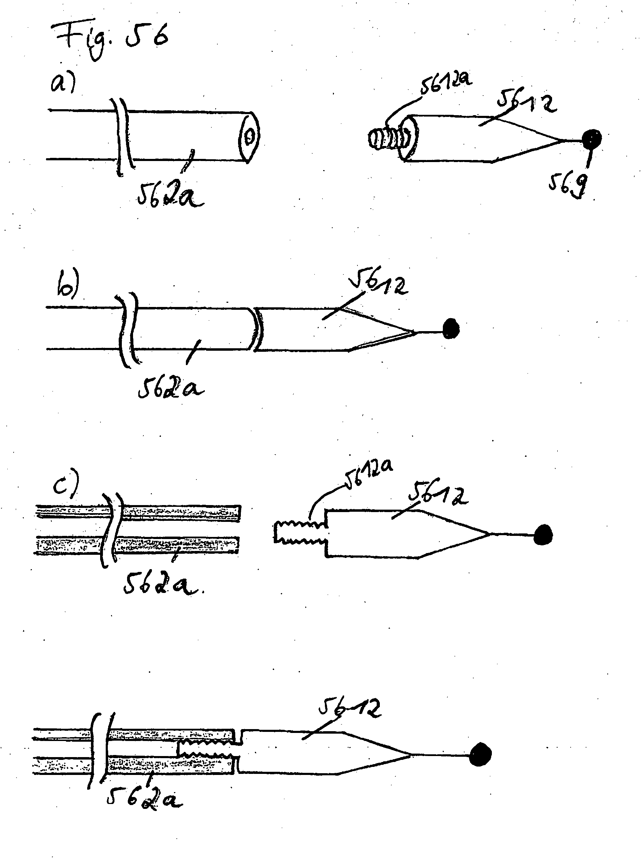

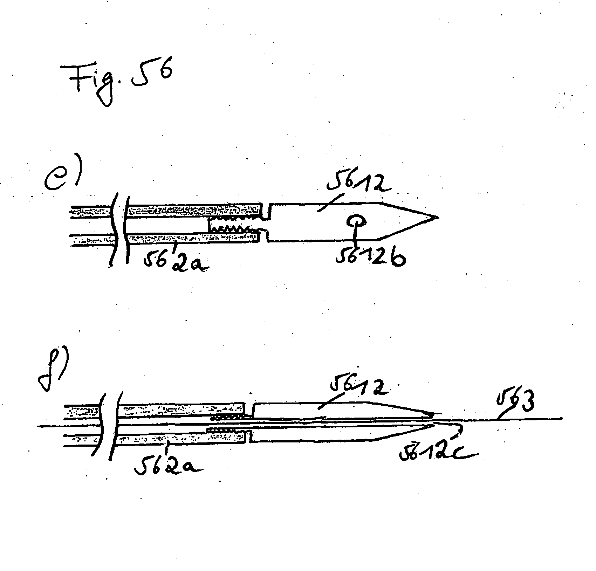

Figuren 56a bis f sind verschiedene Darstellungen eines Drainageschlauches 562a und Aufsatzspitzen 5612. Figur 56a ist eine Darstellung eines Drainageschlauches 562a und einer Aufsatzspitze 5612. Die Aufsatzspitze hat am distalen Ende eine Greifperle 569, am proximalen Ende ein Schraubgewinde 5612a. Figur 56b ist eine Darstellung, bei der die Aufsatzspitze 5612 auf den Drainageschlauch 562a aufgeschraubt ist. Figur 56c ist eine Längsschnittdarstellung von Figur 56a mit Drainageschlauch 562a und Aufsatzspitze 5612. Figur 56d ist eine Längsschnittdarstellung von Figur 56c, mit der auf den Drainageschlauch 562a aufgeschraubten Aufsatzspitze 5612. Figur 56e ist eine Längsschnittdarstellung der auf den Drainageschlauch 562a aufgeschraubten Aufsatzspitze 5612, die mit einem queren Kanal 5612b ausgerüstet ist. Figur 56f ist eine Längsschnittdarstellung einer Variante der auf den Drainagekanal 562a aufgeschraubten Aufsatzspitze 5612. Die Aufsatzspitze ist mit einem Kanal 5612c ausgerüstet. Durch den Kanal 5612c und Drainageschlauch ist ein Führungsdraht 563 eingeführt.

Nachfolgend werden neue, für die Vakuumendoskopie geeignete Legeinstrumente beschrieben.

Endoskopische Lege- oder Greifinstrumente dienen der Platzierung der Vakuumdrainagen. Die Platzierung kann entweder in orthograder Vorschiebetechnik oder in (Durch-) Zugtechnik vorgenommen werden. Bei der orthograden Platzierung ist ein endoskopisches Legeinstrument in den Arbeitskanal des Endoskopes oder einen äußeren Arbeitskanal eingeführt. Es wird an dem distalen Ende des Endoskopes ausgeführt. Die Platzierung der Drainage in (Durch-) Zugtechnik wird angewandt, wenn die zu versorgende Wunde zum einen endoskopisch von innen über einen natürlichen oder künstlichen Zugangsweg erreicht werden kann und zum anderen noch ein weiterer äußerer Zugangsweg,beispielsweise in Form einer äußeren Fistel,besteht.

Die (Durch-) Zugtechnik findet auch ihre Anwendung wenn die endoskopische Vakuumtherapie in Kombination mit offenen oder laparothorakoskopischen Operationen angewandt wird (Rendezvousverfahren). Sie kann auch zur Einlage von konventionellen Drainagen bei der Laparoskopie angewandt werden.

Am Beispiel einer Therapie bei einer Speiseröhrenundichtigkeit mit Fistelung nach außen soll die Zugtechnik dargestellt werden. Mit Hilfe eines Führungsdrahtes oder eines Endoskopes wird das Legeinstrument von außen über die Fistelöffnung bis in die Speiseröhre vorgelegt. Gleichzeitig wird ein Endoskop über den Mund in die Speiseröhre eingeführt und bis zur Undichtigkeitsstelle vorgeführt. Wenn das Legeinstrument an der undichten Stelle der Speiseröhre angekommen ist, wird es mit einer Schlinge gefasst und retrograd durch den Mund wieder ausgeführt. Das Legeinstrument wird mit seinem Befestigungsmechanismus an dem distalen Ende des Fluidkommunikationselementes, der Aufsatzspitze oder dem Schwammkörper angekoppelt und befestigt. Unter endoskopischer Sicht wird dann an dem Legeinstrument gezogen, die Drainage tritt unter Zug über den Mund in die Speiseröhre ein. Die exakte Positionierung wird endoskopisch überdie Speiseröhre kontrolliert. Das Legeinstrument wird wieder von der Koppelung an der Drainage gelöst und durch weiteres Ziehen entfernt. Wenn die Spitze des Fluidkommunikationselementes, Aufsatzspitze oder Schwammkörper mit einem Faden armiert ist, kann das obige Manöver mit dem Faden unter Nutzung der obigen Technik vorgenommen werden.

Besonders vorteilhaft ist das (Durch-) Zugverfahren auch anwendbar, wenn eine Drainagenkonstruktion gewählt wurde, bei der der Schwammkörper im mittleren Abschnitt des Fluidkommunikationselementes liegt. Dann kann der Schwammkörper durch das Ziehen an einem der Enden des Fluidkommunikationselements positioniert werden. Es kann dann sowohl über nur einen Schenkel des Fluidkommunikationselementes, gleichzeitig über beide Schenkel oder wechselnd gesaugt werden.

Das Legeinstrument besteht aus einem Perlengreifer. In einer Kunststoffhülse ist eine Seele aus Metall oder Kunststoff eingeführt. Die Seele spaltet sich an ihrem distalen Ende zwei- oder mehrblättrig auf. Es besteht am distalen Ende der Seele eine Spannung der Blätter nach außen, so dass sie sich beim Heraustreten aus dem distalen Ende der Hülse blühtenartig öffnet und beim Zurückziehen in die Hülse schließt. Die Blätter sind am Ende löffelartig ausgeformt, so dass beim Schließen der Seele ein kugel oder linsenförmiger Hohlraum entsteht. Am distalen Ende verbleibt nach dem Schließen eine kleine Öffnung: In die blütenartig geöffnete Seele kann die Greifperle der Aufsatzspitze, des Fluidkommunikationselementes oder Fluidsammelelement eingelegt werden. Wenn die Seele geschlossen ist, sitzt die Perle fest ein. Beim Öffnen löst sie sich wieder leicht aus und Legeinstrument und Greifperle sind entkoppelt.

Besonders vorteilhaft ist das Legeinstrument zum Aufnehmen eines Führungsdrahts ausgebildet. Der Perlengreifer kann in den Arbeitskanal eines Endoskopes eingeführt werden. Das Legeinstrument ist insbesondere 80 cm bis 250 cm lang.

Ein weiteres Legeinstrument besteht aus einem Haken. In eine Kunststoffhülse ist eine drahtförmige Seele aus Metall- oder Kunststoff eingeführt. Am distalen Ende ist die Seele mit einem Haken ausgerüstet, mit dem eine Fadenschlaufe oder eine Öse gefasst werden kann. Nach dem Ausfahren des Hakens aus der Hülse kann die Fadenschlaufe oder Öse der Ansatzspitze, des Fluidkommunikationselementes oder Fluidsammelelement durch das Zurückziehen des Hakens befestigt werden. Beim Öffnen des Hakens löst sich die Verbindung wieder. Besonders vorteilhaft lässt sich in das Legeinstrument ein Führungsdraht einbringen. Das Haken kann in den Arbeitskanal eines Endoskopes eingeführt werden.

Weiterhin hat es sich als hilfreich erwiesen, an der Spitze eine zugfest befestigte Greifperle, Faden- oder Drahtschlaufe, Öse und/oder ein Faden vorzusehen wobei die Spitze im letzteren Fall einen querverlaufenden Kanal aufweist, in den der Faden eingeführt werden kann.

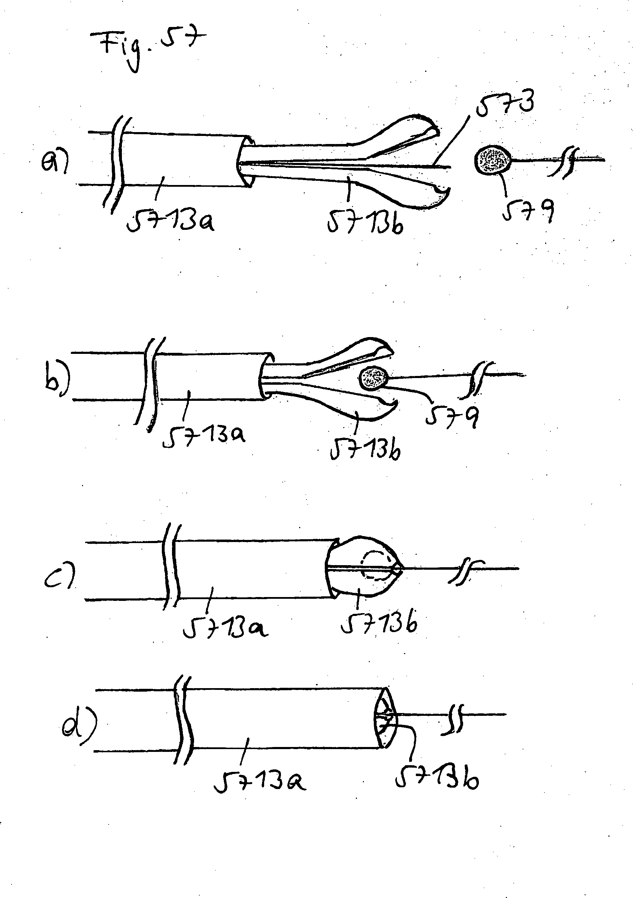

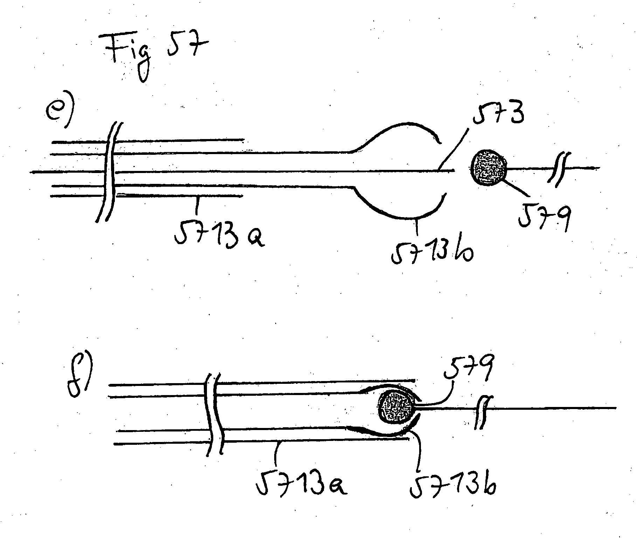

Figuren 57a bis f sind verschiedene Darstellungen eines endoskopischen Legeinstrumentes 5713a/b, mit dem eine Greifperle 579 gegriffen werden kann. Figur 57a ist eine Darstellung des geöffneten Instrumentes. Aus einer Hülse 5713a ist eine zweiblättrige Seele 5713b herausgeführt, die sich geöffnet hat. Außerdem tritt ein Führungsdraht 573 aus der Hülse heraus. In Figur 57b ist der Führungsdraht 573 zurückgezogen, die Greifperle 579 wird mit der Seele 5713b gegriffen. In Figur 57c ist dargestellt, wie die Greifperle gegriffen wurde. Die Seele 5713b wurde in die Hülse 5713a zurückgezogen, dabei hat sich die Seele 5713b geschlossen. In Figur 57d ist die geschlossene Seele 5713b dargestellt, die die Greifperle 579 gegriffen hat, und in die Hülse 5713a zurückgezogen ist. Figur 57e ist eine Längsschnittdarstellung von 57a mit Hülse 5713a, geöffneter Seele 5713b, Führungsdraht 573 und Greifperle 579. Figur 57f ist eine Längsschnittdarstellung von 57d. Die geschlossene Seele 5713b ist mit der gegriffenen Greifperle 579 in die Hülse 5713a zurückgezogen worden.

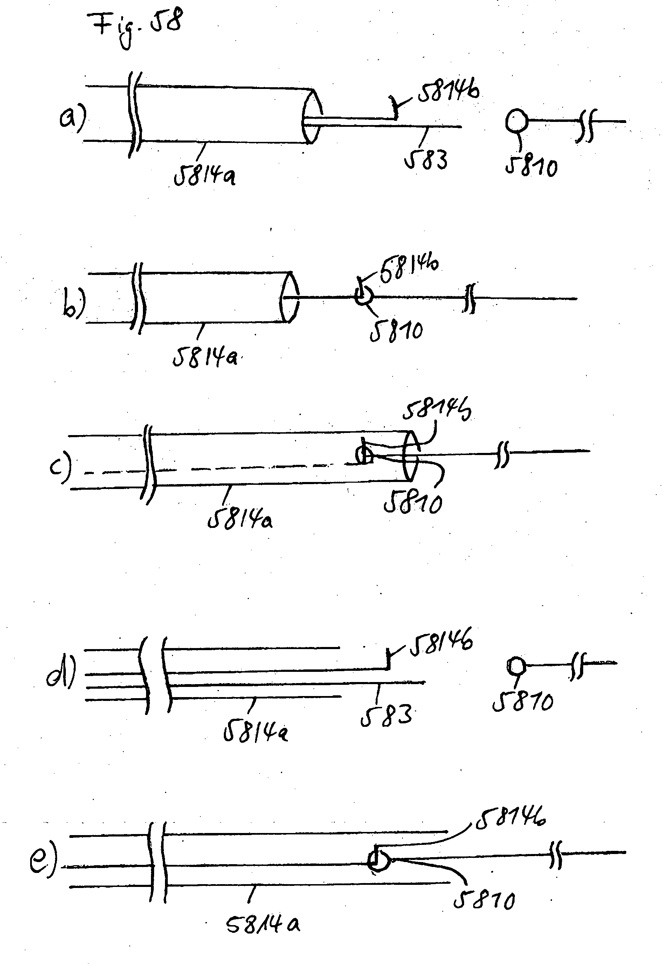

Figuren 58a bis e sind verschiedene Darstellungen eines weiteren endoskopischen Legeinstrumentes, mit dem eine Öse 5810 gegriffen werden kann. Figur 58a ist eine Darstellung des geöffneten Instrumentes. Aus einer Hülse 5814a ist ein Haken 5814b herausgeführt. Außerdem tritt ein Führungsdraht 583 aus der Hülse aus. In Figur 58b ist der Führungsdraht 583 zurückgezogen, die Öse 5810 wird mit Haken 5814b gegriffen. In Figur 58c ist dargestellt, wie der Haken mit der gegriffenen Öse 5810 in die Hülse 5814a zurückgezogen wurde. Figur 58d ist eine Längsschnittdarstellung des Legeinstrumentes der Figur 58a, mit Hülse 5814a, Haken 5814b, Führungsdraht 583 und Öse 5810. Figur 58e ist eine Längsschnittdarstellung von Fig. 58c. Der Haken 5814b ist mit der gegriffenen Öse 5810 in die Hülse 5814a zurückgezogen worden.

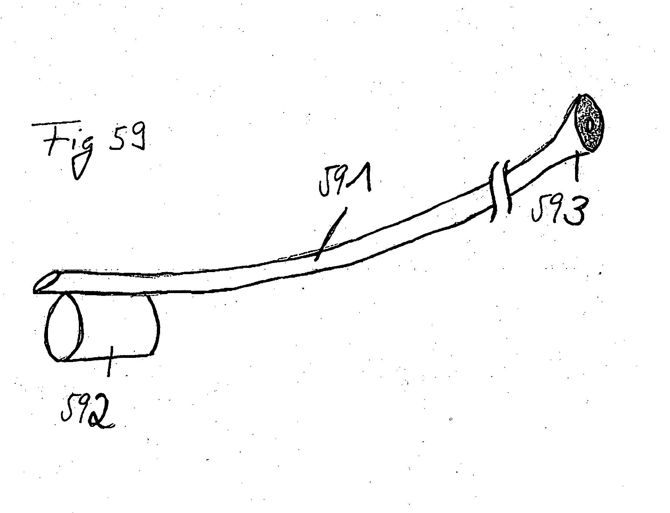

Fig. 59 ist eine Darstellung einer Einführhilfe 591 mit einer Hülse 592 zur Befestigung auf einem Distalende eines Endoskops. Die Einführhilfe 591 ist am distalen Ende angeschrägt, wobei die längere Seite der Anschrägung auf dem Endoskop zu liegen kommt, um Verletzungen beim Einführen des Endoskops zu vermeiden. Am proximalen Ende befindet sich ein Ventil 593, um das Austreten des Untersuchungsgases zu verhindern.

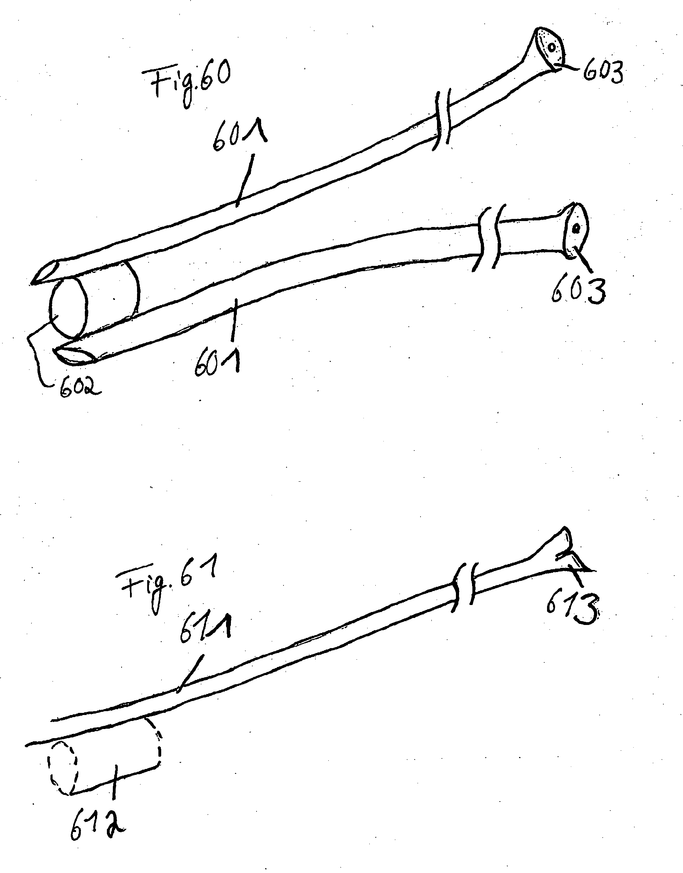

Fig. 60 ist eine Darstellung mit 2 Einführhilfen 601 unterschiedlichen Kalibers.

Fig. 61 zeigt einen Längsschnitt einer Einführhilfe 611, mit einer Befestigungshülse 612 mit Ventil 613.

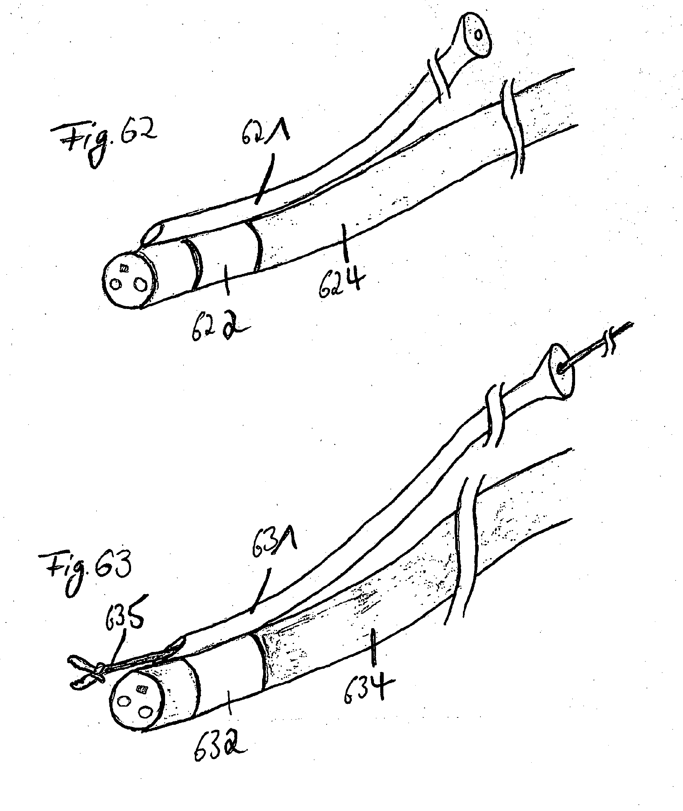

Fig. 62 zeigt eine Darstellung einer Einführhilfe 621 mit einer Befestigungshülse 622 auf einem distalen Ende eines Endoskops 624.

Fig. 63 ist eine Darstellung einer Einführhilfe 631 mit einer Befestigungshülse 632 auf einem distalen Ende eines Endoskops 634. In die Einführhilfe ist eine endoskopische Zange 635 eingeführt worden.

## Patentansprüche

1. Vakuumsystem für die endoskopische intracavitäre, intraluminale oder intracorporale Vakuumtherapie, zum Absaugen von Körperflüssigkeiten, Wundsekreten oder Gasen aus einem Hohlvolumen wie einer Körperhöhle, einem Hohlorgan, einem Gewebeabszess oder einem Intestinallumen, insbesondere beim Herstellen eines passageren endoskopischen Verschlusses eines Intestinallumens, wobei das Vakuumsystem umfasst:
- eine Vakuumpumpe, die einen Steuereingang zum Empfang eines Steuersignals für eine Steuerung ihrer Saugleistung hat und die unterdruckseitig einen Anschluss für eine Vakuumdrainageanordnung aufweist, und
- eine mit dem Steuereingang der Vakuumpumpe verbundene oder verbindbare Druck-Regeleinheit,
-- die einen Messsignaleingang zum Empfang mindestens eines Druck-Messsignals aufweist, welches ein Maß für einen am zu behandelnden Hohlvolumen herrschenden Druck oder Unterdruck bildet,
-- und die ausgebildet ist,
nach Vorgabe
a) eines Unterdruckwertes an dem zu behandelnden Hohlvolumen, der aus einem vordefinierten Unterdruck-Werteintervall wählbar ist, und
b) einer Evakuierungszeitspanne, deren zwischen 0,5 und 5 Sekunden liegender Wert wählbar ist,
i) unter Einrechnen eines vorbestimmten Totvolumens der an die Vakuumpumpe anschließbaren Vakuumdrainageanordnung eine zum Herstellen des vorgegebenen Unterdrucks an dem zu behandelnden Hohlvolumen in der vorgegebenen Evakuierungszeitspanne erforderliche erste Saugleistung der Vakuumpumpe zu ermitteln und dem Steuereingang der Vakuumpumpe ein entsprechendes erstes Steuersignal zuzuführen,
ii) nach Herstellen des vorgegebenen Unterdrucks am zu behandelnden Hohlvolumen das Druck-Messsignal zu überwachen und in Abhängigkeit vom aktuellen Druck-Messsignal eine zur Aufrechterhaltung des vorgegebenen Unterdrucks erforderliche zweite Saugleistung der Vakuumpumpe zu ermitteln und dem Steuereingang der Vakuumpumpe ein entsprechendes zweites Steuersignal zuzuführen; und
iii) nach Herstellen des vorgegebenen Unterdrucks am zu behandelnden Hohlvolumen bei Vorliegen einer eine vordefinierte Schwelle überschreitenden Abweichung des gemessenen Drucks oder Unterdrucks vom vorgegebenen Unterdruck eine zum Herstellen des vorgegebenen Unterdrucks innerhalb der vorgegebenen Evakuierungszeitspanne erforderliche dritte Saugleistung zu ermitteln und dem Steuereingang der Vakuumpumpe ein entsprechendes drittes Steuersignal zuzuführen; wobei
- die Vakuumpumpe ausgebildet ist, unterdruckseitig in Abhängigkeit von dem an ihrem Steuereingang aktuell anliegenden Steuersignal eine durch das Steuersignal bestimmte Saugleistung zu erzeugen.

2. Vakuumsystem nach Anspruch 1, bei dem das vordefinierte Unterdruck-Werteintervall sich über Unterdruckwerte gegenüber einem Umgebungsdruck zwischen einem minimalen Unterdruck von 60mmHg und einem maximalen Unterdruck von 500mmHg erstreckt.

3. Vakuumsystem nach Anspruch 1, welches zusätzlich eine mit der Druck-Regeleinheit verbundene Nutzereingabeeinheit umfasst, die ausgebildet ist, eine Nutzereingabe der Evakuierungszeitspanne und/oder eines Unterdruckwertes anzunehmen und der Druck-Regeleinheit zuzuleiten, und bei dem die Druck-Regeleinheit ausgebildet ist, das betreffende Steuersignal unter Einrechnung der aktuellen Nutzereingabe zu ermitteln und dem Steuereingang der Vakuumpumpe zuzuleiten.

4. Vakuumsystem nach einem der vorherigen Ansprüche, bei dem die Nutzereingabeeinheit einen sperrbaren Modus-Schalter aufweist, der eine nutzerseitige Einstellung entweder eines Therapiemodus oder eines Endoskopiemodus erlaubt, wobei die Druck-Regeleinheit ausgebildet ist, im Therapiemodus nur das zweite oder dritte Steuersignal, jedoch nicht das erste Steuersignal auszugeben, und wobei das vordefinierte Unterdruck-Werteintervall sich im Therapiemodus über Unterdruckwerte gegenüber einem Umgebungsdruck zwischen einem minimalen Unterdruck von 60mmHg und einem maximalen Unterdruck von 250mmHg erstreckt.

5. Vakuumsystem nach einem der vorherigen Ansprüche, mit einer Vakuumdrainageanordnung, die mit der Vakuumpumpe unterdruckseitig verbunden ist und die einen unterdruckstabilen Sekretauffangbehälter aufweist, der ausgebildet ist, im Betrieb anfallendes Sekret und Gas, welches durch die Vakuumpumpe angesaugt wird, aufzunehmen und/oder abzuleiten, und bei dem die Drück-Regeleinheit ausgebildet ist, ein Volumen des Sekretauffangbenhälters als Teil des Totvolumens einzurechnen.

6. Vakuumsystem nach Anspruch 5, bei dem die Vakuumdrainageanordnung zusätzlich einen Vorsekretauffangbehälter aufweist, der dem Sekretauffangbehälter vorgeschaltet und mit diesem fluidleitend verbunden ist, und bei dem die Druck-Regeleinheit ausgebildet ist, ein Volumen des Vorsekretauffangbehälters als weiteren Teil des Totvolumens einzurechnen.

7. Vakuumsystem nach Anspruch 5 oder 6, bei dem die Druck-Regeleinheit ausgebildet ist, ein zusätzliches Volumen, welches mindestens ein unterdruckstabiles Fluidkommunikationselement, insbesondere ein Drainageschlauch bildet, der distal mit einem Fluidsammelelement und proximal mit dem Sekretauffangbehälter oder dem Vorsekretauffangbehälter verbindbar ist, als weiteren Teil des Totvolumens einzurechnen.

8. Vakuumsystem nach einem der vorherigen Ansprüche, bei dem die Vakuumpumpe eine Pumpenkombination mindestens zweier Pumpeinheiten umfasst, von denen eine erste Pumpeinheit ausgebildet ist, ein Vorvakuum zu erzeugen, das einen geringeren Unterdruck aufweist ist als der vorgegebene Unterdruck, und eine zweite Pumpeinheit ausgebildet ist, nach Herstellen des Vorvakuums das Vakuum herzustellen.

9. Vakuumsystem nach einem der vorherigen Ansprüche, bei dem die Druck-Regeleinheit ausgebildet ist, der Vakuumpumpe initial und vorrübergehend einen vorbestimmbaren ersten, höheren Unterdruck vorzugeben und nach Ablauf einer durch Nutzereingabe bestimmbaren Zeitspanne den Unterdruck auf einen vorbestimmbaren zweiten, vergleichsweise niedrigen Unterdruckwert zu regeln.

10. Vakuumsystem nach einem der vorherigen Ansprüche, bei dem die Vakuumpumpe eine Mehrzahl unterdruckseitiger Anschlüsse für beide Enden eines einzigen Drainageschlauchs oder ein oder mehrere Enden mehrerer Drainageschläuche aufweist, und bei der die Druck-Regeleinheit ausgebildet ist, auf eine entsprechende Nutzereingabe über die Nutzereingabeeinheit hin die Vakuumpumpe anzusteuern, wahlweise entweder einseitig nur an einem der Anschlüsse oder alternierend an zweien der Anschlüsse oder zeitgleich an zwei Anschlüssen zu saugen oder zu spülen.

11. Endoskopie-Anordnung mit
- einem Vakuumsystem nach einem der vorstehenden Ansprüche,
- einer Overtubeeinheit, welche unterdruckseitig mit der Vakuumpumpe des Vakuumsystems durch mindestens ein Fluidkommunikationselement verbunden ist und die ein Fluidsammelelement trägt,
- einem Endoskop, welches in die Overtubeeinheit eingeführt oder einführbar ist und relativ zur Overtubeeinheit in einer von proximal nach distal oder umgekehrt weisenden Richtung verschiebbar ist und
- einer Unterdruckmesssonde, die mit der Druck-Regeleinheit des Vakuumsystems verbunden ist.

12. Endoskopie-Anordnung nach Anspruch 11, bei der das Endoskop unterdruckseitig mit der Vakuumpumpe des Vakuumsystems durch ein Fluidkommunikationselement in Form von Drainageschläuchen und/oder in Form eines Kanals im Endoskop verbunden ist und ein weiteres Fluidsammelelement trägt.

13. Endoskopie-Anordnung nach Anspruch 11 oder 12, bei der das Fluidsammelelement am distalen Ende der Overtubeeinheit und/oder das weitere Fluidsammelelement am distalen Ende des Endoskops befestigt ist.

14. Endoskopie-Anordnung nach Anspruch 12 oder 13, bei dem das Endoskop mindestens einen in seinem inneren verlaufenden Arbeitskanal aufweist, welcher Perforationsöffnungen nach außen aufweist, über die das weitere Fluidsammelelement fluidleitend verbunden ist.

15. Endoskopie-Anordnung nach einem der Ansprüche 11 bis 14, bei der das Fluidsammelelement oder das weitere Fluidsammelelement einen Polyurethanschwamm aufweist.

16. Endoskopie-Anordnung nach einem der. Ansprüche 11 bis 15, bei der das Fluidsammelelement oder das weitere Fluidsammelelement eine offenporige fluidleitende Folie ist oder bei der das Fluidsammelelement oder das weitere Fluidsammelelement zusätzlich an seiner Außenfläche eine offenporige fluidleitende Folie aufweist.

17. Endoskopie-Anordnung nach Anspruch 16, bei der die Folie zum Endoskop hin fluidleitend ausgebildet ist.

18. Endoskopie-Anordnung nach einem der Ansprüche 11 bis 17, bei der das Endoskop als das weitere Fluidsammelelement einen Polyurethanschwamm trägt, während die Overtubeeinheit als das Fluidsammelelement eine Folie trägt.

19. Endoskopie-Anordnung nach einem der Ansprüche 11 bis 18, mit einem Vakuumsystem nach Anspruch 9, bei der beide Längsenden des Drainageschlauches einen Anschluss zum Verbinden mit der Vakuumpumpe aufweisen, und bei der die Schwammdrainageeinheit zwischen den Längsende des Drainageschlauches befestigt ist.

20. Endoskopie-Anordnung nach einem der Ansprüche 11 bis 19, bei der das Fluidsammelelement und/oder das weitere Fluidsammelelement abschnittsweise mit einer Oberflächenversiegelung zum Verschluss offener Poren versehen ist und in anderen Abschnitten die Oberflächenversiegelung nicht aufweist.

21. Endoskopie-Anordnung nach einem der Ansprüche 11 bis 20, bei der der Drainageschlauch distal mit einer konischen Spitze endet.

22. Endoskopie-Anordnung nach Anspruch 21, bei der an der Spitze des Drainageschlauchs eine Greifperle, Faden- loder Drahtschlaufe, Öse und/oder ein Faden zugfest befestigt ist; wobei die Spitze im letzteren Fall einen querverlaufenden Kanal aufweist, in den der Faden eingeführt werden kann.

23. Endoskopie-Anordnung nach Anspruch 22, bei der die distale Spitze des Drainageschlauchs eine projektilartige Aufsatzspitze aus Kunststoff oder Metall ist, die mit einem Steck- und/oder Schraubelement zugfest an dem Ende des Drainageschlauches befestigbar ausgebildet ist.

24. Endoskopie-Anordnung nach einem der Ansprüche 11 bis 23, bei der der Drainageschlauch ausgebildet ist, das Einführen eines Führungsdrahtes in das Lumen des Drainageschlauchs zu gestatten und bei der hierfür eine distale Spitze des Drainageschlauches und die Aufsatzspitze einen Längskanal aufweisen, in den der Führungsdraht eingeführt werden kann.

25. Endoskopie-Anordnung nach einem der Ansprüche 11 bis 24, bei der ein Schwammkörper des Fluidsammelelements distal mit einer Spitze endet und an der Spitze und/oder in dem Schwammkörper integriert eine Greifperle, Faden- oder Drahtschlaufe, Öse und/oder ein Faden zugfest befestigt ist.

26. Endoskopie-Anordnung nach einem der Ansprüche 11 bis 25, bei der eine der Unterdruckmesssonden in, auf oder an dem distal angeordneten Fluidsammelelement angeordnet und mit der Druck-Regeleinheit des Vakuumsystems verbunden ist.

27. Endoskopie-Anordnung nach Anspruch 26, bei der die Unterdruckmesssonde drahtförmig konstruiert ist und über den Drainageschlauch zum Fluidsammelelement herangeführt ist.

28. Endoskopie-Anordnung nach einem der Ansprüche 11 bis 27, bei der der Overtube proximal und distal an das auf ihm aufsitzende Fluidsammelelement unmittelbar angrenzend je eine ringförmige lippenartige Verdickung aufweist.

29. Endoskopie-Anordnung nach einem der Ansprüche 11 bis 28, bei der der Drainageschlauch an unterschiedlichen Längsabschnitten unterschiedliche Durchmesser hat.

30. Endoskopie-Anordnung nach einem der Ansprüche 11 bis 29, bei der das Fluidsammelelement einen Schwammkörper hat, der an seiner Außenfläche mindestens eine Aussparung oder in seinem Inneren einen Kanal zur Aufnahme einer Sonde aufweist, die sich zum Betrieb der Endoskopie-Anordnung in die Aussparung bzw. den Kanal einlegen lässt.

31. Endoskopie-Anordnung nach einem der Ansprüche 11 bis 30, bei dem in einem Schwammkörper des Fluidsammelelements ein von proximal nach distal geführter Kanal angeordnet ist, durch den ein mit Poren des Schwammkörpers nicht fluidleitend verbundener Tubus zum Durchleiten von Köiperflüssigkeiten wie Sekreten oder Speichel führt.

32. Endoskopie-Anordnung nach Anspruch 31, bei der der Tubus unterdruckstabil ist und eine Länge von 5 bis 20 cm und einen inneren Durchmesser von 5 bis 20 mm aufweist.

33. Endoskopie-Anordnung nach Anspruch 31 oder 32, bei der der Tubus innen hydrophil ist und bei dem eine Oberflächenversiegelung des Fluidsammelelementes hydrophil ist.

34. Endoskopie-Anordnung nach einem der Ansprüche 31 bis 33, bei der ein proximales und/oder ein distales Ende des Tubus gegenüber einem Mittelteil des Tubus nach außen beweglich und unter Unterdruckanlage aufspreizbar ist.

35. Endoskopie-Anordnung nach einem der Ansprüche 31 bis 34, bei der der Tubus ohne Fixierung im Kanal der Schwammdrainageeinheit liegt.

36. Endoskopie-Anordnung nach einem der Ansprüche 11 bis 35, bei der Außendurchmesser des Fluidkommunikationselements und des Fluidsammelelements an einen Innendurchmesser eines inneren Arbeitskanals des Endoskops angepasst sind, so dass sie innerhalb des Arbeitskanals verschieblich sind und ihre Platzierung über den inneren Arbeitskanal des Endoskops vorgenommen werden kann.

37. Endoskopie-Anordnung nach einem der Ansprüche 11 bis 36, bei dem das Fluidsammelelement einen Schwammkörper aufweist und ein in dem Schwammkörper gelegener Kanal mit einer Oberflächenversiegelung ausgerüstet ist, welche aus einer längsprofilierten Folie gefertigt ist, die in von proximal nach distal weisender Längsrichtung von fluidleitenden Kanälen profiliert ist.

38. Endoskopie-Anordnung nach einem der Ansprüche 11 bis 37, bei dem der Overtube, das Fluidsammelelement, ein Pusher und der äußere Arbeitskanal mit einem über die komplette Länge erstreckenden Längsschlitz versehen sind.

## Claims

1. A vacuum system for endoscopic, intracavitary, intraluminal or intracorporeal vacuum therapy for aspirating body fluids, wound secretions or gases from a hollow volume such as a body cavity, a hollow organ, a tissue abscess or an intestinal lumen, in particular when producing a temporary endoscopic closure of an intestinal lumen, the vacuum system comprising:
- a vacuum pump which has a control input for receiving a control signal for the control of its suction power, and which, on the negative pressure side, has a connection for a vacuum drainage system, and
- a pressure regulating unit connected or that can be connected to the control input of the vacuum pump,
-- and which has a measurement signal input for receiving at least one pressure measurement signal which forms a measure for a pressure or negative pressure prevailing on the hollow volume to be treated,
-- and which is designed
according to the requirements
a) of a negative pressure value on the hollow volume to be treated and which can be chosen from a pre-defined negative pressure value interval, and
b) of an evacuation period, the value of which, lying between 0.5 and 5 seconds, can be chosen,
i) allowing for a pre-determined dead volume of the vacuum drainage system that can be connected to the vacuum pump, to determine a first suction power of the vacuum pump required to produce the pre-specified negative pressure on the hollow volume to be treated in the pre-specified evacuation period, and to deliver a corresponding first control signal to the control input of the vacuum pump,
ii) after establishing the pre-specified negative pressure at the hollow volume to be treated, to monitor the pressure measurement signal, and dependently upon the current pressure measurement signal, to determine a second suction power of the vacuum pump required to maintain the pre-specified negative pressure and to deliver a corresponding second control signal to the control input of the vacuum pump; and
iii) after establishing the pre-specified negative pressure at the hollow volume to be treated, with the presence of a deviation of the measured pressure or negative pressure from the pre-specified negative pressure exceeding a pre-defined threshold, to determine a third suction power required to establish the pre-specified negative pressure within the pre-specified evacuation period and to deliver a corresponding third control signal to the control input of the vacuum pump;
- the vacuum pump being designed to generate a suction power determined by the control signal on the negative pressure side dependently upon the control signal currently lying at its control input.

2. The vacuum system according to Claim 1, wherein the pre-defined negative pressure value interval extends over negative pressure values, with respect to an ambient pressure, between a minimum negative pressure of 60 mmHg and a maximum negative pressure of 500 mmHg.

3. The vacuum system according to Claim 1, which additionally comprises a user input unit connected to the pressure regulating unit and which is designed to receive a user input of the evacuation period and/or of a negative pressure value and to convey it to the pressure regulating unit, and wherein the pressure regulating unit is designed to determine the respective control signal, allowing for the current user input, and to convey it to the control input of the vacuum pump.

4. The vacuum system according to any of the preceding claims, wherein the user input unit has a blockable mode switch which allows user-side setting either of a therapy mode or of an endoscopy mode, the pressure regulating unit being designed to emit only the second or third control signal, but not the first control signal, in the therapy mode, and in the therapy mode the pre-defined negative pressure value interval extending over negative pressure values, with respect to an ambient pressure, between a minimum negative pressure of 60 mmHg and a maximum negative pressure of 250 mmHg.

5. The vacuum system according to any of the preceding claims, comprising a vacuum drainage system which is connected to the vacuum pump on the negative pressure side and which has a negative pressure-stable secretion collection container which is designed to receive and/or convey away any secretion or gas occurring during operation and which is aspirated by the vacuum pump, and wherein the pressure regulating unit is designed to allow for a volume of the secretion collection container as part of the dead volume.

6. The vacuum system according to Claim 5, wherein the vacuum drainage system additionally has a pre-secretion collection container which is connected upstream of the secretion collection container and is connected to the latter such as to convey fluid, and wherein the pressure regulating unit is designed to allow for a volume of the pre-secretion collection container as an additional part of the dead volume.

7. The vacuum system according to Claim 5 or 6, wherein the pressure regulating unit is designed to allow for an additional volume, which forms at least one negative pressure-stable fluid communication element, in particular a drainage tube, that can be connected distally to a fluid collecting element and proximally to the secretion collection container or the pre-secretion collection container, as an additional part of the dead volume.

8. The vacuum system according to any of the preceding claims, wherein the vacuum pump comprises a pump combination of at least two pump units, of which a first pump unit is designed to generate a pre-vacuum which has a lower negative pressure than the pre-specified negative pressure and a second pump unit is designed to establish the vacuum after establishing the pre-vacuum.

9. The vacuum system according to any of the preceding claims, wherein the pressure regulating unit is designed to specify initially and temporarily for the vacuum pump a pre-determinable first higher negative pressure, and after a period of time that can be determined by the user input has passed, to adjust the negative pressure to a pre-determinable second, comparatively low negative pressure value.

10. The vacuum system according to any of the preceding claims, wherein the vacuum pump has a plurality of negative pressure-side connections for both ends of a single drainage tube or one or more ends of a number of drainage tubes, and wherein the pressure regulating unit is designed to control the vacuum pump according to a corresponding user input via the user input unit, optionally to suck or flush either on one side at just one of the connections or alternatively at two of the connections or at two connections at the same time.

11. An endoscopy system comprising
- a vacuum system according to any of the preceding claims,
- an overtube unit which is connected on the negative pressure side to the vacuum pump of the vacuum system by means of at least one fluid communication element and which supports a fluid collecting element,
- an endoscope which is introduced or can be introduced into the overtube unit and can be moved relatively to the overtube unit in a direction pointing from proximal to distal or vice versa, and
- a negative pressure measuring probe which is connected to the pressure regulating unit of the vacuum system.

12. The endoscopy system according to Claim 11, wherein the endoscope is connected on the negative pressure side to the vacuum pump of the vacuum system by a fluid communication element in the form of drainage tubes and/or in the form of a channel in the endoscope and supports an additional fluid collecting element.

13. The endoscopy system according to Claim 11 or 12, wherein the fluid collecting element is fastened to the distal end of the overtube unit and/or the additional fluid collecting element is fastened to the distal end of the endoscope.

14. The endoscopy system according to Claim 12 or 13, wherein the endoscope has at least one working channel running in its interior and which has perforation openings to the outside by means of which the additional fluid collecting element is connected such as to convey fluid.

15. The endoscopy system according to any of Claims 11 to 14, wherein the fluid collecting element or the additional fluid collecting element has a polyurethane sponge.

16. The endoscopy system according to any of Claims 11 to 15, wherein the fluid collecting element or the additional fluid collecting element is an open-pored fluid-conveying film or wherein the fluid collecting element or the additional fluid collecting element additionally has an open-pored, fluid-conveying film on its outer surface.

17. The endoscopy system according to Claim 16, wherein the film is designed to convey fluid towards the endoscope.

18. The endoscopy system according to any of Claims 11 to 17, wherein the endoscope supports a polyurethane sponge as the additional fluid collecting element, while the overtube unit supports a film as the fluid collecting element.

19. The endoscopy system according to any of Claims 11 to 18, comprising a vacuum system according to Claim 9, wherein both longitudinal ends of the drainage tube have a connection for connecting to the vacuum pump, and wherein the sponge drainage unit is fastened between the longitudinal ends of the drainage tube.

20. The endoscopy system according to any of Claims 11 to 19, wherein the fluid collecting element and/or the additional fluid collecting element is partially provided with surface sealing for closing open pores and does not have surface sealing in other sections.

21. The endoscopy system according to any of Claims 11 to 20, wherein the drainage tube ends distally with a conical tip.

22. The endoscopy system according to Claim 21, wherein a gripping bead, a thread or wire loop, an eye and/or a thread is fastened with tensile strength to the tip of the drainage tube; in the last case the tip having a transversely running channel into which the thread can be introduced.

23. The endoscopy system according to Claim 22, wherein the distal tip of the drainage tube is a projectile-like extension tip made of plastic or metal and which, comprising a plug and/or screw element, is designed to be able to be fastened with tensile strength to the end of the drainage tube.

24. The endoscopy system according to any of Claims 11 to 23, wherein the drainage tube is designed to allow the introduction of a guide wire into the lumen of the drainage tube and wherein for this purpose a distal tip of the drainage tube and the extension tip has a longitudinal channel into which the guide wire can be introduced.

25. The endoscopy system according to any of Claims 11 to 24, wherein a sponge body of the fluid collecting element ends distally with a tip, and a gripping bead, a thread or wire loop, an eye and/or a thread is fastened with tensile strength to the tip and/or integrated into the sponge body.

26. The endoscopy system according to any of Claims 11 to 25, wherein one of the negative pressure measuring probes is disposed in, on or against the distally arranged fluid collecting element and is connected to the pressure regulating unit of the vacuum system.

27. The endoscopy system according to Claim 26, wherein the negative pressure measuring probe is designed like a wire and is introduced to the fluid collecting element via the drainage tube.

28. The endoscopy system according to any of Claims 11 to 27, wherein the overtube, directly adjacent proximally and distally to the fluid collecting element sitting on it, respectively has an annular, lip-like thickening.

29. The endoscopy system according to any of Claims 11 to 28, wherein the drainage tube has different diameters at different longitudinal sections.

30. The endoscopy system according to any of Claims 11 to 29, wherein the fluid collecting element has a sponge body which has on its outer surface at least one recess or has on its inside a channel for receiving a probe which can be placed in the recess or the channel for operating the endoscopy system.

31. The endoscopy system according to any of Claims 11 to 30, wherein a channel guided from proximal to distal is disposed in a sponge body of the fluid collecting element, through which channel a lens tube connected to pores of the sponge body such as not to convey fluid leads to the conveyance of body fluids such as secretions or saliva.

32. The endoscopy system according to Claim 31, wherein the lens tube is negative pressure-stable and has a length of 5 to 20 cm and an inner diameter of 5 to 20 mm.

33. The endoscopy system according to Claim 31 or 32, wherein the lens tube is hydrophilic on the inside, and wherein surface sealing of the fluid collecting element is hydrophilic.

34. The endoscopy system according to any of Claims 31 to 33, wherein a proximal and/or a distal end of the lens tube can be moved to the outside with respect to a middle part of the lens tube and can be spread open when negative pressure is applied.

35. The endoscopy system according to any of Claims 31 to 34, wherein the lens tube lies in the channel of the sponge drainage unit without any fixing.

36. The endoscopy system according to any of Claims 11 to 35, wherein the outside diameter of the fluid communication element and of the fluid collecting element are adapted to an inside diameter of an inner working channel of the endoscope so that they can be moved within the working channel and can be positioned by means of the inner working channel of the endoscope.

37. The endoscopy system according to any of Claims 11 to 36, wherein the fluid collecting element has a sponge body and a channel placed in the sponge body is equipped with surface sealing which is produced from a longitudinally profiled film which is profiled by channels conveying fluid in the longitudinal direction pointing from proximal to distal.

38. The endoscopy system according to any of Claims 11 to 37, wherein the overtube, the fluid collecting element, the pusher and the outer working channel are provided with a longitudinal slot extending over the entire length.
I

## Revendications

1. Système à vide pour le traitement endoscopique intracavitaire, intraluminal ou intracorporel, l'aspiration de fluides corporels, de sécrétions de plaies ou de gaz hors d'un volume creux tel qu'une cavité corporelle, un organe creux, un abcès tissulaire ou une lumière intestinale, notamment lors de la réalisation d'une fermeture endoscopique passagère d'une lumière intestinale, le système à vide comprenant :
- une pompe à vide qui possède une entrée de commande destinée à recevoir un signal de commande permettant de commander sa puissance d'aspiration, et qui présente, du côté dépression, une connexion pour un ensemble de drainage aspiratif, et
- une unité de régulation de la pression qui est reliée ou susceptible d'être reliée à l'entrée de commande de la pompe à vide,
-- laquelle présente une entrée de signaux de mesure destinée à recevoir au moins un signal de mesure de la pression, lequel est déterminant d'une pression ou une dépression régnant dans le volume creux à traiter,
-- et laquelle est constituée pour,
après la détermination
a) d'une valeur de dépression au niveau du volume creux à traiter, susceptible d'être choisie dans un intervalle de valeurs de dépression prédéfinies, et
b) d'une période d'évacuation dont la valeur, située entre 0,5 et 5 secondes, peut être choisie,
i) déterminer, en tenant compte d'un volume mort prédéterminé de l'ensemble de drainage aspiratif raccordable à la pompe à vide, une première puissance d'aspiration de la pompe à vide nécessaire à l'obtention de la dépression prédéfinie au niveau du volume creux à traiter dans la période d'évacuation prédéfinie, et apporter à l'entrée de commande de la pompe à vide un premier signal de commande correspondant,
ii) après obtention de la dépression prédéfinie au niveau du volume creux à traiter, surveiller le signal de mesure de la pression et, en fonction du signal de mesure de la pression effectif, déterminer une deuxième puissance d'aspiration de la pompe à vide nécessaire pour maintenir la dépression prédéfinie et apporter à l'entrée de commande de la pompe à vide un deuxième signal de commande correspondant ; et
iii) après obtention de la dépression prédéfinie au niveau du volume creux à traiter, en présence d'un écart dans la pression ou la dépression mesurée par rapport à la dépression prédéfinie, avec dépassement d'un seuil prédéfini, déterminer une troisième puissance d'aspiration nécessaire pour obtenir la dépression prédéfinie à l'intérieur de la période d'évacuation prédéfinie, et apporter à l'entrée de commande de la pompe à vide un troisième signal de commande correspondant ; en sachant que
- la pompe à vide est conçue pour produire, du côté dépression, une puissance d'aspiration qui est fonction du signal de commande effectivement présent au niveau de son entrée de commande et qui est déterminée par le signal de commande.

2. Système à vide selon la revendication 1, dans lequel l'intervalle de valeurs de dépression prédéfinies couvre des valeurs de dépression, par rapport à une pression ambiante, qui vont d'une dépression minimale de 60 mmHg à une dépression maximale de 500 mmHg.

3. Système à vide selon la revendication 1, comprenant en outre une unité d'entrée utilisateur qui est reliée à l'unité de régulation de la pression, laquelle unité d'entrée utilisateur est conçue pour accepter une entrée utilisateur concernant la période d'évacuation et/ou une valeur de dépression et pour la transmettre à l'unité de régulation de la pression, et dans lequel l'unité de régulation de la pression est conçue pour déterminer le signal de commande en question, en tenant compte de l'entrée utilisateur effective, et pour le transmettre à l'entrée de commande de la pompe à vide.

4. Système à vide selon l'une des revendications précédentes, dans lequel l'unité d'entrée utilisateur présente un commutateur de modes verrouillable qui permet un réglage côté utilisateur soit d'un mode de traitement, soit d'un mode endoscopique, l'unité de régulation de la pression étant conçue pour n'émettre, dans le mode de traitement, que le deuxième ou le troisième signal de commande, mais pas le premier signal de commande, et l'intervalle de valeurs de dépression prédéfinies couvrant, dans le mode de traitement, les valeurs de dépression, par rapport à une pression ambiante, qui vont d'une dépression minimale de 60 mmHg à une dépression maximale de 250 mmHg.

5. Système à vide selon l'une des revendications précédentes, comportant un ensemble de drainage aspiratif qui est raccordé à la pompe à vide du côté dépression et qui présente un récipient de recueil de sécrétions stable à la dépression qui est conçu pour recevoir et/ou évacuer, à l'usage, les sécrétion et les gaz produits qui ont été aspirés par la pompe à vide, et dans lequel l'unité de régulation de la pression est conçue pour tenir compte du volume du récipient de recueil de sécrétions en tant que composante du volume mort.

6. Système à vide selon la revendication 5, dans lequel l'ensemble de drainage aspiratif présente en outre un récipient amont de recueil de sécrétions qui est situé en amont du récipient de recueil de sécrétions et qui est raccordé avec ce dernier de manière à permettre la conduction des fluides, et dans lequel l'unité de régulation de la pression est conçue pour tenir compte du volume du récipient amont de recueil de sécrétions en tant que composante supplémentaire du volume mort.

7. Système à vide selon la revendication 5 ou 6, dans lequel l'unité de régulation de la pression est conçue pour tenir compte, en tant que composante supplémentaire du volume mort, d'un volume supplémentaire formant au moins un élément de communication fluidique stable à la dépression, notamment un tube de drainage, qui peut être raccordé distalement à un élément de recueil de fluide et proximalement au récipient de recueil de sécrétions ou au récipient amont de recueil de sécrétions.

8. Système à vide selon l'une des revendications précédentes, dans lequel la pompe à vide comprend un assemblage d'au moins deux unités de pompe dont une première unité de pompe est conçue pour produire un vide préalable présentant une dépression inférieure à la dépression prédéfinie, et une deuxième unité de pompe est conçue pour produire le vide après la production du vide préalable.

9. Système à vide selon l'une des revendications précédentes, dans lequel l'unité de régulation de la pression est conçue pour définir sur la pompe à vide, initialement et de manière temporaire, une première dépression supérieure prédéterminable, et pour régler cette dépression, après l'écoulement d'une période définissable par l'entrée utilisateur, sur une deuxième valeur de dépression prédéterminable qui est comparativement moindre.

10. Système à vide selon l'une des revendications précédentes, dans lequel la pompe à vide présente une pluralité de connexions du côté dépression, pour les deux extrémités d'un tube de drainage unique ou pour une ou plusieurs extrémités de plusieurs tubes de drainage, et dans lequel l'unité de régulation de la pression est conçue pour commander la pompe à vide par le biais d'une entrée utilisateur correspondante transmise via l'unité d'entrée utilisateur afin de réaliser une aspiration ou un rinçage, au choix, soit unilatéralement uniquement au niveau de l'une des connexions, ou bien alternativement au niveau de deux des connexions, ou encore simultanément au niveau de deux connexions.

11. Ensemble endoscopique comportant
- un système à vide selon l'une des revendications précédentes,
- une unité de surtube qui est raccordée, du côté dépression, à la pompe à vide du système à vide par au moins un élément de communication fluidique et qui porte un élément de recueil de fluide,
- un endoscope qui est introduit ou peut être introduit dans l'unité de surtube et coulissé, par rapport à l'unité de surtube, dans un sens orienté de proximal vers distal ou inversement, et
- une sonde de mesure de dépression qui est raccordée à l'unité de régulation de la pression du système à vide.

12. Ensemble endoscopique selon la revendication 11, dans lequel l'endoscope est raccordé, du côté dépression, à la pompe à vide du système à vide par un élément de communication fluidique sous la forme de tubes de drainage et/ou sous la forme d'un canal existant dans l'endoscope, et dans lequel l'endoscope porte un élément supplémentaire de recueil de fluide.

13. Ensemble endoscopique selon la revendication 11 ou 12, dans lequel l'élément de recueil de fluide est fixé à l'extrémité distale de l'unité de surtube et/ou l'élément supplémentaire de recueil de fluide est fixé à l'extrémité distale de l'endoscope.

14. Ensemble endoscopique selon la revendication 12 ou 13, dans lequel l'endoscope présente au moins un canal de travail qui traverse son intérieur et qui présente des perforations ouvertes vers l'extérieur grâce auxquelles l'élément supplémentaire de recueil de fluide est raccordé de manière permettant la conduction des fluides.

15. Ensemble endoscopique selon l'une des revendications 11 à 14, dans lequel l'élément de recueil de fluide ou l'élément supplémentaire de recueil de fluide présente une éponge en polyuréthanne.

16. Ensemble endoscopique selon l'une des revendications 11 à 15, dans lequel l'élément de recueil de fluide ou l'élément supplémentaire de recueil de fluide consiste en une feuille poreuse permettant la conduction des fluides ou dans lequel l'élément de recueil de fluide ou l'élément supplémentaire de recueil de fluide présente en outre, sur sa surface extérieure, une feuille poreuse permettant la conduction des fluides.

17. Ensemble endoscopique selon la revendication 16, dans lequel la feuille est conçue pour permettre la conduction des fluides vers l'endoscope.

18. Ensemble endoscopique selon l'une des revendications 11 à 17, dans lequel l'endoscope porte une éponge en polyuréthanne en tant que ledit élément supplémentaire de recueil de fluide, tandis que l'unité de surtube porte une feuille en tant que ledit élément de recueil de fluide.

19. Ensemble endoscopique selon l'une des revendications 11 à 18, comportant un système à vide selon la revendication 9, dans lequel les deux extrémités longitudinales du tube de drainage présentent une connexion destinée au raccord avec la pompe à vide, et dans lequel l'unité de drainage à éponge est fixée entre les extrémités longitudinales du tube de drainage.

20. Ensemble endoscopique selon l'une des revendications 11 à 19, dans lequel l'élément de recueil de fluide et/ou l'élément supplémentaire de recueil de fluide sont pourvus, sur certaines parties, d'une imperméabilisation de surface destinée à boucher les pores ouverts, cette imperméabilisation de surface n'étant pas présente sur certaines autres parties.

21. Ensemble endoscopique selon l'une des revendications 11 à 20, dans lequel le tube de drainage se termine distalement par une pointe conique.

22. Ensemble endoscopique selon la revendication 21, dans lequel une bille de préhension, une boucle de fil ou de fil métallique, un oeillet et/ou un fil sont fixés à la pointe du tube de drainage de manière permettant de résister à la traction, la pointe présentant, dans ce dernier cas, un canal transversal dans lequel ledit fil peut être introduit.

23. Ensemble endoscopique selon la revendication 22, dans lequel la pointe distale du tube de drainage consiste en un embout en forme de projectile réalisé en matière plastique ou en métal et conçu pour être fixé sur l'extrémité du tube de drainage par un élément d'emboîtement et/ou de vissage de manière à résister à la traction.

24. Ensemble endoscopique selon l'une des revendications 11 à 23, dans lequel le tube de drainage est conçu pour permettre l'introduction d'un fil de guidage dans la lumière du tube de drainage et dans lequel, à cet effet, une pointe distale du tube de drainage et l'embout présentent un canal longitudinal dans lequel le fil de guidage peut être introduit.

25. Ensemble endoscopique selon l'une des revendications 11 à 24, dans lequel un corps d'éponge de l'élément de recueil de fluide se termine distalement par une pointe, et dans lequel une bille de préhension, une boucle de fil ou de fil métallique, un oeillet et/ou un fil sont fixés sur la pointe d'une manière permettant de résister à la traction et/ou sont intégrés dans le corps d'éponge.

26. Ensemble endoscopique selon l'une des revendications 11 à 25, dans lequel l'une des sondes de mesure de dépression est disposée dans, sur ou au niveau de l'élément de recueil de fluide agencé distalement en étant raccordée à l'unité de régulation de la pression du système à vide.

27. Ensemble endoscopique selon la revendication 26, dans lequel la sonde de mesure de dépression est construite en forme de fil et est rapprochée de l'élément de recueil de fluide par l'intermédiaire du tube de drainage.

28. Ensemble endoscopique selon l'une des revendications 11 à 27, dans lequel le surtube présente, respectivement proximalement et distalement, un épaississement circulaire du type lèvre bordant directement l'élément de recueil de fluide installé sur ledit surtube.

29. Ensemble endoscopique selon l'une des revendications 11 à 28, dans lequel le tube de drainage présente des diamètres différents en différents tronçons longitudinaux.

30. Ensemble endoscopique selon l'une des revendications 11 à 29, dans lequel l'élément de recueil de fluide possède un corps d'éponge qui présente, sur sa surface extérieure, au moins un évidement ou, dans son intérieur, un canal destiné à recevoir une sonde qui est mise en place dans ledit évidement ou ledit canal en vue de l'actionnement de l'ensemble endoscopique.

31. Ensemble endoscopique selon l'une des revendications 11 à 30, dans lequel un canal est disposé dans un corps d'éponge de l'élément de recueil de fluide selon une orientation proximale-distale, dans lequel canal passe une tubulure destinée au passage de fluides corporels tels que des sécrétions ou de la salive, ladite tubulure étant en liaison avec les pores du corps d'éponge d'une manière ne permettant pas la conduction des fluides.

32. Ensemble endoscopique selon la revendication 31, dans lequel la tubulure est stable à la dépression et présente une longueur allant de 5 à 20 cm et un diamètre intérieur allant de 5 à 20 mm.

33. Ensemble endoscopique selon la revendication 31 ou 32, dans lequel la tubulure est hydrophile à l'intérieur et une imperméabilisation de surface de l'élément de recueil de fluide est hydrophile.

34. Ensemble endoscopique selon l'une des revendications 31 à 33, dans lequel une extrémité proximale et/ou une extrémité distale de la tubulure sont déplaçables vers l'extérieur par rapport à une partie intermédiaire de la tubulure et peuvent se déployer lors de l'application d'une dépression.

35. Ensemble endoscopique selon l'une des revendications 31 à 34, dans lequel la tubulure est placée sans fixation dans le canal de l'unité de drainage à éponge.

36. Ensemble endoscopique selon l'une des revendications 11 à 35, dans lequel le diamètre extérieur de l'élément de communication fluidique et celui de l'élément de recueil de fluide sont adaptés à un diamètre intérieur d'un canal de travail interne de l'endoscope, de manière à être déplaçables à l'intérieur du canal de travail et de manière que leur mise en place puisse s'effectuer par le biais du canal de travail interne de l'endoscope.

37. Ensemble endoscopique selon l'une des revendications 11 à 36, dans lequel l'élément de recueil de fluide présente un corps d'éponge et un canal disposé dans le corps d'éponge est pourvu d'une imperméabilisation de surface composée d'une feuille à profilage longitudinal qui est profilée par des canaux conducteurs de fluides dirigés dans une direction longitudinale proximale-distale.

38. Ensemble endoscopique selon l'une des revendications 11 à 37, dans lequel le surtube, l'élément de recueil de fluide, un poussoir et un canal de travail externe sont pourvus d'une fente longitudinale qui se prolonge sur l'intégralité de leur longueur.
